(19) Europäisches Patentamt
European Patent Office
Office européen des brevets

(11) Publication number: **0 615 165 A2**

(12) **EUROPEAN PATENT APPLICATION**

(21) Application number: **94103725.1**

(51) Int. Cl.$^5$: **G03G 5/06**

(22) Date of filing: **10.03.94**

(30) Priority: **12.03.93 JP 52492/93**
**18.03.93 JP 58873/93**

(43) Date of publication of application:
**14.09.94 Bulletin 94/37**

(84) Designated Contracting States:
**DE FR GB IT NL**

(71) Applicant: **KONICA CORPORATION**
**26-2, Nishishinjuku 1-chome,**
**Shinjuku-ku**
**Tokyo 160 (JP)**

(72) Inventor: **Hirose, Naohiro, c/o Konica Corporation**
**1 Sakura-machi**
**Hino-shi, Tokyo (JP)**
Inventor: **Oshiba, Tomomi, c/o Konica Corporation**
**1 Sakura-machi**
**Hino-shi, Tokyo (JP)**

(74) Representative: **Henkel, Feiler, Hänzel & Partner**
**Möhlstrasse 37**
**D-81675 München (DE)**

(54) **Electrophotographic photoreceptor.**

(57) An electrophotographic photoreceptor is disclosed. The photoreceptor contains a 9-substituted imidefluorene as a electron transfermaterial as defined in the specification. It provides a multilayered photoreceptor for positive charge having an excellent electron transferability.

EP 0 615 165 A2

## FIELD OF THE INVENTION

The present invention relates to an electro-photographic photoreceptor to form an electrostatic latent image. More specifically, the present invention relates to an electrophotographic photoreceptor having a layer containing a compound capable of transferring electrons.

## BACKGROUND OF THE INVENTION

Electrophotographic photoreceptors using an organic photoconductor have so far been actively studied because of their advantages of non-environmental pollution, high productivity and low cost, and some, of them are practically used as photoreceptors for medium-speed copying machines. Electrophotographic photoreceptors fall into two types; namely, the multi-layer type and the single-layer type. And, in general, photoreceptors using an organic photoconductor have multi-layer structure comprising a charge generation layer which generates charges when irradiated with light and a charge transfer layer which transfers generated charges. In this case, high-molecular materials, such as poly-N-vinylcarbazole, or low-molecular compounds, such as pyrazoline, hydrazone and triphenylamine derivatives, are used as charge transfer materials.

Since any of these charge transfer materials has a positive hole transfer property, these are mostly used in a developing method which electrifies the surface of a photoreceptor negatively. Therefore, toners which have been used in high-speed copying machines cannot be employed and, as a result, high quality images cannot be easily obtained. Further, such negative electrification on the surface of a photoreceptor is liable to cause problems of generating ozone by reaction with oxygen in the air during electrification and, thereby, polluting environment and degrading the surface of a photoreceptor.

Though there has been developed a multi-layer photoreceptor for positive electrification having reverse layer structure comprising a lower charge transfer layer and an upper charge generation layer, it is insufficient in electrified voltage and low in printing durability and, therefore, requires a protective layer to be provided further on the charge generation layer.

An effective solution of the above problems is to make up a photoreceptor which is positively electrified on its surface, by use of a charge transfer material with electron-transferring property in the charge transfer layer. 2,4,7-trinitro-9-fluorenone is known as such a charge transfermaterial, but its low solubility makes it difficult to obtain a sensitivity enough for practical use by combining it with a conventional charge generation material. In recent years, there are proposed electron transfer materials having a solubilizing group in their electron receptive structure as a result of studies made for the improvement of the property of 2,4,7-trinitro-9-fluorenone. Examples thereof include those described in Japanese Pat. O.P.I. Pub. Nos. 206349/1989, 135362/1990, 290666/1991 and the collection of articles entitled "Japan Hard Copy '92", p.173 (1992). However, any of these compounds is still low in sensitivity and cannot provide images of adequate quality when made up into a photoreceptor in combination with a conventional charge generation material.

## SUMMARY OF THE INVENTION

In view of the foregoing problems, it is an object of the present invention to provide an electrophotographic photoreceptor which uses a charge transfer material having charge transfer property.

It is a further object of the present invention to provide a multi-layered electrophotographic photoreceptor for positive electrification which exhibits excellent electrophotographic properties, namely high sensitivity, low residual potential and good image forming property.

The electrophotographic photoreceptor of the invention comprises a conductive support and a photoreceptive layer unit containing, as a charge transfer material, one of the compounds represented by formulas A, B, C, D, E, F, G, H and I.

A

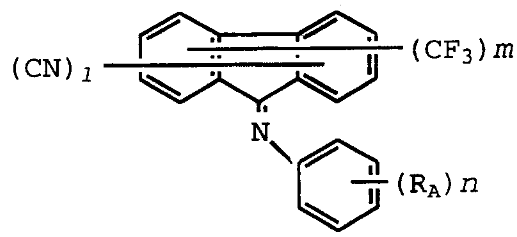

in formula, $l$, $m$ and n represent integers of $l \geqq 0$, $m \geqq 0$ and $n \geqq 0$, respectively, provided $m \geqq 2$ when $l = 0$, $m \geqq 1$ when $l \geqq 1$ and $m \geqq 0$ when $l \geqq 2$ are satisfied; $R_A$ represents a nitro, OH or $CF_3$ group, a halogen, a substituted or unsubstituted alkyl, alkoxy, aryl, aralkyl or alkylamino group, or a $CONH_2$, $COOR_A'$, $CONHR_A'$, $CON(R_A')_2$ or $COR_A'$ group; $R_A'$ represents a substituted or unsubstituted alkyl, aryl or aralkyl group; and $R_A$ may be the same or different when $n \geqq 2$.

B

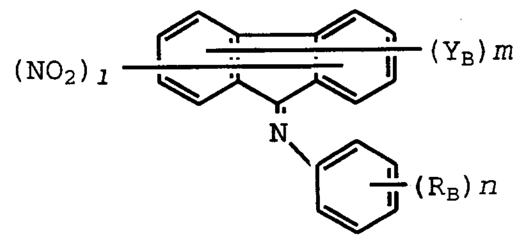

in formula, $l$, $m$ and n represent integers of $l \geqq 0$, $m \geqq 0$ and $n \geqq 0$, respectively; $R_B$ represents a nitro, OH, a halogen, $CF_3$, a substituted or unsubstituted alkyl, alkoxy, aryl, aralkyl or alkylamino group, or a $CONH_2$, $COOR_B'$, $CONHR_B'$, $CON(R_B')_2$ or $COR_B'$ group; $R_B'$ represents a substituted or unsubstituted alkyl, aryl or aralkyl group; and $R_A$ may be the same or different when $n \geqq 2$; $Y_B$ is CN or $CF_3$.

C

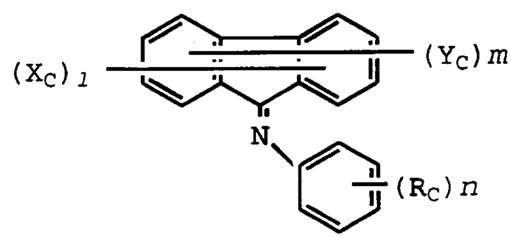

in formula, $l$, $m$ and n represent integers of $l \geqq 1$, $m \geqq 0$ and $n \geqq 1$, respectively, provided $m \geqq 1$ when $l = 1$, and $m \geqq 0$ when $l \geqq 2$ are satisfied; $R_C$ represents a nitro, OH or $CF_3$ group, a halogen, a substituted or unsubstituted alkyl, alkoxy, aryl, aralkyl or alkylamino group, or a $CONH_2$, $COOR_C'$, $CONHR_C'$, $CON(R_C')_2$ or $COR_C'$ group; $R_C'$ represents a substituted or unsubstituted alkyl, aryl or aralkyl group; and $R_C$ may be the same or different when $n \geqq 2$; $X_C$ is $OCOR_C'$, $COOR_C'$, $CONHR_C'$, $COR_C'$, CHO or $CON(R_C')_2$; $Y_C$ is CN, $NO_2$, halogen, $CF_3$, COOH, $SO_2NH_2$, $SOR_C'$ or $SO_2R_C'$.

D

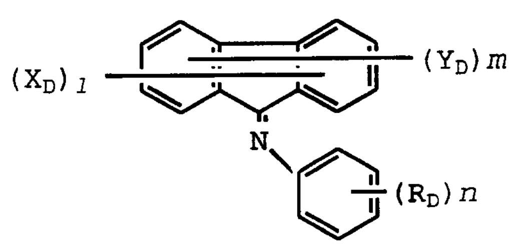

in formula, $l$, $m$ and n represent integers of $l \geqq 1$, $m \geqq 0$ and $n \geqq 1$, respectively, provided $m \geqq 1$ when $l = 1$, and $m \geqq 0$ when $l \geqq 2$ are satisfied; $R_D$ represents a nitro, OH, a halogen, $CF_3$, a substituted or unsubstituted alkyl, alkoxy, aryl, aralkyl or alkylamino group, or a $CONH_2$, $COOR_D'$, $CONHR_D'$, $CON(R_D')_2$ or $COR_D'$ group; $R_D'$ represents a substituted or unsubstituted alkyl, aryl or aralkyl group; and $R_D$ may be the same or different when $n \geqq 2$; $X_D$ is COOH, $SO_2NH_2$, $SOR_D'$ or $SO_2R_D'$; $Y_D$ is CN, $NO_2$, halogen or $CF_3$.

E

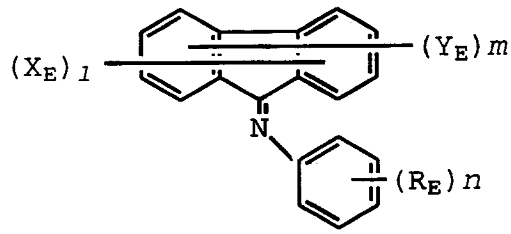

in formula, $l$, $m$ and n represent integers of $l \geqq 1$, $m \geqq 0$ and $n \geqq 1$, respectively, provided $m \geqq 1$ when $l = 1$, $m \geqq 0$ when $l \geqq 2$ are satisfied; $R_E$ represents a nitro, OH or $CF_3$ group, a halogen, a substituted or unsubstituted alkyl, alkoxy, aryl, aralkyl or alkylamino group, or a $CONH_2$, $COOR_E'$, $CONR_E'$, $CON(R_A')_2$ or $COR_E'$ group; $R_E'$ represents a substituted or unsubstituted alkyl, aryl or aralkyl group; and $R_E$ may be the same or different when $n \geqq 2$; $X_E$ is halogen; $Y_E$ is CN, $NO_2$, or $CF_3$.

F

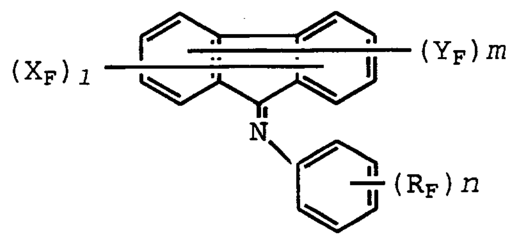

in formula, $l$, $m$ and n represent integers of $l \geqq 2$, $m \geqq 1$ and $n \geqq 1$, respectively; $R_F$ represents a nitro, OH, a halogen, $CF_3$, a substituted or unsubstituted alkyl, alkoxy, aryl, aralkyl or alkylamino group, or a $CONH_2$, $COOR_F'$, $CONHR_F'$, $CON(R_F')_2$ or $COR_F'$ group; $R_F'$ represents a substituted or unsubstituted alkyl, aryl or aralkyl group; and $R_F$ may be the same or different when $n \geqq 2$; $R_{0F}$ is a substituted or unsubstituted alkyl, aryl or aralkyl group; $X_F$ is CN, $NO_2$, halogen atom, $CF_3$, CHO, COOH, $SO_2NH_2$, $SOR_F'$ or $SO_2R_F'$, $OCOR_F'$, $CONHR_F'$, $COOR_F'$, $CONHR_F'$ or $COR_F'$.

G

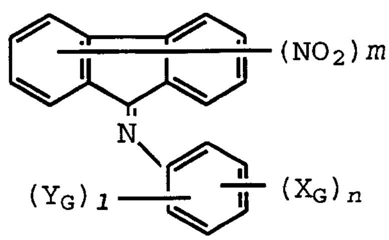

in formula, $l$, $m$ and n represent integers of $l \geqq 0$, $m \geqq 2$ and $n \geqq 1$, respectively; $X_G$ represents a nitro, OH, a halogen, CN, a substituted or unsubstituted aryl or aralkyl group, or a $N(R_G')_2$, $OR_G'$, $OCOR_G'$, $COOR_G'$, $CONHR_G'$, $CON(R_G')_2$ or $COR_G'$ group; $R_G'$ represents a substituted or unsubstituted aryl or aralkyl group; and $X_G$ may be the same or different when $n \geqq 2$; $Y_G$ is a substituted or unsubstituted alkyl group.

H

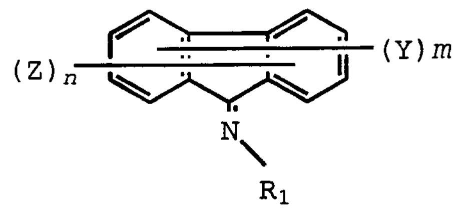

in formula, Y is an $NO_2$, CN, $CF_3$, $COR_2$, $COOR_2$, $CONHR_2$, a halogen, $SOR_2$, or $SO_2R_2$; Z is an alkyl, or aryl group, OH, or $N(R_2)_2$; $R_1$ is an alkyl group; $R_2$ is an alkyl, or aryl group; $m$ is an integer of $m \geqq 2$, n is an integers of $n \geqq 0$, respectively, provided the sum of $m$ and $n$ being 2 to 8; and Y, Z and $R_2$ may be the same or different when each of them exists two or more.

I

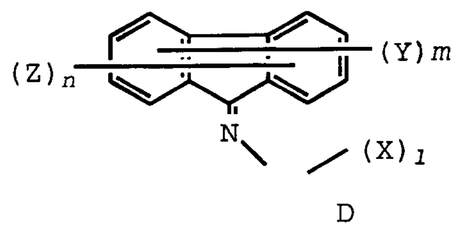

in formula, D is a group of atoms to form a heterocycle; X and Y each represent $NO_2$, CN, $CF_3$, $COR_2$, $COOR_2$, $CONHR_2$, a halogen atom, $SOR_2$, $SO_2R_2$ or $OCOR_2$; Z is an alkyl or aryl group, $OR_2$, OH, or N-$(R_2)_2$; $R_1$ is an alkyl group; $R_2$ is an alkyl or aryl group; $l$, $m$ and n represent 0 or positive integers respectively, provided $l + m \geqq 2$, and $2 \leqq m + n \leqq 8$; and Y, Z and $R_2$ may be the same or different when each of them exists two or more.

**BRIEF DESCRIPTION OF THE DRAWING.**

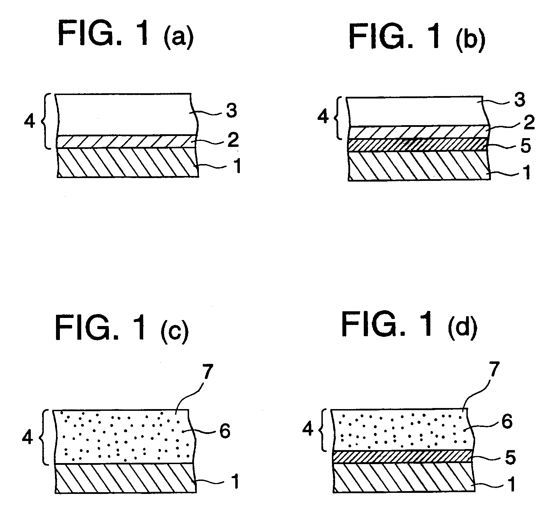

Figure 1 illustrates a sectional view of an embodiment of the photoreceptor of the invention.

1 Electric conductive support
2 Charge generating layer
3 Charge transfer layer
4 Photosensitive layer
5 Inter layer
6 Layer containing charge transfer material
7 Charge transfer material

**DETAIL DISCLOSURE OF THE INVENTION**

In a preferred embodiment of the invention, there are used compounds of formulas a-1 to g-1 derived from the above compounds of formulas A to G, respectively, by placing restrictions on the structural constituents of formulas A to G. Formulas of a-1 to g-1 are disclosed.

a-1

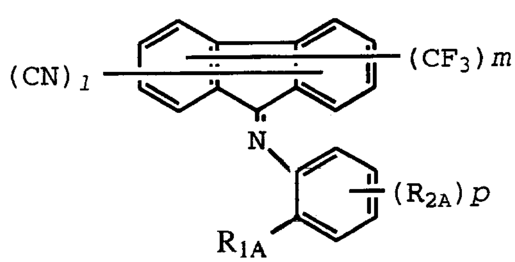

in the formula *l* and *m* are the same as defined in the formula A, *p* is an integer of $p \geqq 0$; $R_{1A}$ and $R_{2A}$ are an alkyl group that may have a substituent, cyano group or halogen atom.

b-1

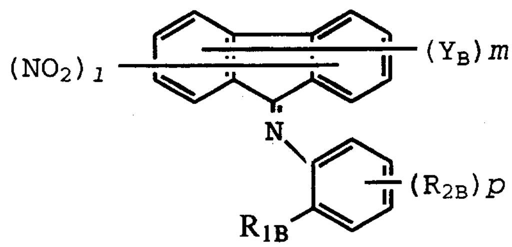

in the formula *l* and *m* are the same as defined in the formula B, *p* is an integer of $p \geqq 0$; $R_{1B}$ and $R_{2B}$ are an alkyl group that may have a substituent, cyano group or halogen atom.

c-1

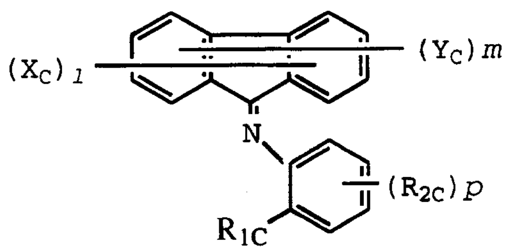

in the formula $X_C$, $Y_C$, *l* and *m* are the same as defined in the formula C, *p* is an integer of $p \geqq 0$; $R_{1C}$ and $R_{2C}$ are an alkyl group that may have a substituent, cyano group or halogen atom.

d-1

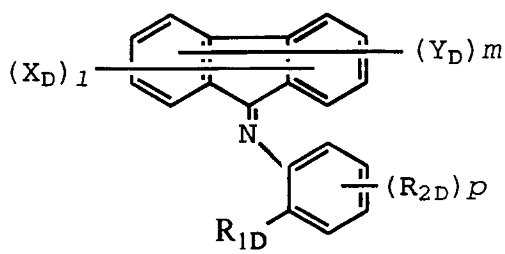

in the formula $X_D$, $Y_D$ *l* and *m* are the same as defined in the formula D, *p* is an integer of $p \geqq 0$; $R_{1D}$ and $R_{2D}$ are, respectively, an alkyl group that may have a substituent, cyano group or halogen atom.

e-1

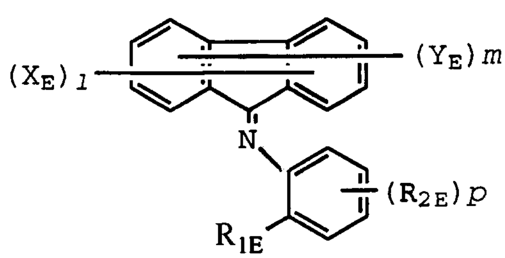

in the formula $X_E$, $Y_E$, *l* and *m* are the same as defined in the formula E, *p* is an integer of $p \geqq 0$; $R_{1E}$ and $R_{2E}$ are an alkyl group that may have a substituent, cyano group or halogen atom.

f-1

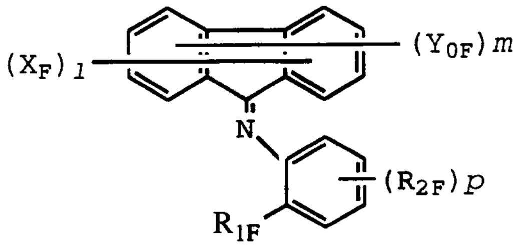

in the formula $X_F$, $R_{0F}$,*l* and *m* are the same as defined in the formula F, *p* is an integer of $p \geqq 0$; $R_{1F}$ and $R_{2F}$ are an alkyl group that may have a substituent, cyano group or halogen atom.

g-1

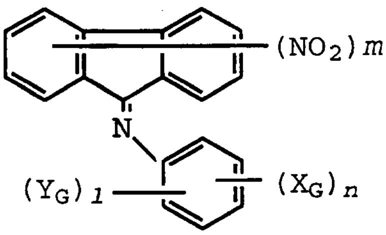

in the formula *l*, *m* and n represent integers of $l \geqq 0$, $m \geqq 3$ and $n \geqq 1$, respectively; $X_G$ represents a nitro, a halogen, CN, a substituted or unsubstituted aryl or group, or a $COOR_G'$, $OCOR_G'$, $CONHR_G'$, $CON(R_G')_2$ or $COR_G'$ group; $R_G'$ represents a substituted or unsubstituted aryl or aralkyl group; the other groups are the same as defined in the formula G.

The high performance the charge transfer material of the invention has is attributed to its superiority over conventional charge transfer materials in capability of maintaining a high compatibility with a binder for a long time.

The following are typical examples of the compounds represented by the above formulas and methods for synthesizing them:

(A) Compounds Represented by Formula A

Example of Compounds:

A－1

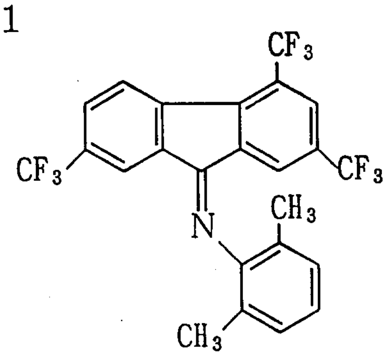

A－2

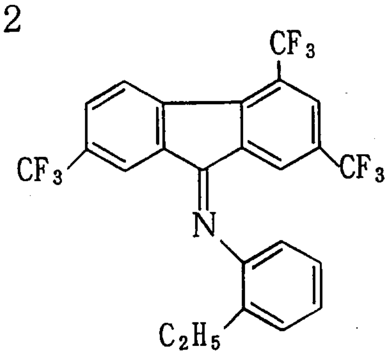

A－3

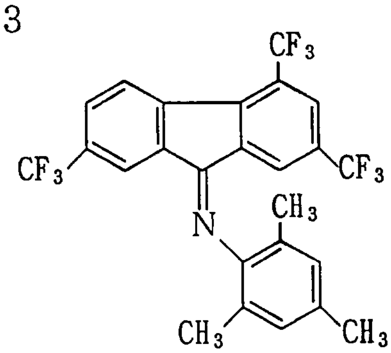

A－4

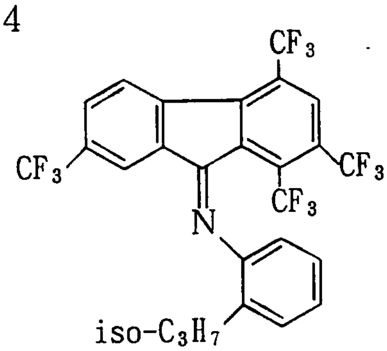

A－5

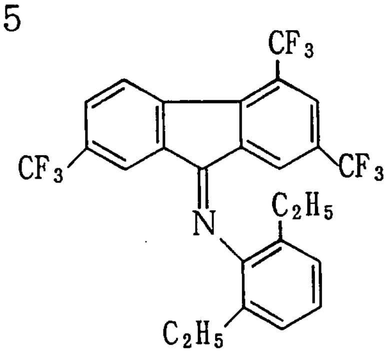

A－6

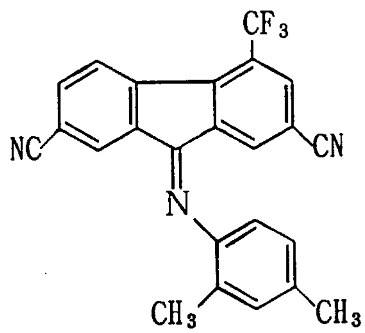

A − 7

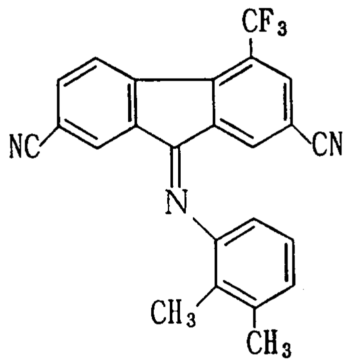

A − 8

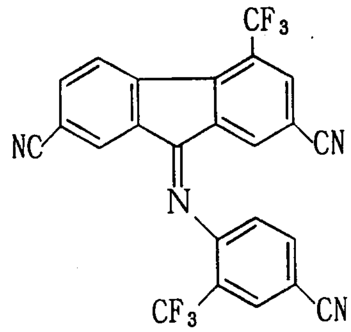

A − 9

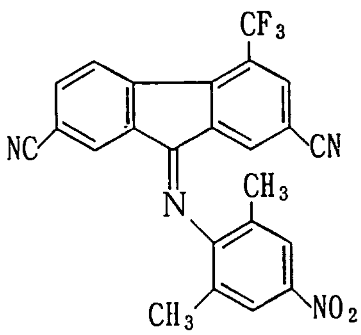

A − 10

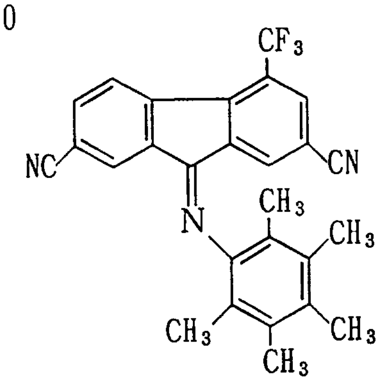

A − 11

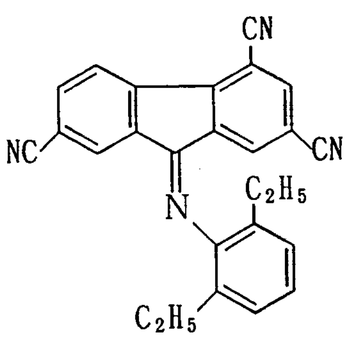

A − 12

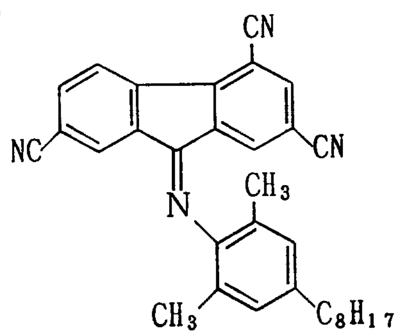

A-13

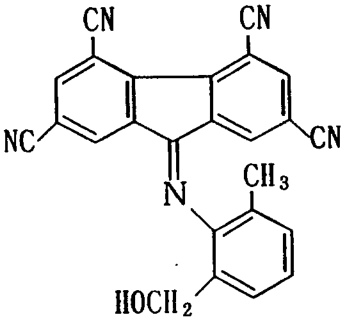

A-14

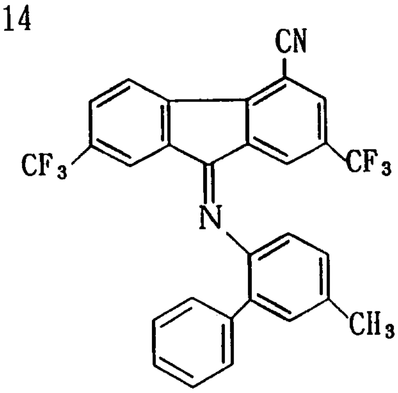

A-15

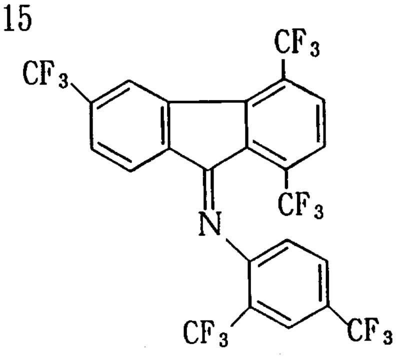

A-16

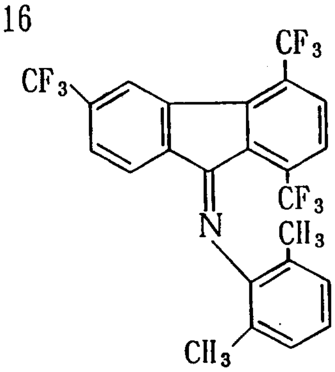

A-17

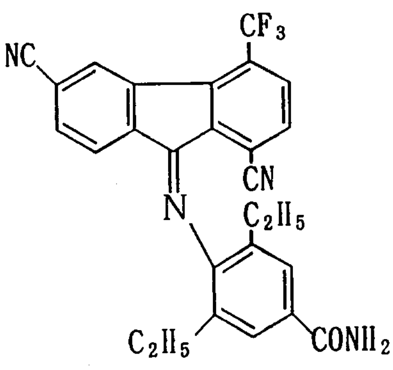

A-18

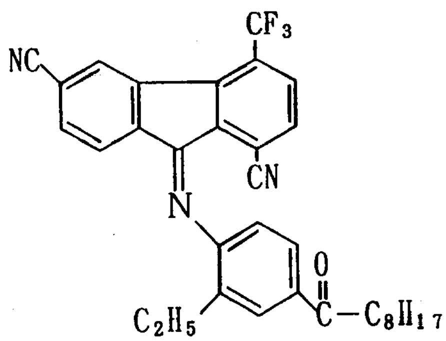

A－19

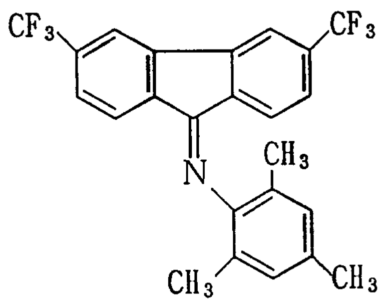

A－20

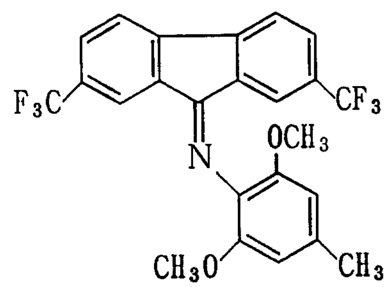

A－21

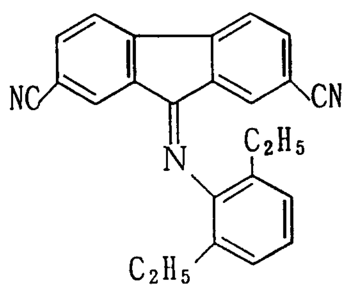

A－22

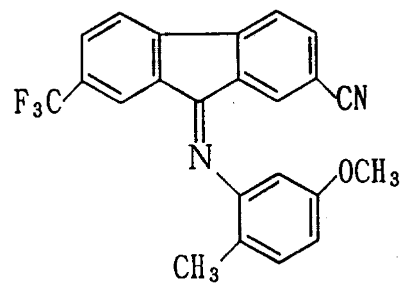

A－23

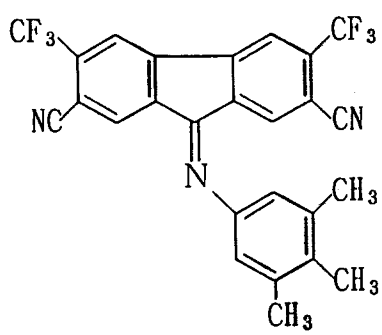

A－24

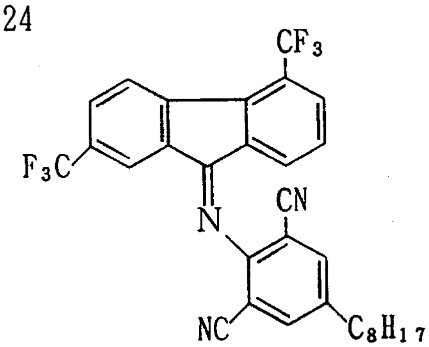

A − 25

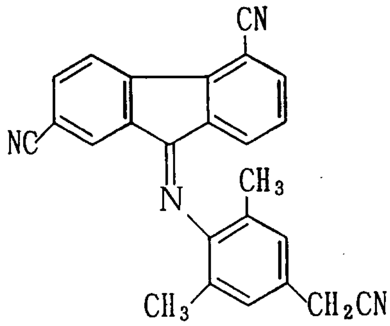

A − 26

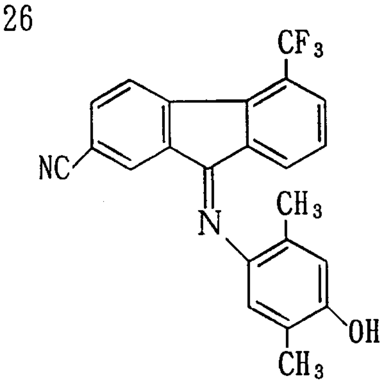

A − 27

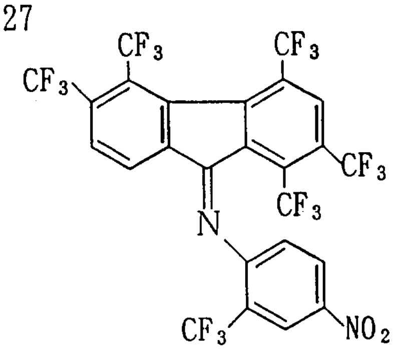

A − 28

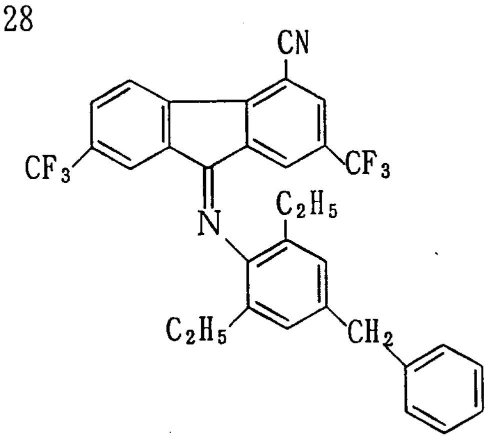

A − 29

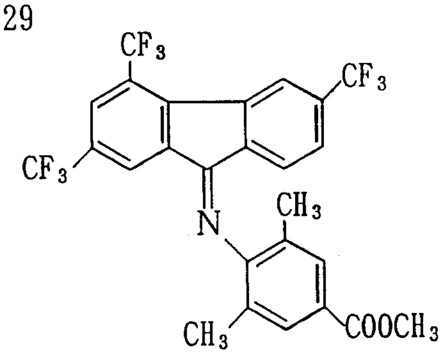

A − 30

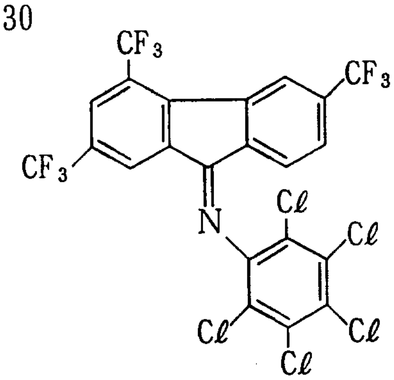

Synthesis Example

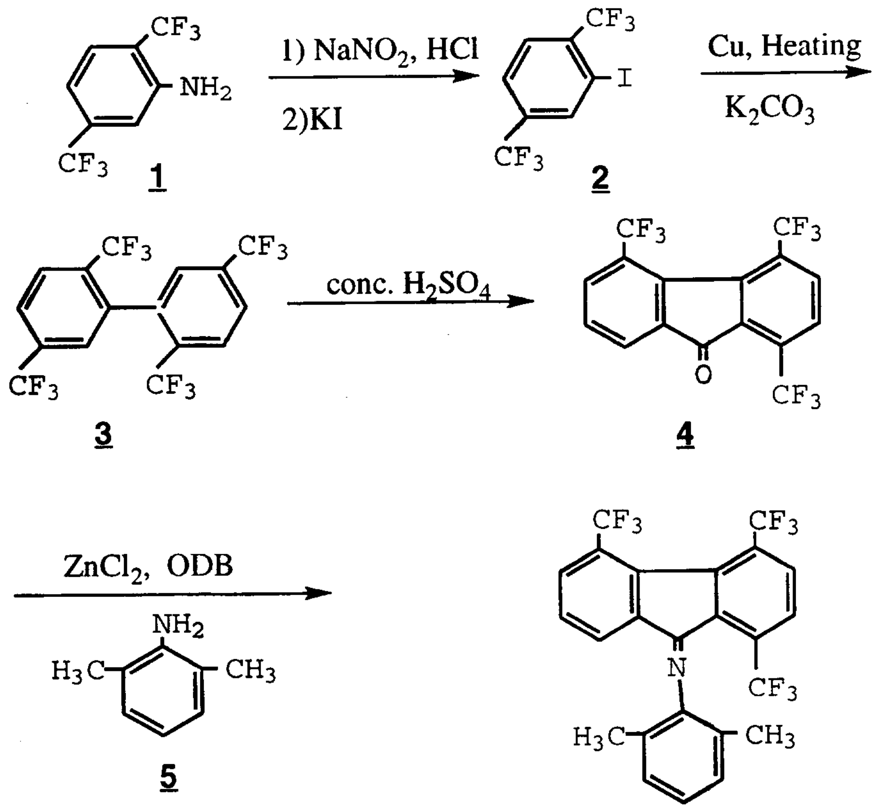


1 2 3 4 5

Exemplified Compound A-16

Exemplified Compound A-16 is synthesized according to the synthesis procedure mentioned above. Compound (4) is synthesized according to a method known by J. Chem. Soc., 1071 - 6 (1954), from 2,5-bis(trifluoromethyl)aniline (Made by Aldrich Co. ) as a starting material.

In a 100-ml four-necked flask was placed 10 g of Compound 4, 5 g of 2,6-xylidine (Made by Wako Jun-yaku Co., Ltd.), 20 g of zinc chloride, and 100 ml of o-chlorobenzen (hereafter called as ODB), and then an esterification tube and a thermometer were attached to the flask, followed by heating under reflux for 50 hours. After cooling the reaction mixture, the solid matter was sucked off and washed well with ethyl acetate. The solvent in the mother liquor was distilled away under reduced pressure, and the residue was subjected to silica gel column chromatography. The dissolved matter in a 1:1 mixture of toluene and n-hexane was crystallized from n-hexane. Thus, red crystals of exemplified compound A-16 was obtained in a yield of 9.5 g (75%). Its melting point was 153-155 °C when measured by the method of measuring meting point specified in the Japanese Pharmacopoeia. Since the results obtained by elemental analysis also agreed well with the calculated values, the product was identified as exemplified compound A-16.

| | C | H | N |
|---|---|---|---|
| Calculated Values | 59.15% | 2.90% | 2.87% |
| Measured Values | 58.51% | 3.01% | 2.37% |

Examples of Compound represented by Formula B

Example of Compound.

B – 1

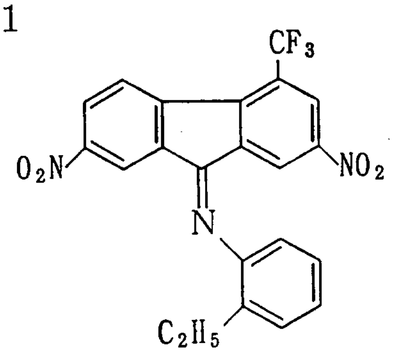

B – 2

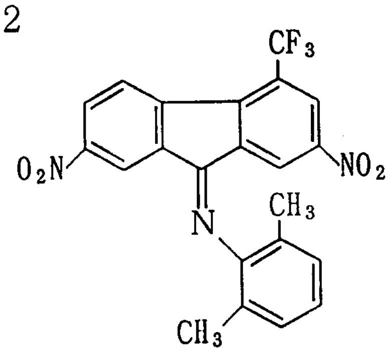

B – 3

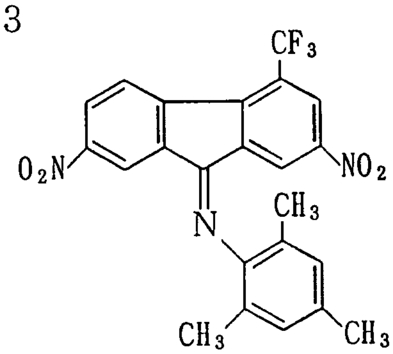

B – 4

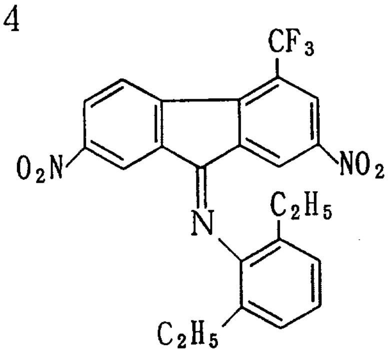

B – 5

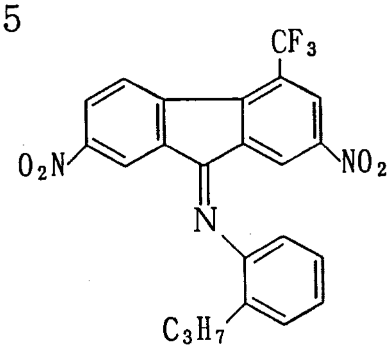

B – 6

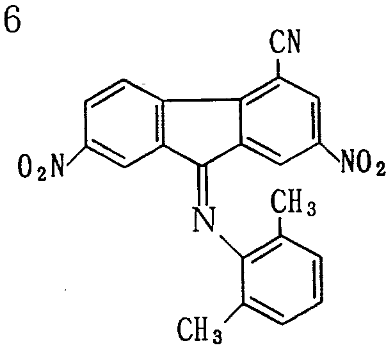

B − 7

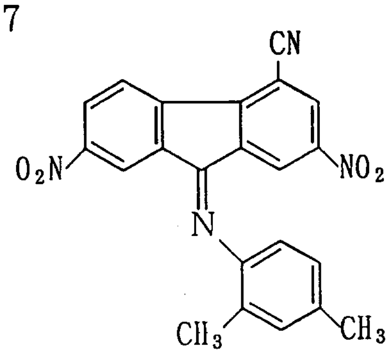

B − 8

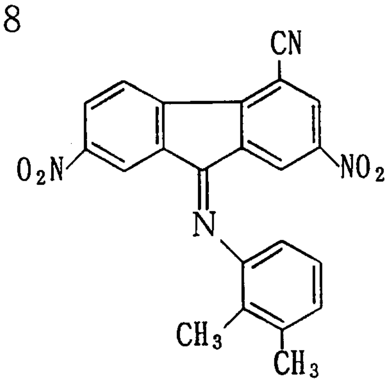

B − 9

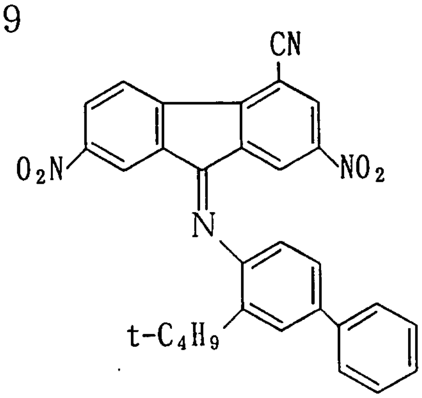

B − 10

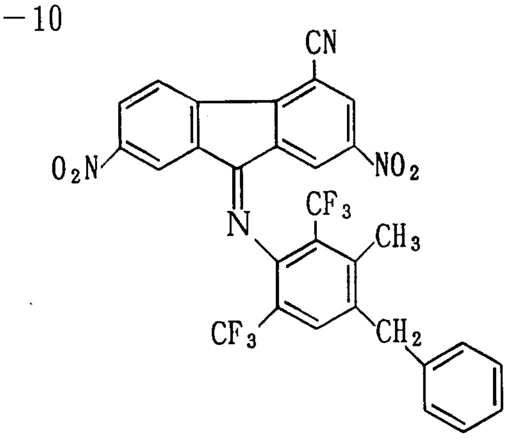

B − 11

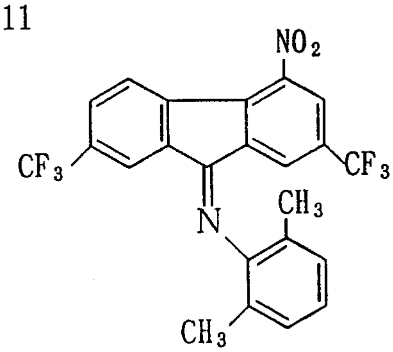

B − 12

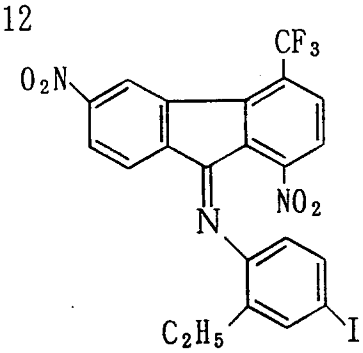

B－13

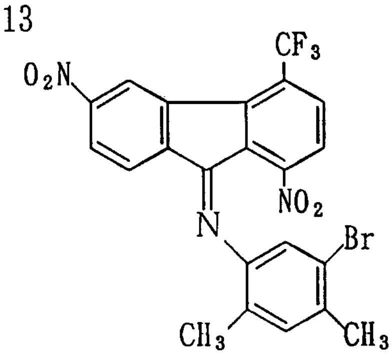

B－14

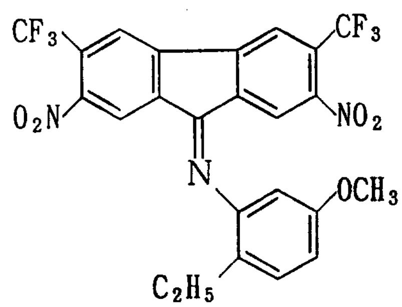

B－15

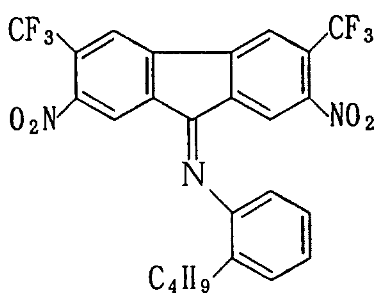

B－16

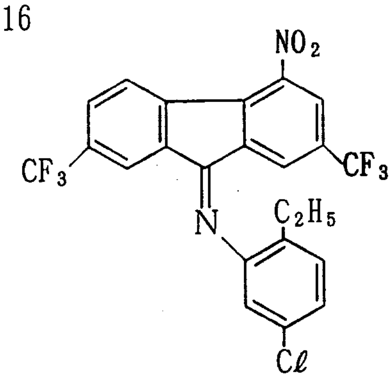

B－17

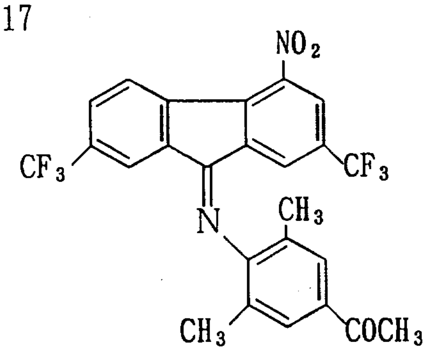

B－18

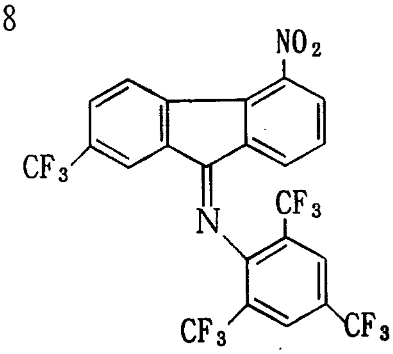

B－19

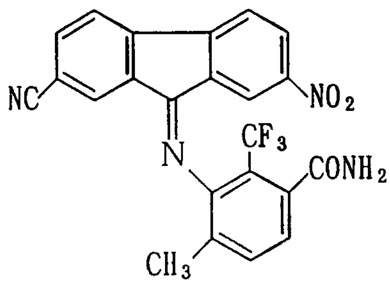

B－20

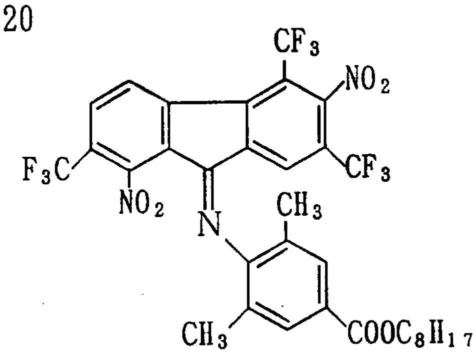

B－21

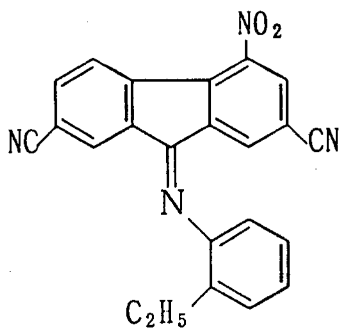

B－22

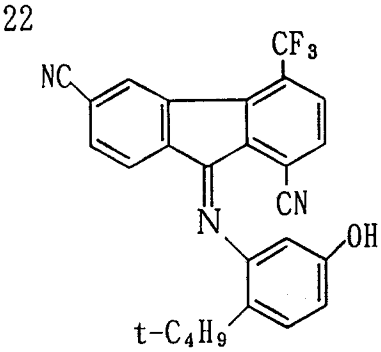

B－23

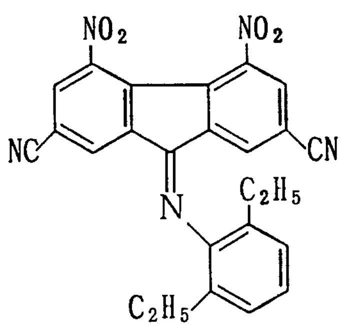

B－24

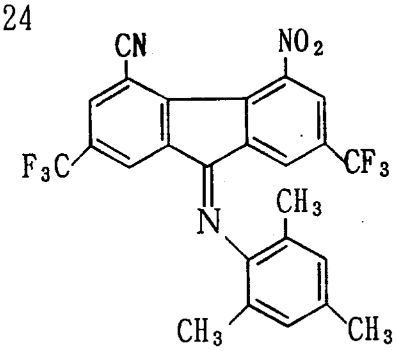

B－25

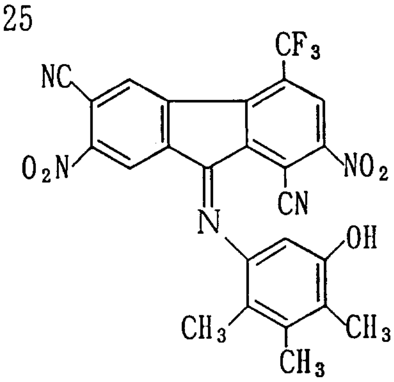

B－26

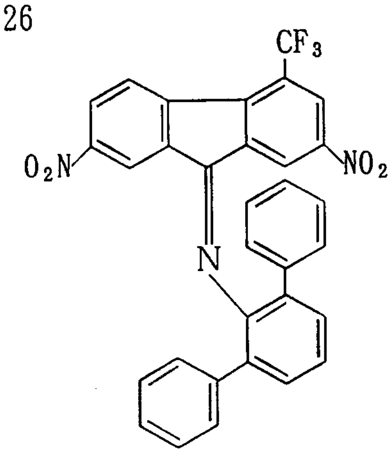

B－27

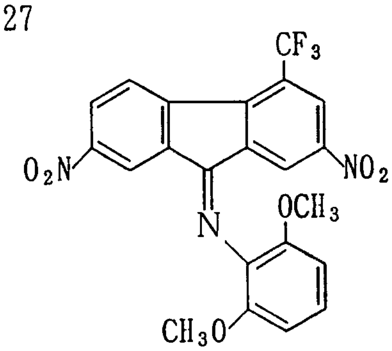

B－28

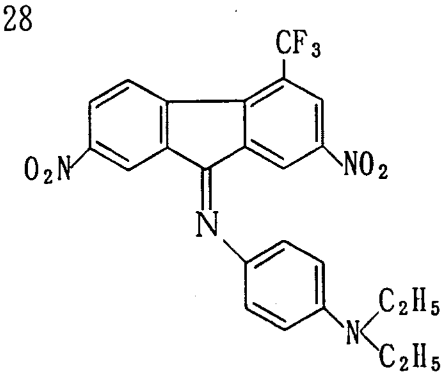

B－29

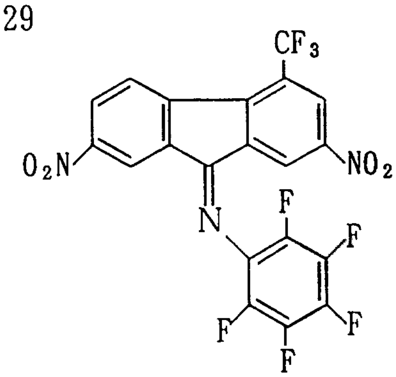

B－30

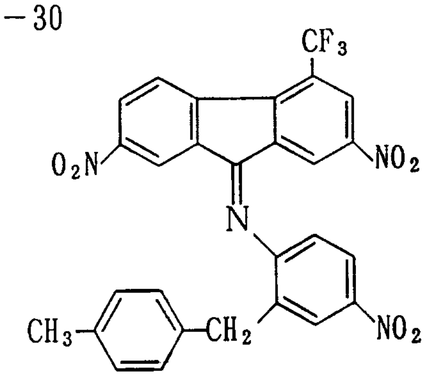

Synthesis Example

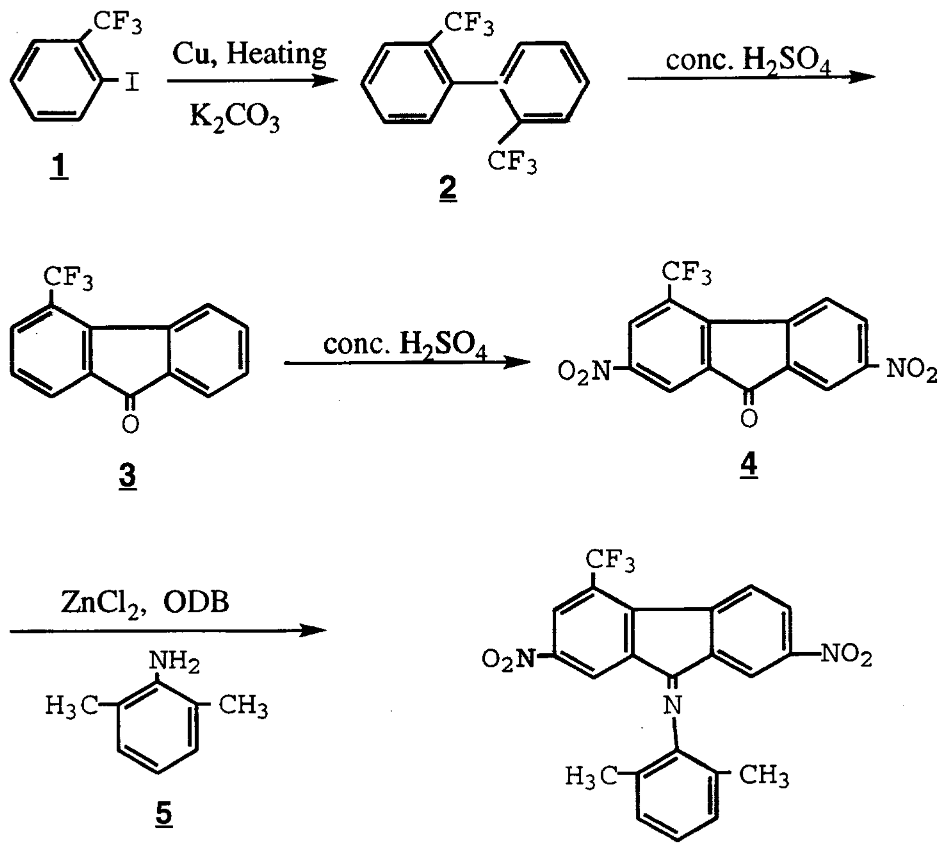


1 2 3 4 5

Exemplified Compound B-2

Exemplified Compound B-2 is synthesized according to the synthesis procedure. Compound (4) is synthesized according to a method known by J. Chem, Soc., 1071 - 6 (1954), from 2-iodobenzotrifluoride (Made by Aldrich Co. ) as a starting material.

In a 100-ml four-necked flask was placed 10 g of Compound (4), 5 g of 2,6-xylidine (Made by Wako Jun-yaku Co., Ltd.), 20 g of zinc chloride, and 100 ml of ODB, and then an esterification tube and a thermometer were attached to the flask, followed by heating under reflux for 50 hours. After cooling the reaction mixture, the solid matter was sucked off and washed well with ethyl acetate. The solvent in the mother liquor was distilled away under reduced pressure, and the residue was subjected to silica gel column chromatography. The dissolved matter in a 1:1 mixture of toluene and n-hexane was crystallized from n-hexane. Thus, red crystals of exemplified compound B-2 was obtained in a yield of 8.5 g (64%). Its melting point was 189-190 °C when measured by the method of measuring meting point specified in the Japanese Pharmacopoeia. Since the results obtained by elemental analysis also agreed well with the calculated values, the product was identified as exemplified compound B-2.

| | C | H | N |
|---|---|---|---|
| Calculated Values | 59.87% | 3.20% | 9.52% |
| Measured Values | 59.71% | 3.31% | 9.40% |

(C) Examples of Compound represented by Formula C

Example of Compound.

C－1

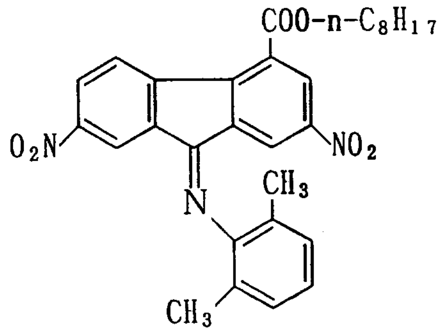

C－2

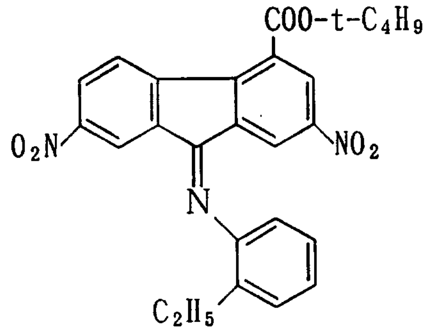

C－3

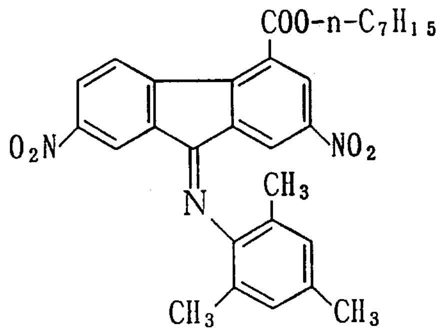

C－4

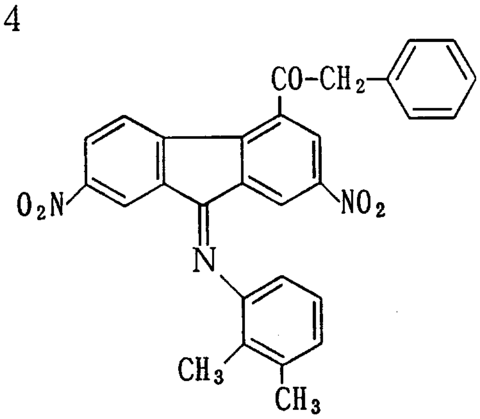

C－5

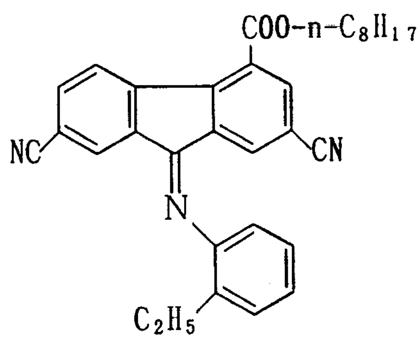

C－6

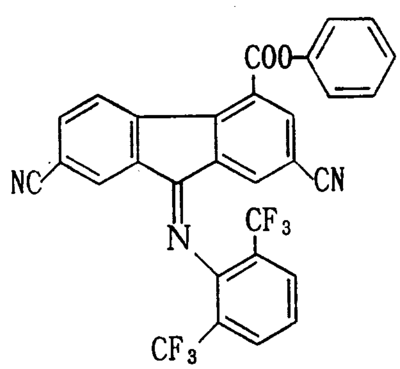

C－7

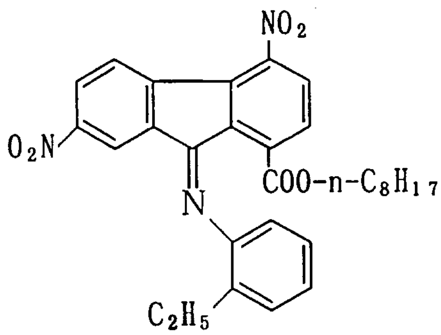

C－8

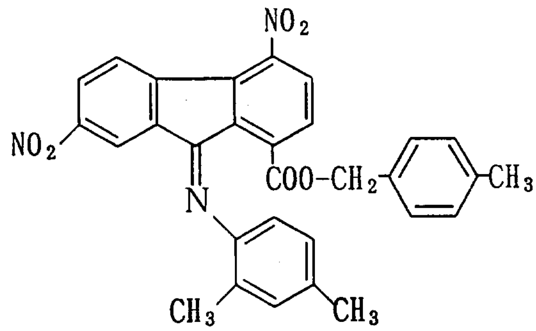

C－9

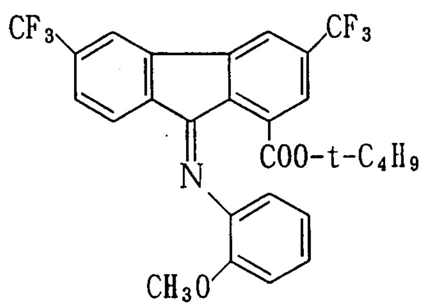

C－10

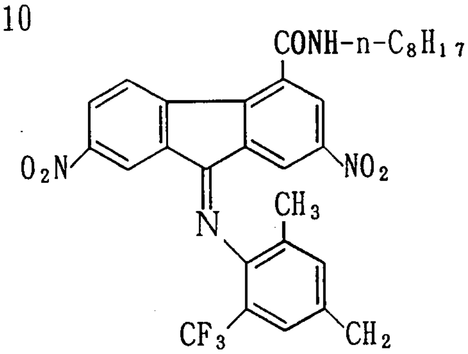

C－11

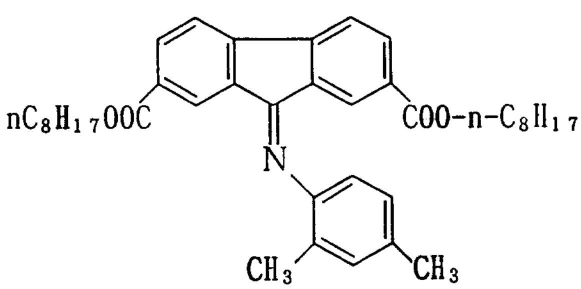

C－12

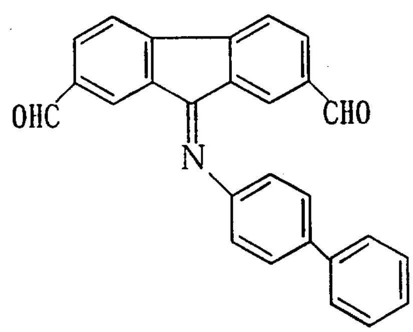

C－13

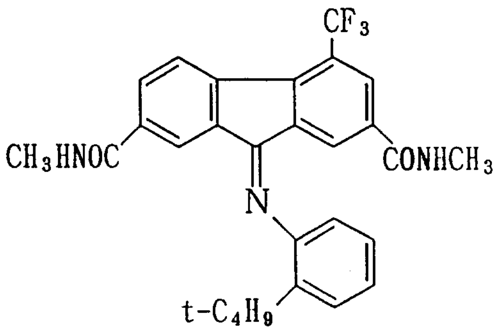

C－14

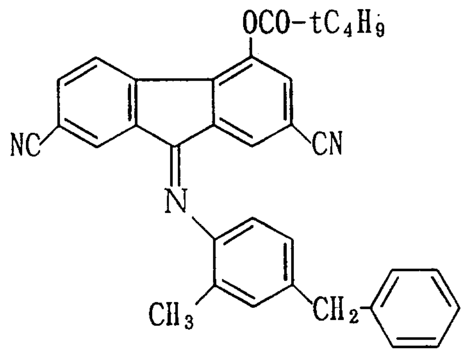

C－15

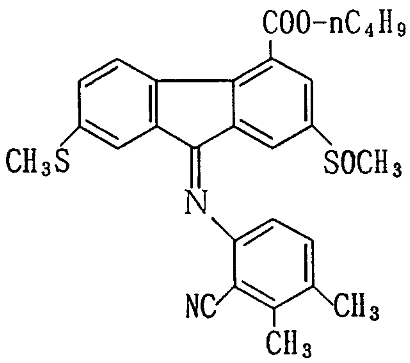

C－16

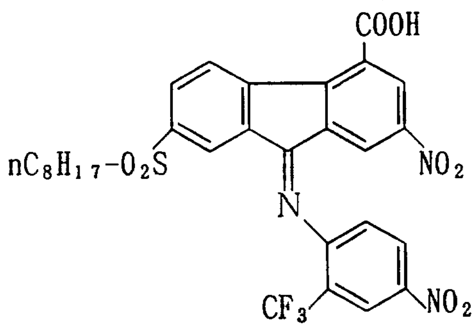

C－17

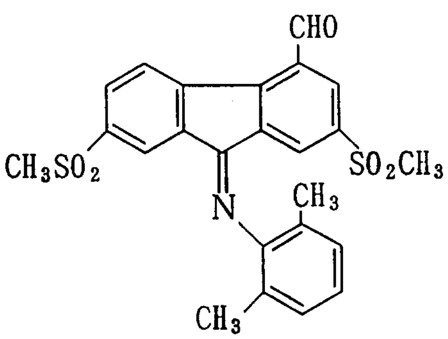

C－18

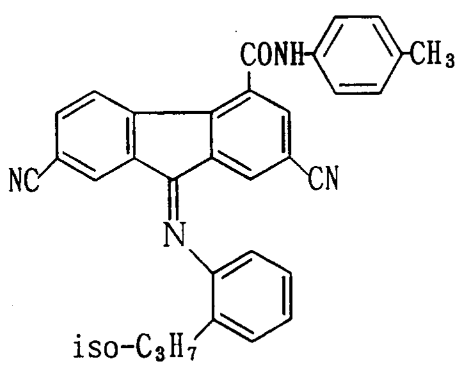

C－19

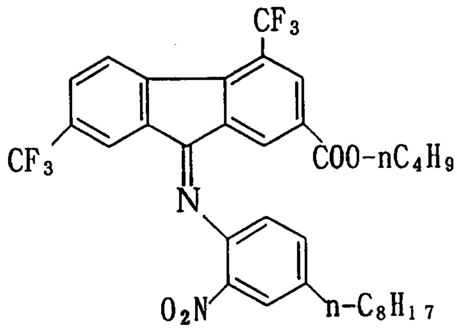

C－20

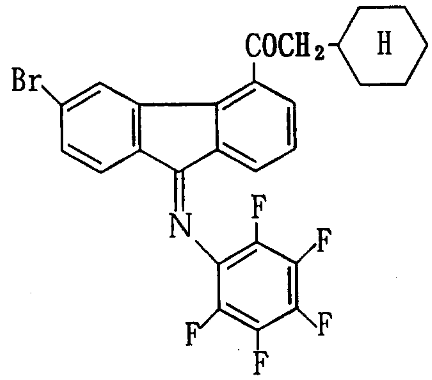

C－21

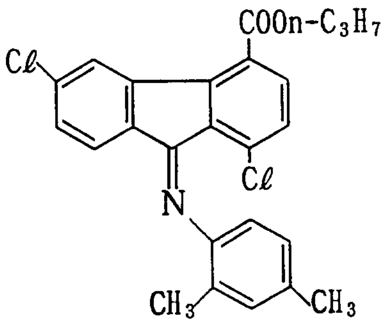

C－22

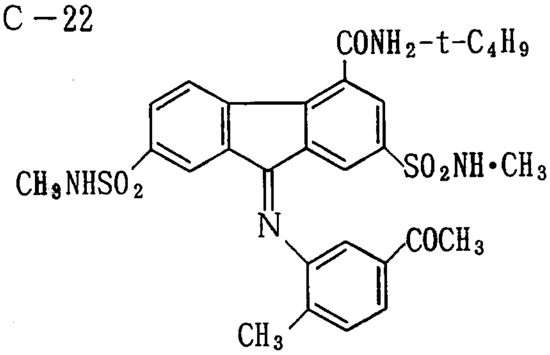

C－23

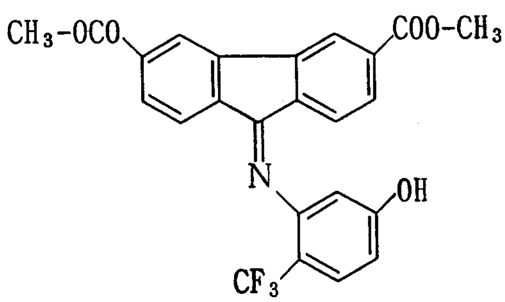

C－24

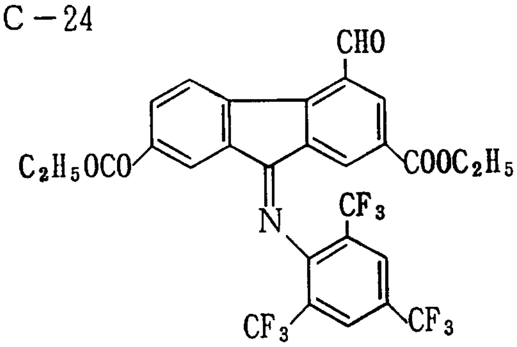

C－25

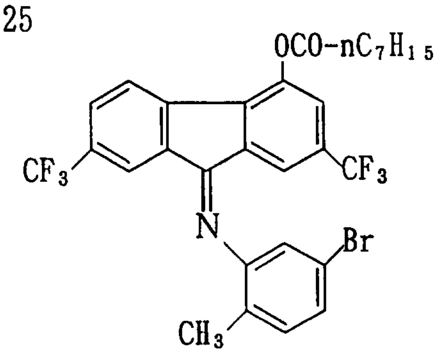

C－26

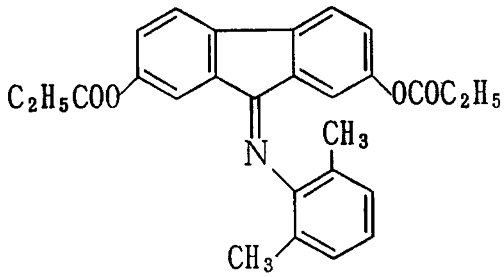

C－27

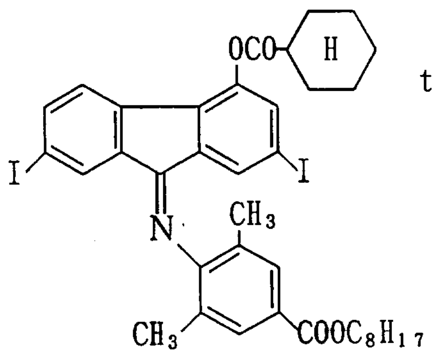

C－28

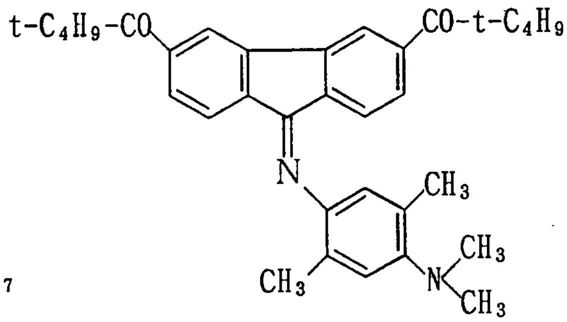

C－29

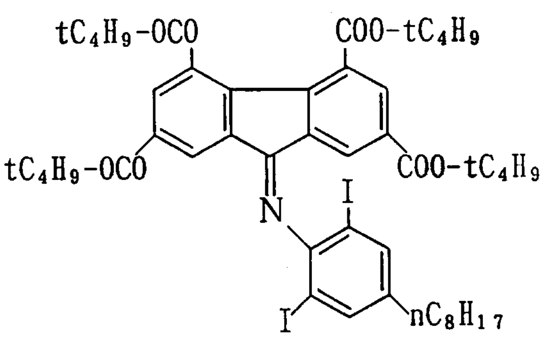

C－30

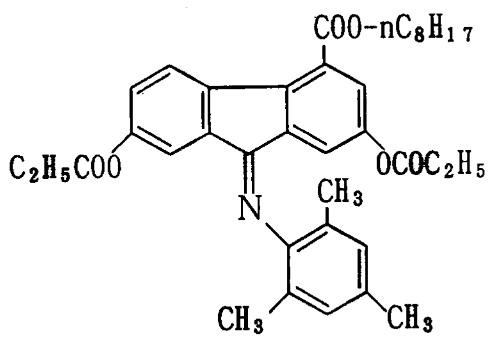

C－31

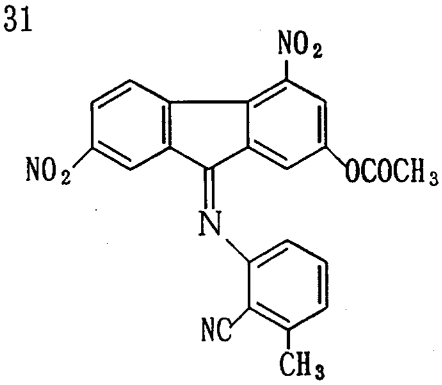

C－32

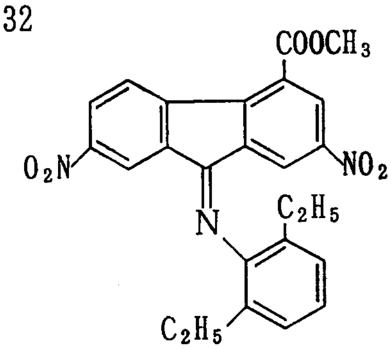

C－33

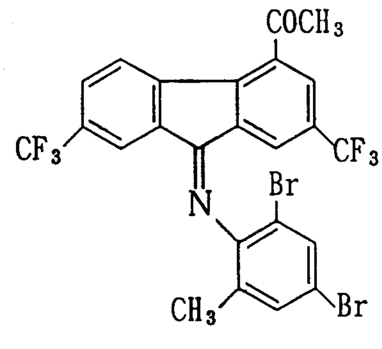

C－34

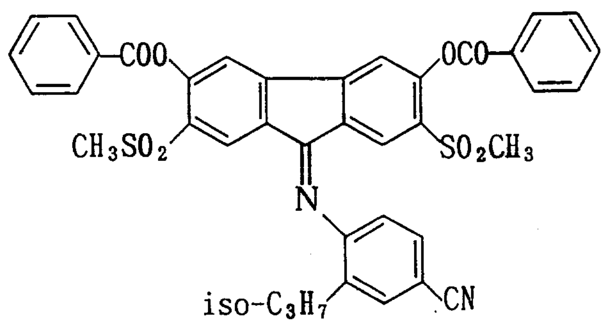

C－35

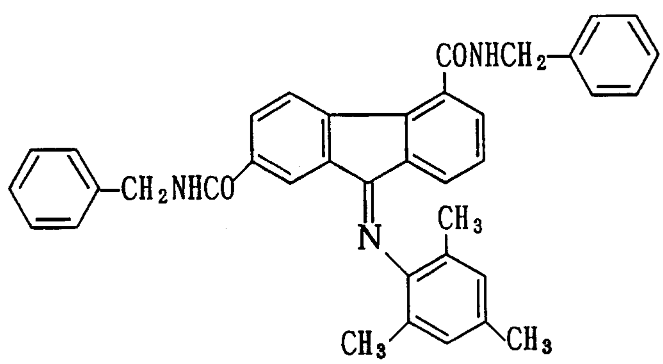

Synthesis Example

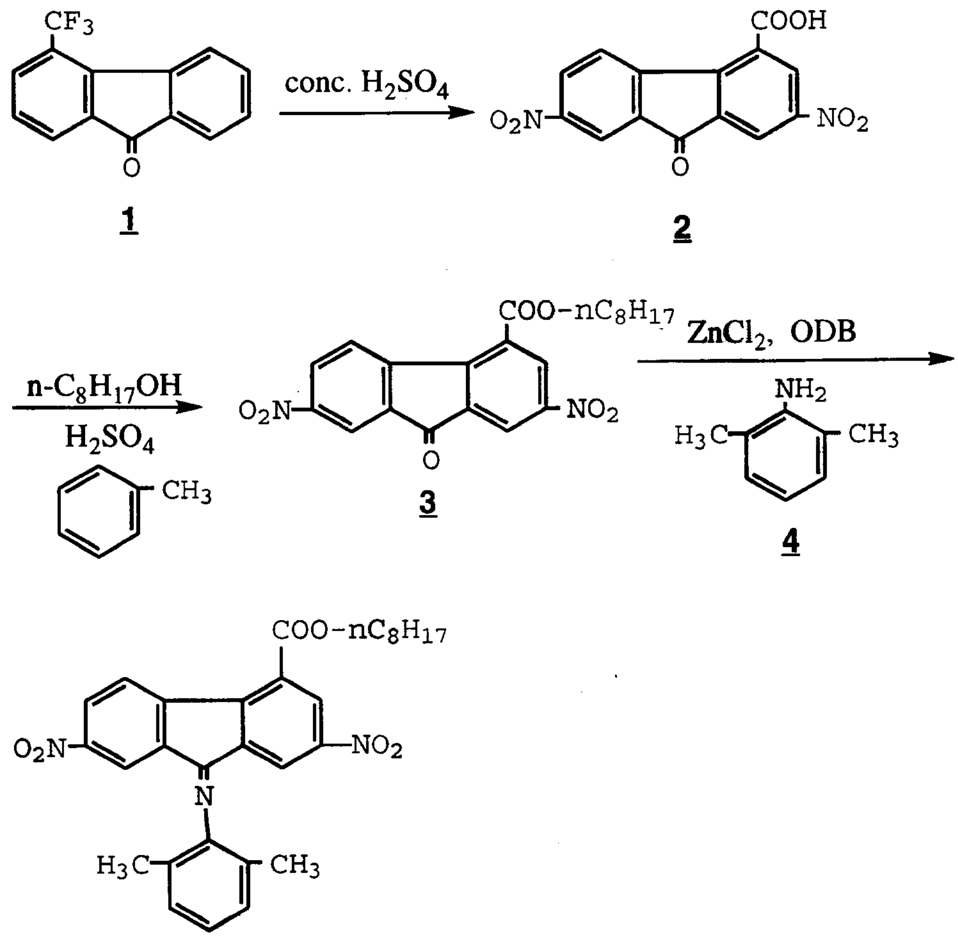


1 2 3 4

Exemplified Compound C-1

Exemplified compound C-1 was synthesized by the above reaction route. Compound (3) was synthesized from 9-fluorenone-4-carboxilic acid (Made by Aldrich Co.) according to normal method.

In a 100-ml four-necked flask was placed 20 g of zinc chloride, 5 g of 2,6-xylidine (5) (Made by Wako Jun-yaku Co., Ltd.) and 100 ml of ODB, and then an esterification tube and a thermometer were attached to the flask, followed by heating under reflux for 50 hours. After cooling the reaction mixture, the solid matter was sucked off and washed well with ethyl acetate. The solvent in the mother liquor was distilled away under reduced pressure, and the residue was subjected to silica gel column chromatography. The dissolved matter in a 1:1 mixture of toluene and n-hexane was crystallized from n-hexane. Thus, red crystals of exemplified compound C-1 was obtained in a yield of 6.8 g (56%). Its melting point was 193-196 °C when measured by the method of measuring meting point specified in the Japanese Pharmacopoeia. Since the results obtained by elemental analysis also agreed well with the calculated values, the product was identified as exemplified compound C-1.

| | C | H | N |
|---|---|---|---|
| Calculated Values | 68.04% | 5.90% | 7.93% |
| Measured Values | 67.59% | 6.05% | 7.72% |

(D) Compounds represented by Formula D

Example of Compound

D－1

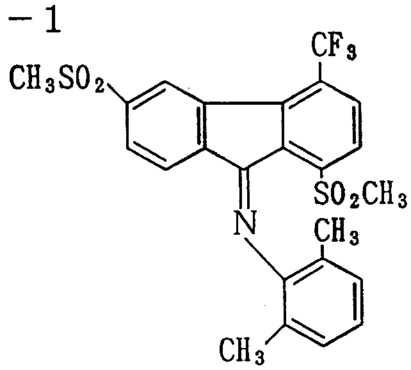

D－2

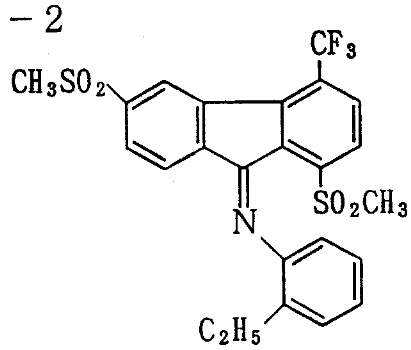

D－3

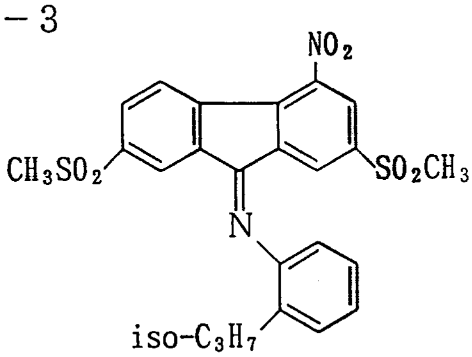

D－4

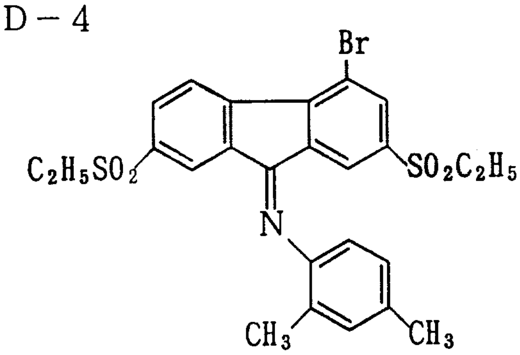

D－5

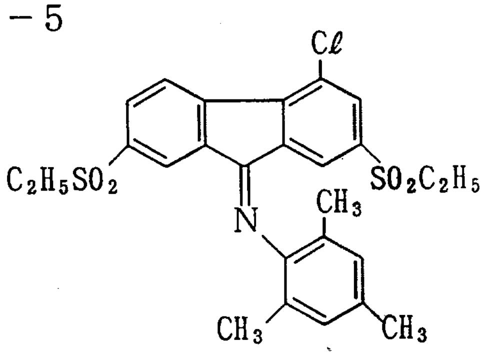

D－6

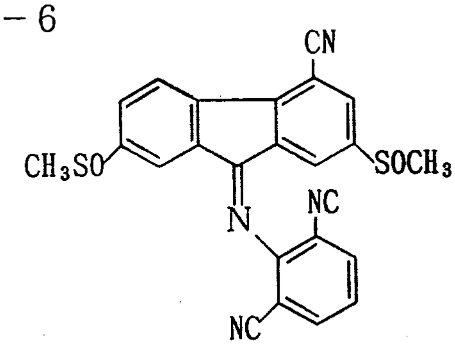

D－7

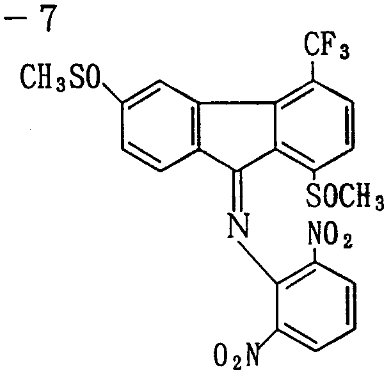

D－8

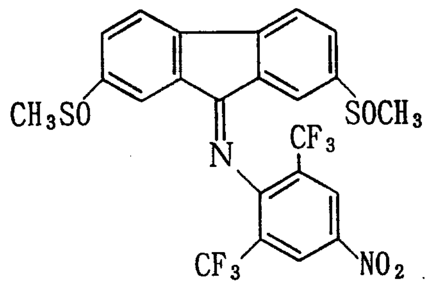

D－9

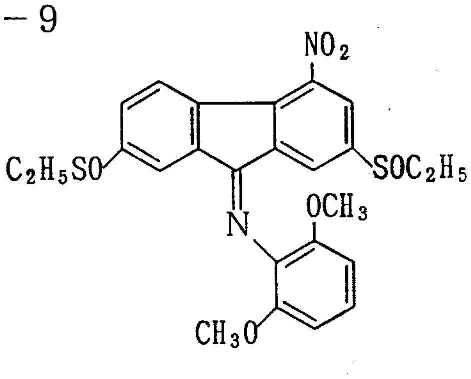

D－10

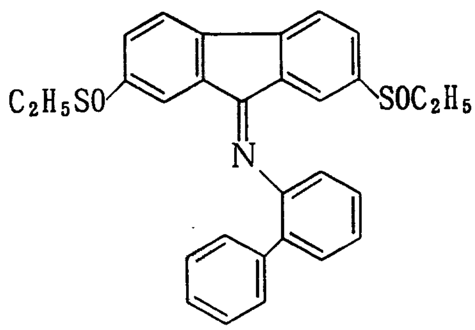

D－11

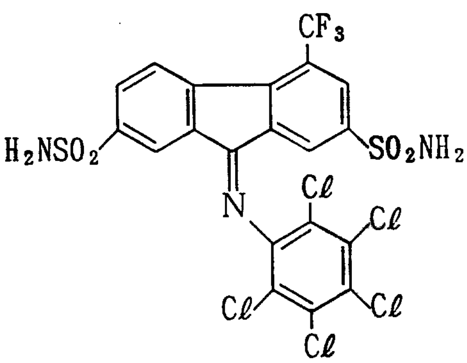

D－12

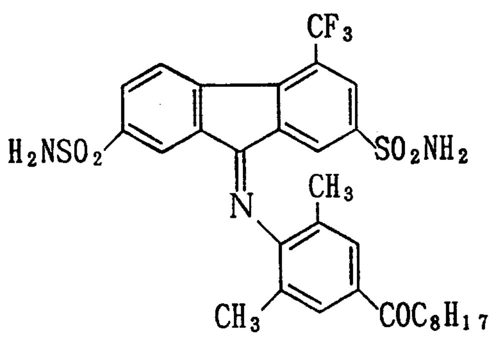

D－13

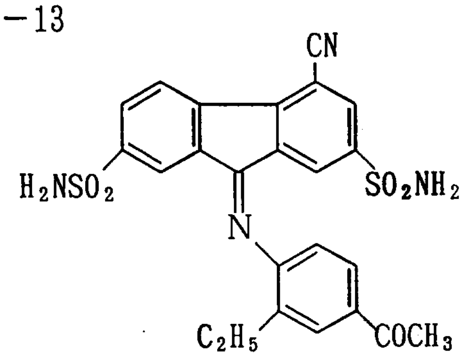

D－14

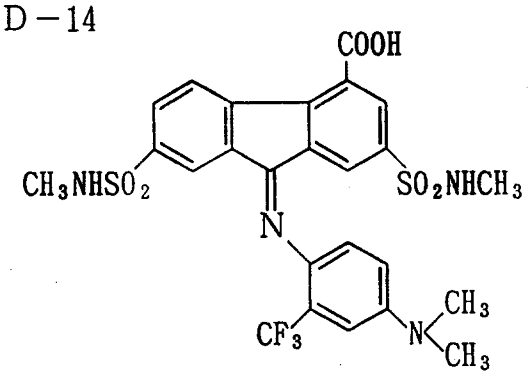

D－15

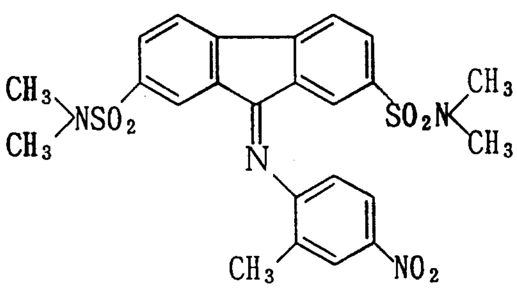

D－16

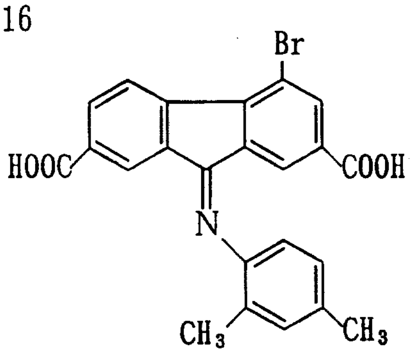

D－17

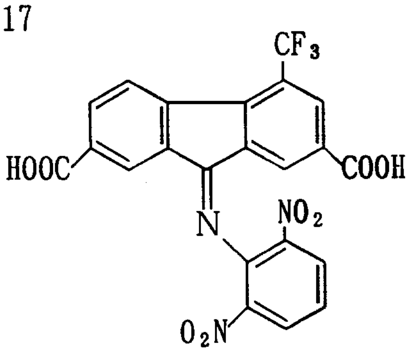

D－18

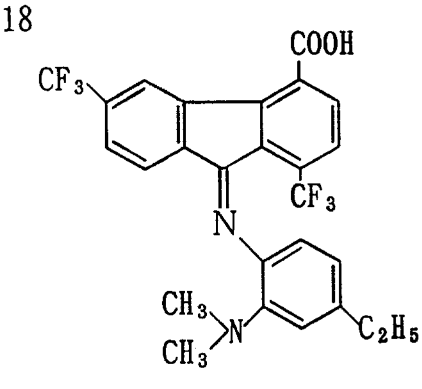

D−19

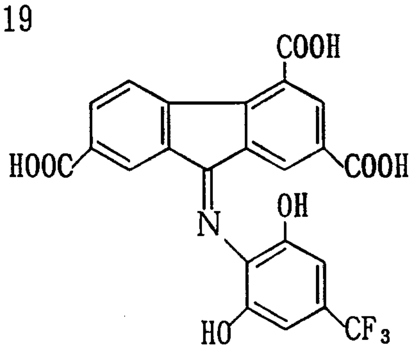

D−20

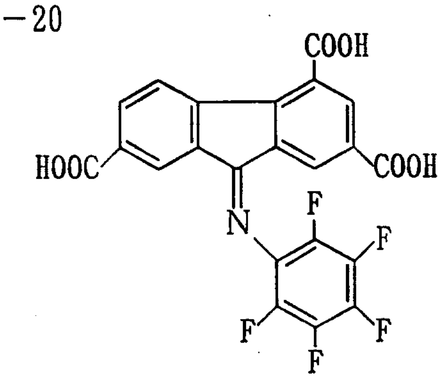

D−21

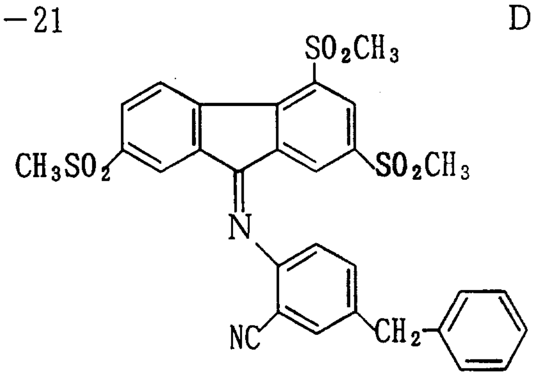

D−22

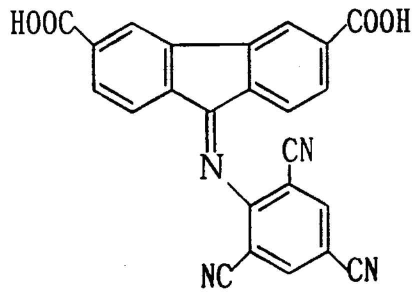

D−23

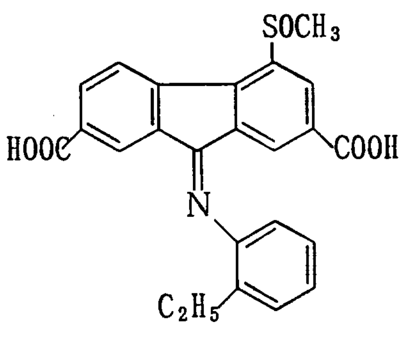

D−24

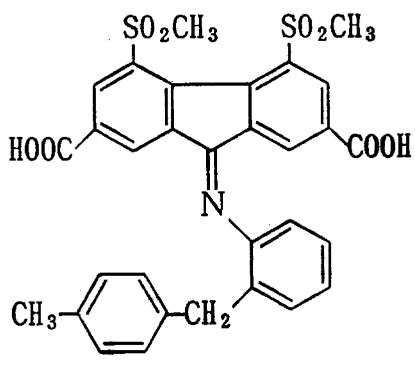

D－25

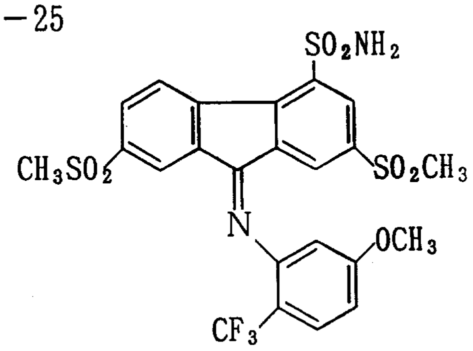

D－26

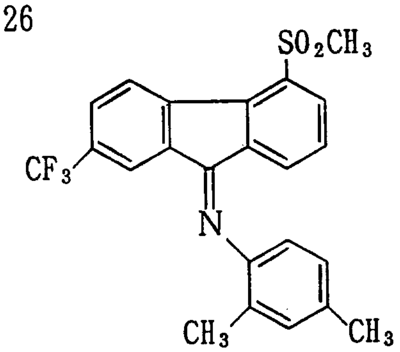

D－27

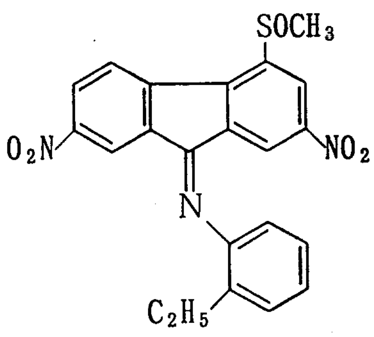

D－28

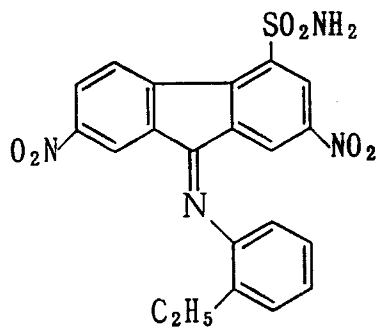

D－29

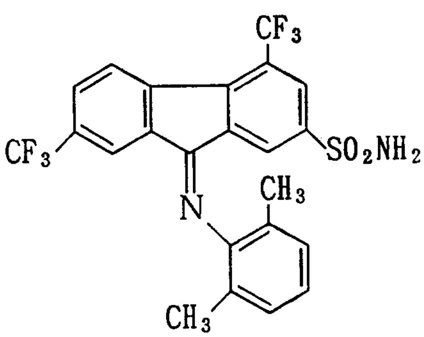

D－30

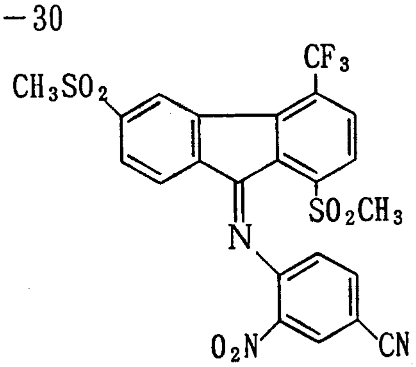

Synthesis Example

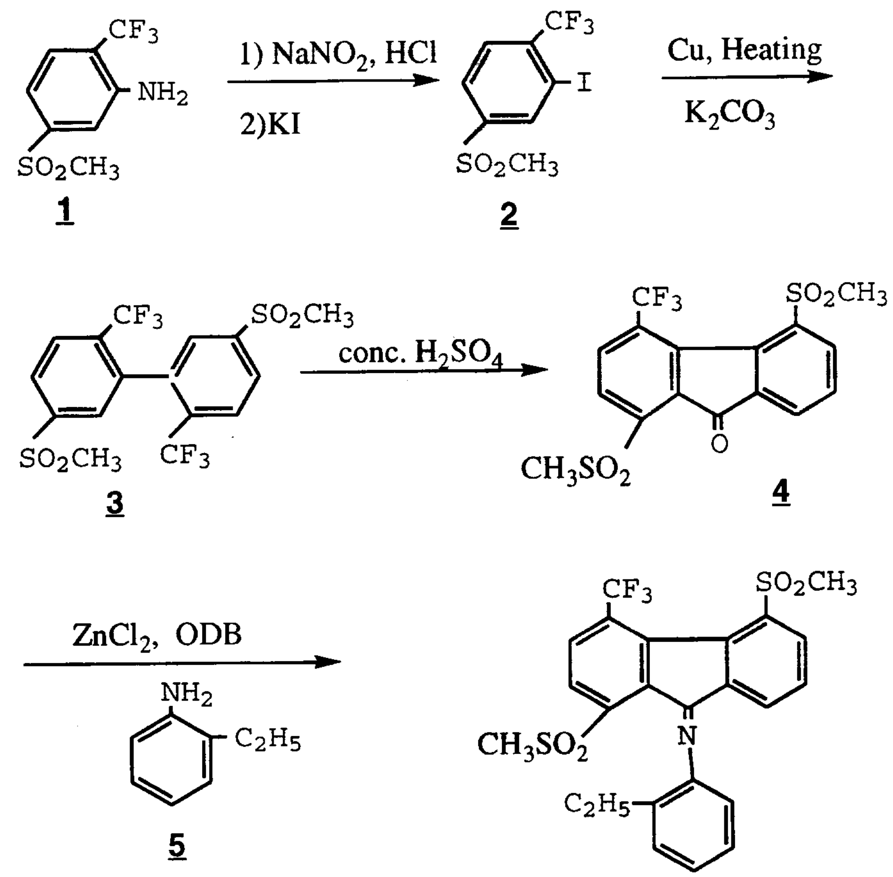


1 2 3 4 5

Exemplified Compound D-2

Exemplified compound D-2 was synthesized by the above reaction route. Compound (4) was synthesized from Compound (1) according to known method (J. Chem. Soc., 1071 - 6 (1954).

In a 100-ml four-necked flask was placed 10 g of the Compound (4), 20 g of zinc chloride, 5 g of ethylaniline (5) (Made by Aldrich Co.) and 100 ml of ODB, and then an esterification tube and a thermometer were attached to the flask, followed by heating under reflux for 120 hours. After cooling the reaction mixture, the solid matter was sucked off and washed well with DMSO. The solvent in the mother liquor was distilled away under reduced pressure, and the residue was subjected to silica gel column chromatography. The dissolved matter in a 9:1 mixture of toluene and n-hexane was crystallized from toluene. Thus, red crystals of exemplified compound D-2 was obtained in a yield of 9.9 g (78 %). Its melting point was 193-194°C when measured by the method of measuring meting point specified in the Japanese Pharmacopoeia. Since the results obtained by elemental analysis also agreed well with the calculated values, the product was identified as exemplified compound D-2.

| | C | H | N |
|---|---|---|---|
| Calculated Values | 56.80% | 3.97% | 2.76% |
| Measured Values | 56.62% | 4.01% | 2.51% |

(E) Compound represented by Formula E

Example of Compound

E – 1

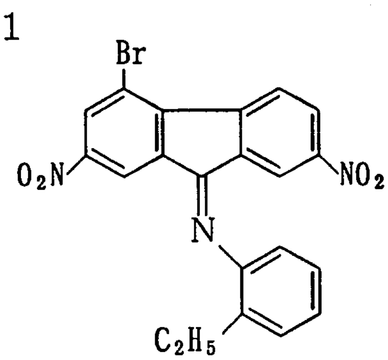

E – 2

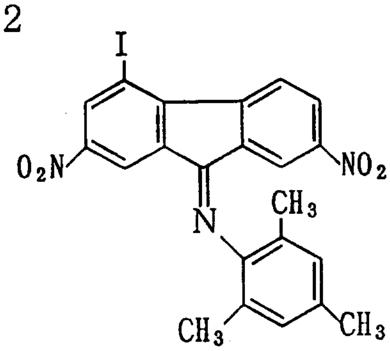

E – 3

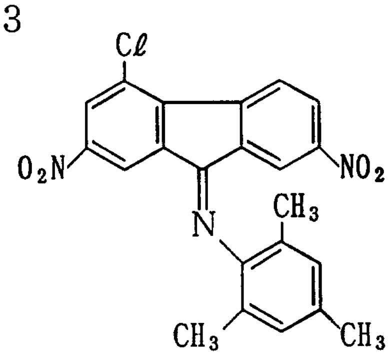

E – 4

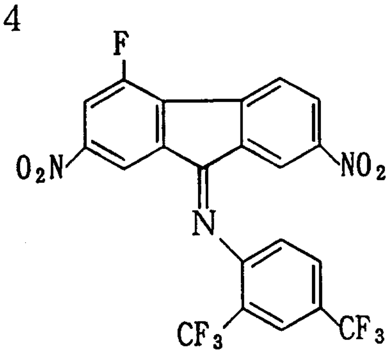

E – 5

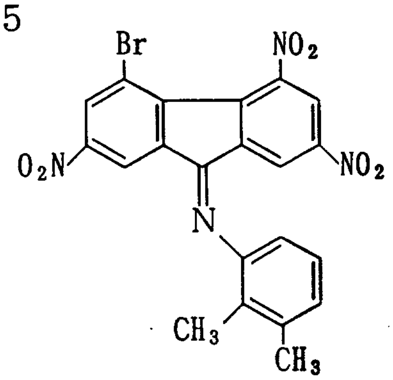

E – 6

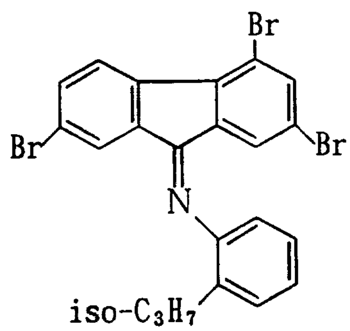

E－7

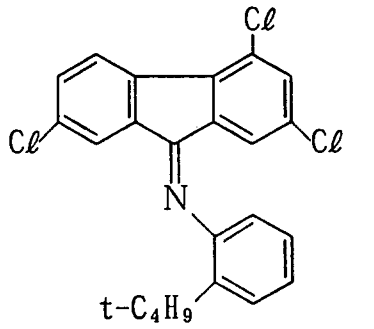

E－8

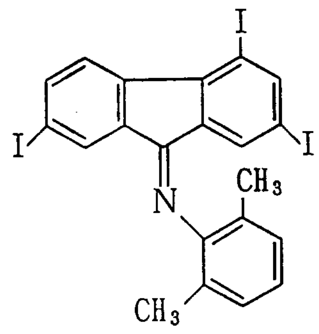

E－9

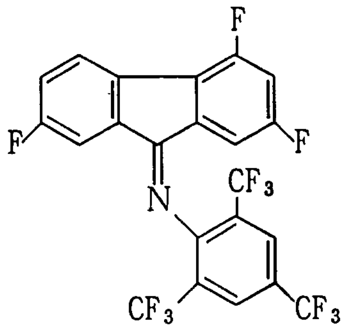

E－10

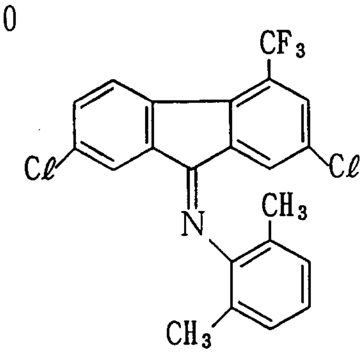

E－11

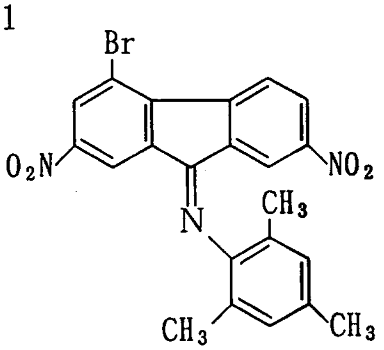

E－12

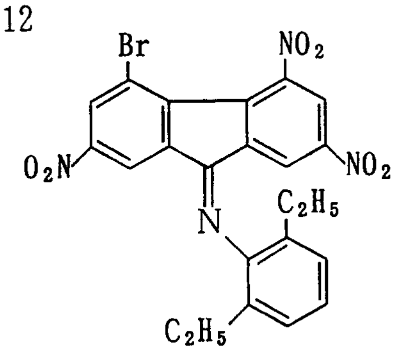

E－13

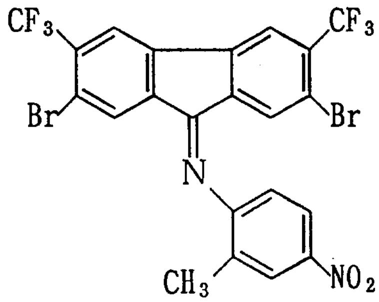

E－14

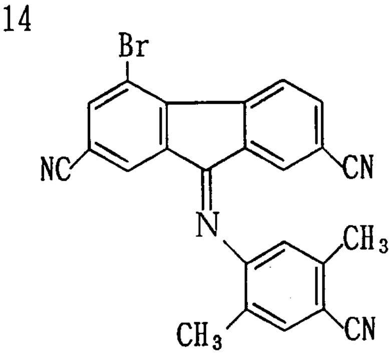

E－15

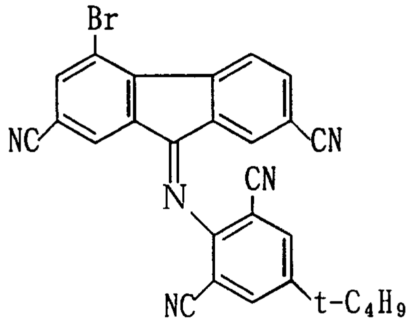

E－16

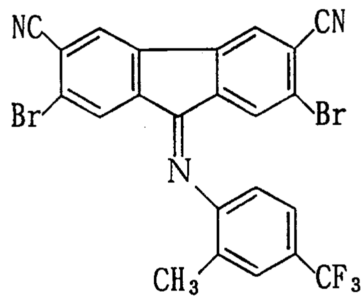

E－17

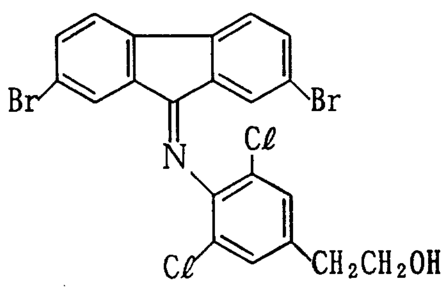

E－18

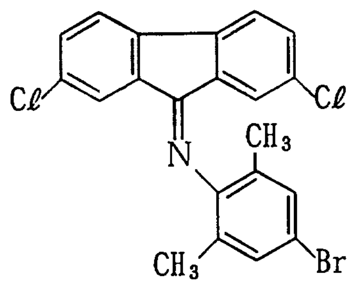

E－19

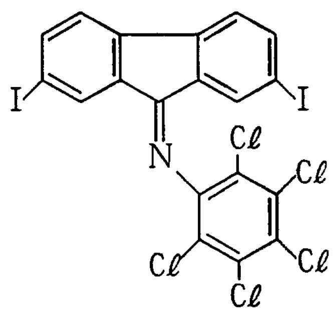

E－20

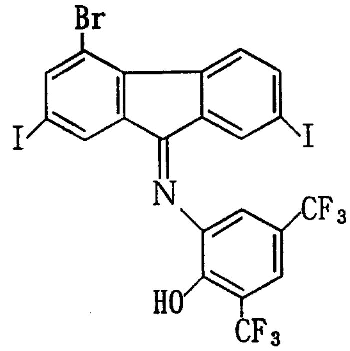

E－21

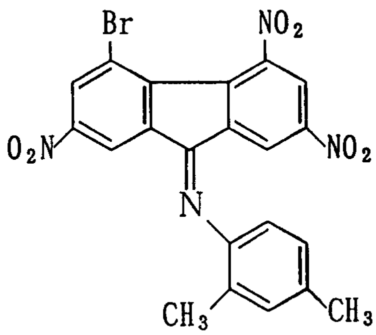

E－22

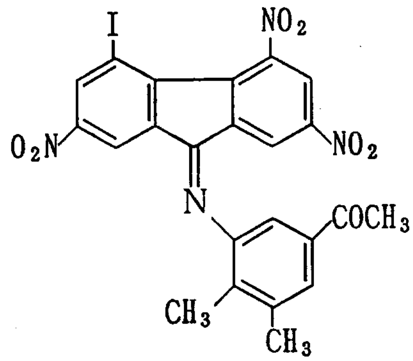

E－23

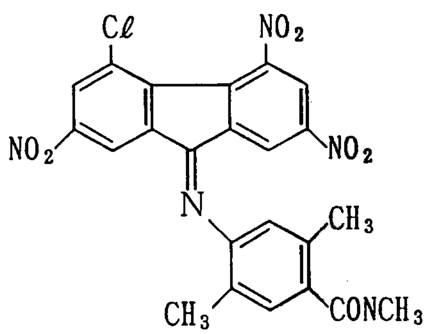

E－24

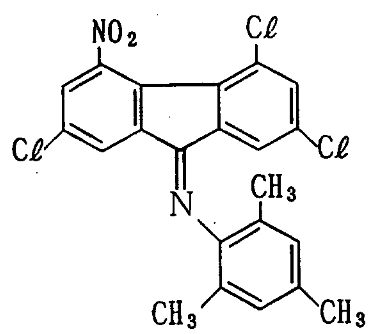

E－25

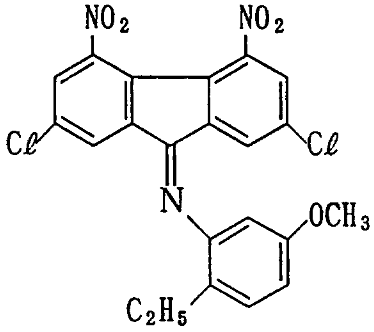

E－26

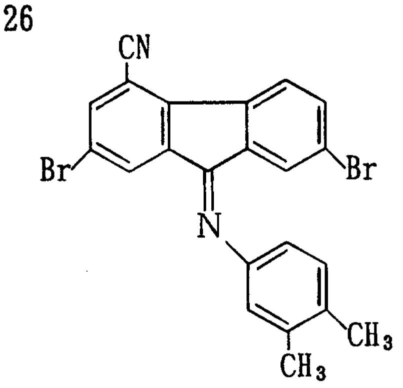

E－27

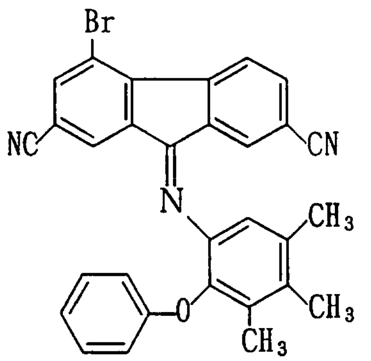

E－28

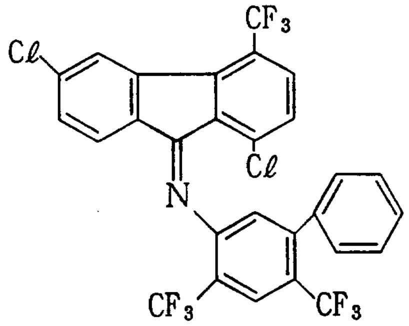

E－29

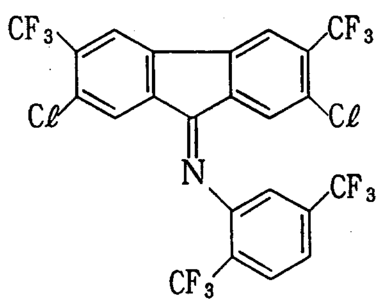

E－30

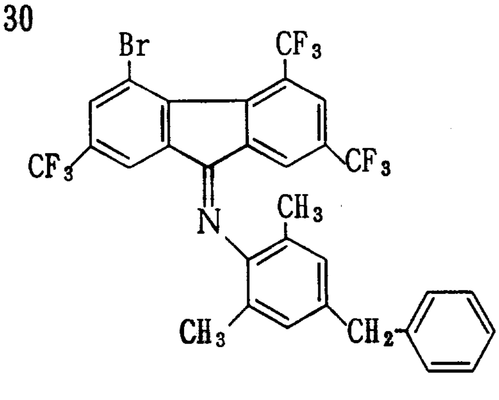

Synthesis Example

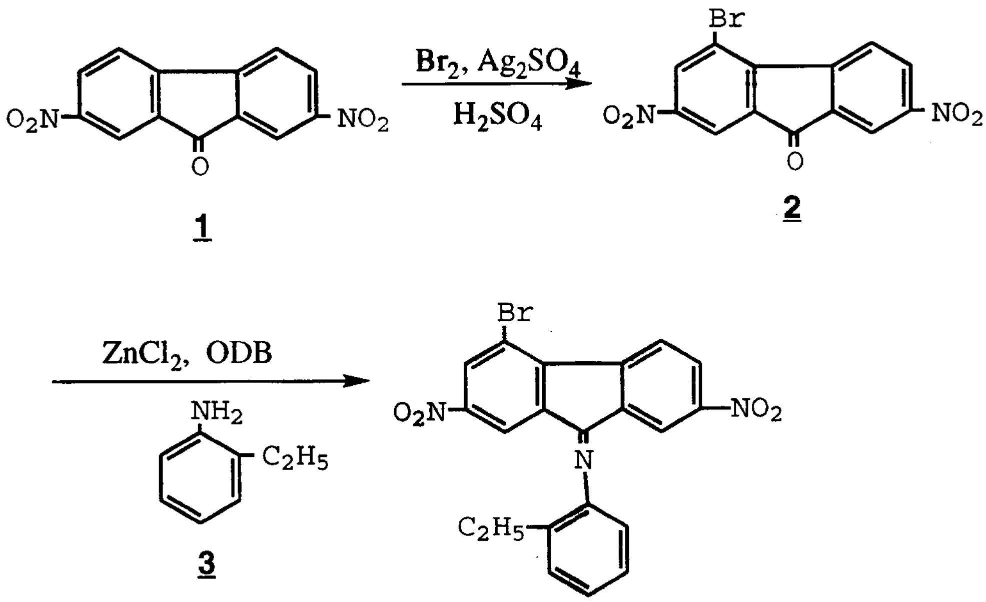


1

2

3

Exemplified Compound E-1

Exemplified compound E-1 was synthesized according to the above reaction route. Compound (2) was synthesized from 2,7-dinitrofluorenone (Made by Aldrich Co.) according to known method (JA L.O.P 1-290654).

In a 100-ml four-necked flask was placed 10 g of the Compound (2), 20 g of zinc chloride, 5 g of 2-ethylaniline (3) (Made by Aldrich Co.) and 100 ml of ODB, and then an esterification tube and a thermometer were attached to the flask, followed by heating under reflux for 60 hours. After cooling the reaction mixture, the solid matter was sucked off and washed well with ethyl acetate. The solvent in the mother liquor was distilled away under reduced pressure, and the residue was subjected to silica gel column chromatography. The dissolved matter in a 1:1 mixture of toluene and n-hexane was crystallized from n-hexane. Thus, red crystals of exemplified compound E-1 was obtained in a yield of 6.8 g (52 %). Its melting point was 176-178 °C when measured by the method of measuring meting point specified in the Japanese Pharmacopoeia. Since the results obtained by elemental analysis also agreed well with the calculated values, the product was identified as exemplified compound E-1.

| | C | H | N |
|---|---|---|---|
| Calculated Values | 55.77% | 3.12% | 9.29% |
| Measured Values | 55.72% | 3.14% | 9.10% |

(F) Compound represented by Formula F

Example of Compound

F－1

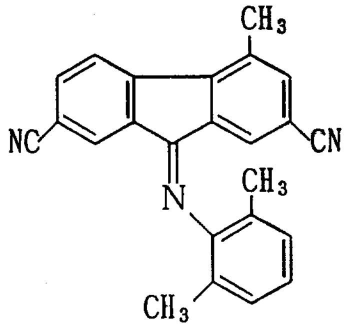

F－2

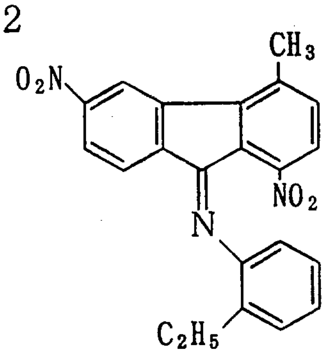

F－3

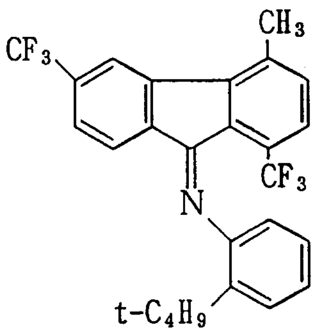

F－4

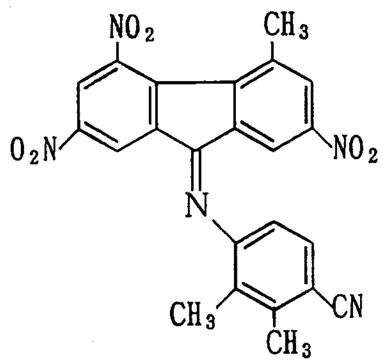

F－5

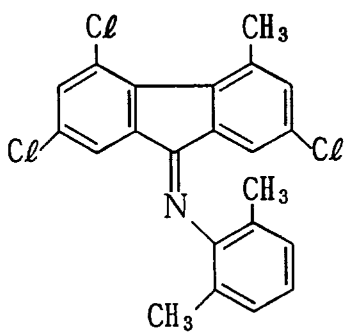

F－6

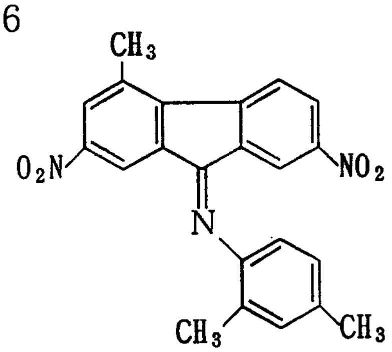

F－7

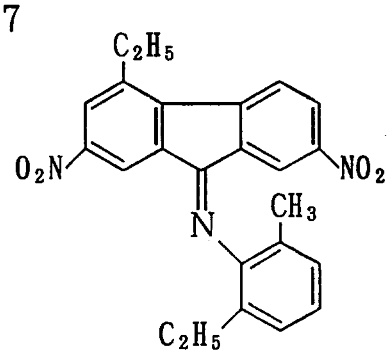

F－8

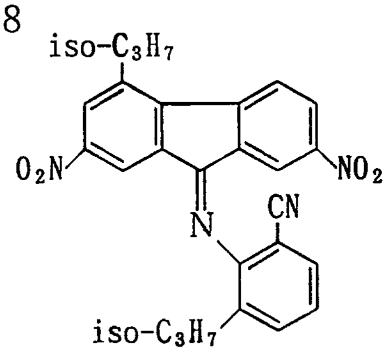

F－9

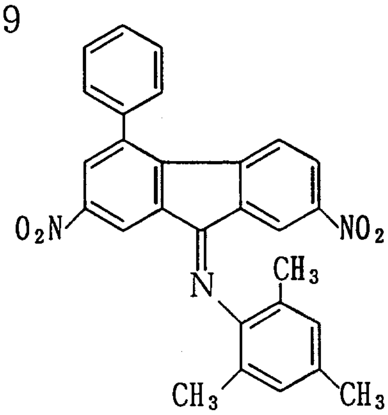

F－10

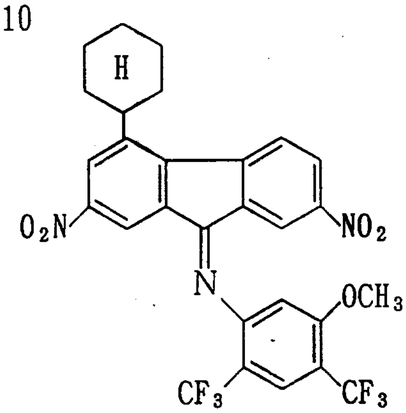

F－11

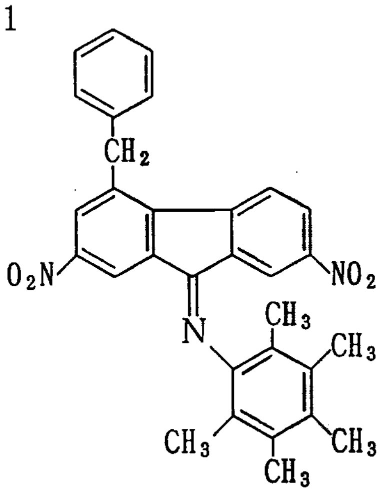

F－12

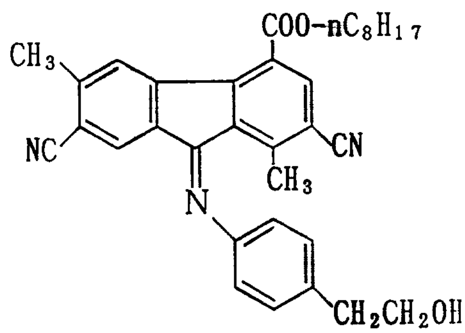

F－13

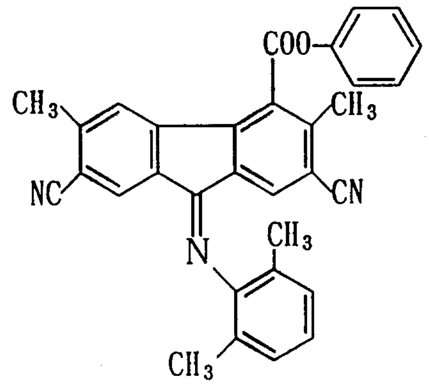

F－14

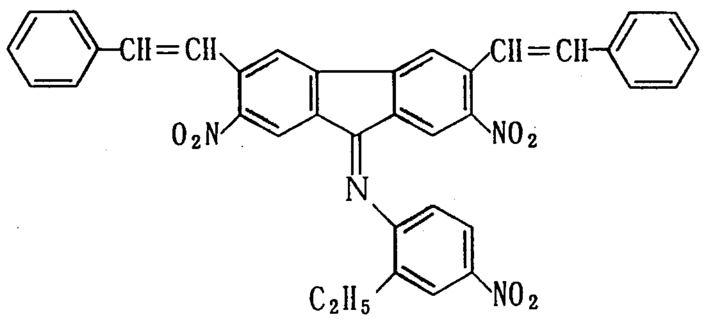

F－15

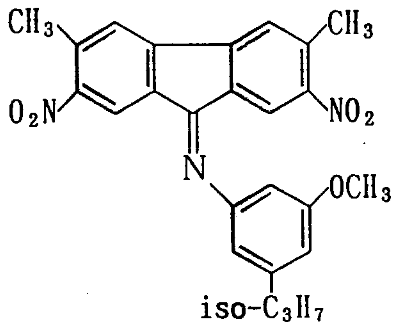

F－16

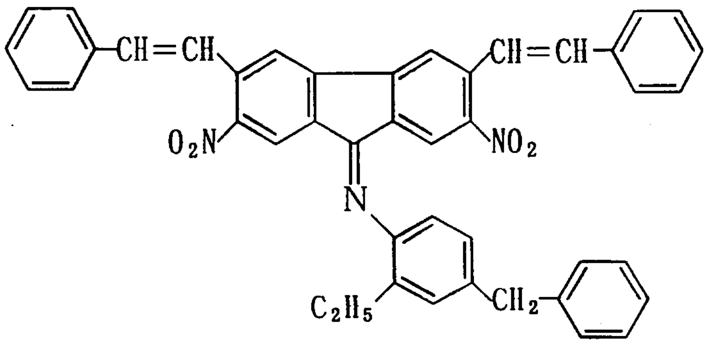

F－17

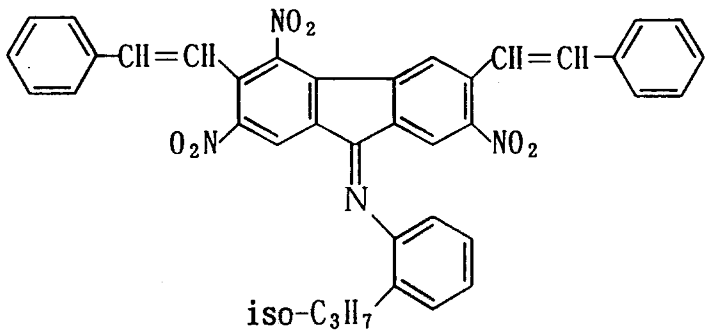

F－18

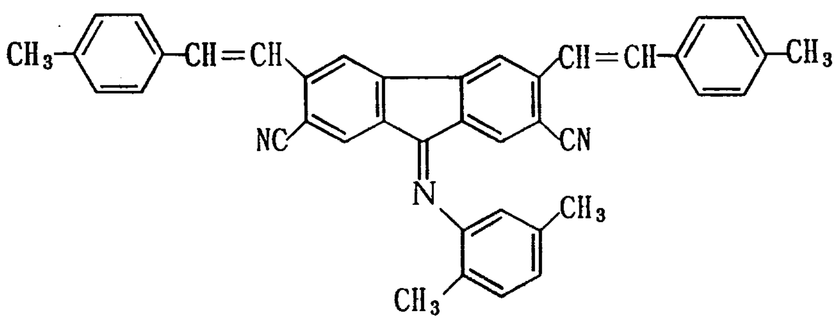

F－19

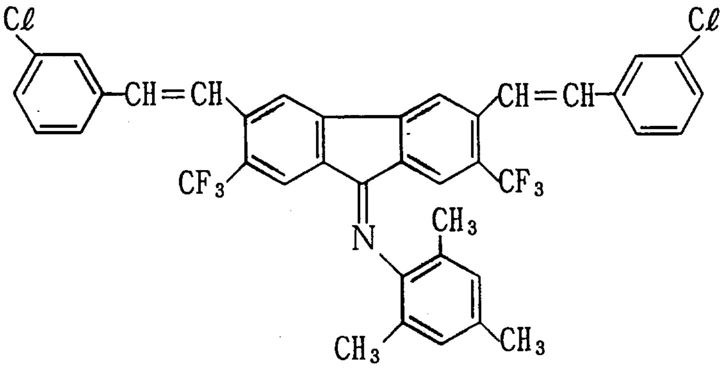

F－20

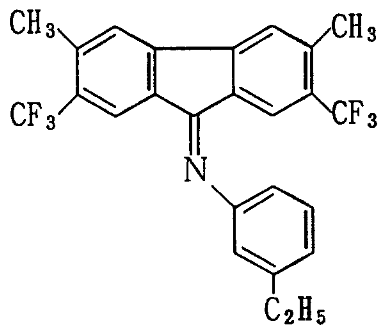

F－21

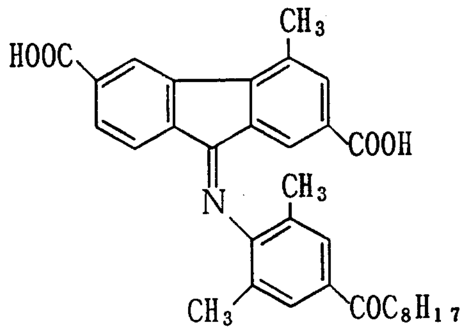

F－22

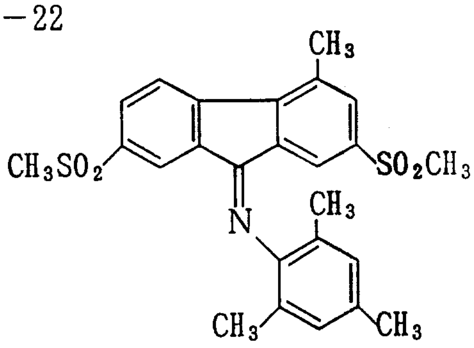

F－23

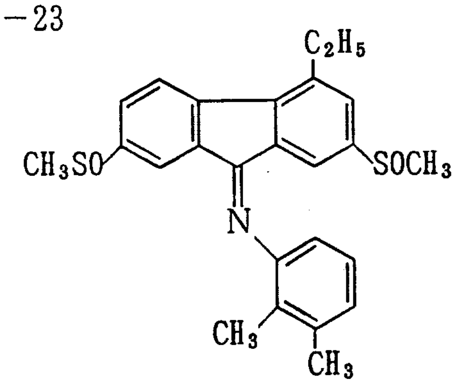

F－24

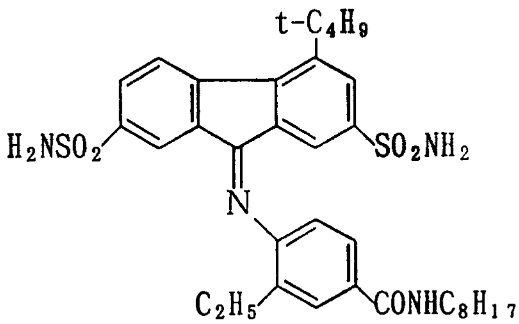

F－25

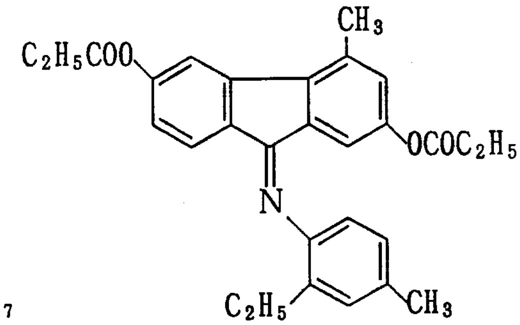

F－26

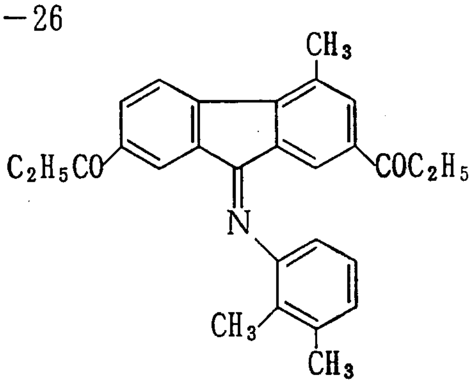

F－27

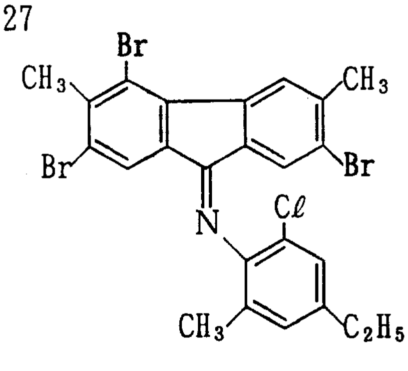

F－28

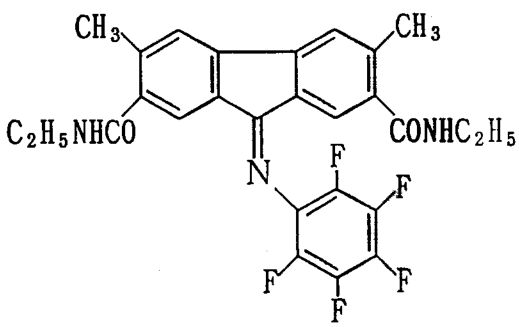

F－29

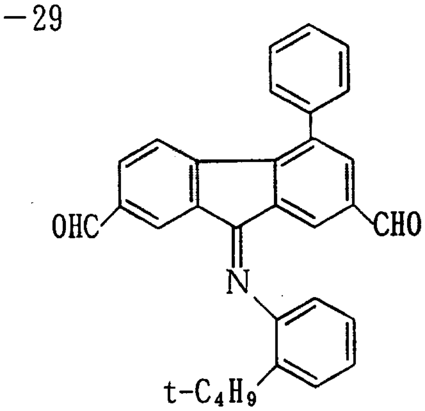

F－30

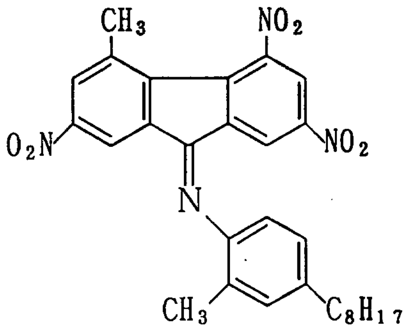

F－31

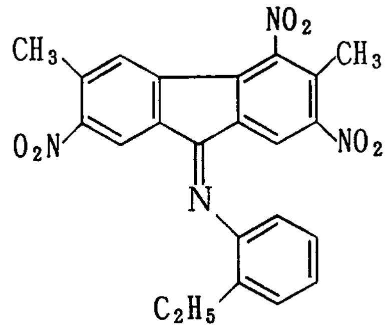

F－32

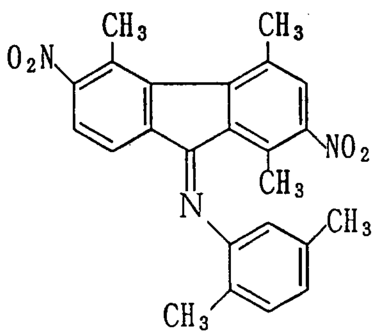

F－33

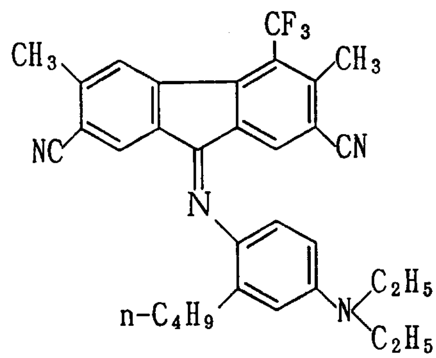

F－34

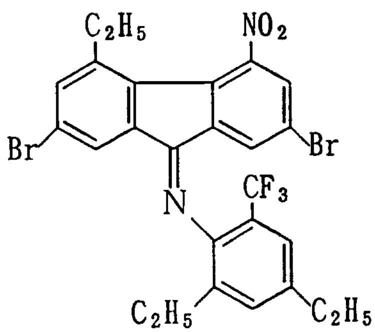

F－35

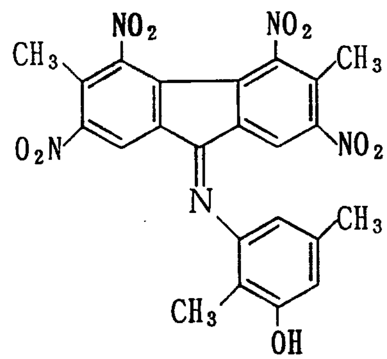

Synthesis Example

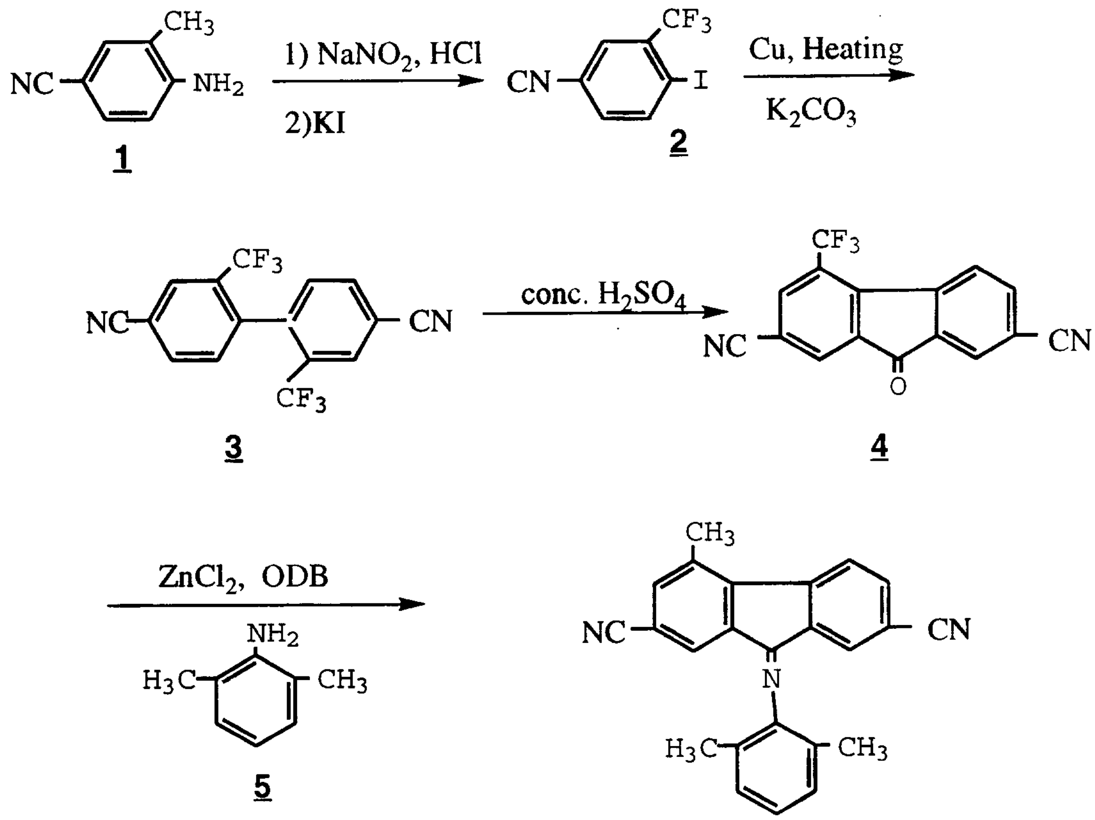


1 2 3 4 5

Exemplified Compound F-1

Exemplified compound F-1 was synthesized according to the above reaction route. Compound (4) was synthesized from 3-methyl-4-aminobenzonitril according to known method (J. Chem. Soc., 1071 - 6 (1954)).

In a 100-ml four-necked flask was placed 10 g of the Compound (4), 20 g of zinc chloride, 5 g of 2,6-xylidine (5) (Made by Wako Jun-yaku Co., Ltd.) and 100 ml of ODB, and then an esterification tube and a thermometer were attached to the flask, followed by heating under reflux for 75 hours. After cooling the reaction mixture, the solid matter was sucked off and washed well with ethyl acetate. The solvent in the mother liquor was distilled away under reduced pressure, and the residue was subjected to silica gel column chromatography. The dissolved matter in a 1:1 mixture of toluene and n-hexane was crystallized from n-hexane. Thus, red crystals of exemplified compound F-1 was obtained in a yield of 11.9 g (84 %). Its melting point was 159-160 ° C when measured by the method of measuring meting point specified in the Japanese Pharmacopoeia. Since the results obtained by elemental analysis also agreed well with the calculated values, the product was identified as exemplified compound F-1.

| | C | H | N |
|---|---|---|---|
| Calculated values | 82.97% | 4.93% | 12.09% |
| Measured Values | 82.93% | 4.95% | 12.03% |

(G) Compound represented by formula G

Example of Compound

G−1

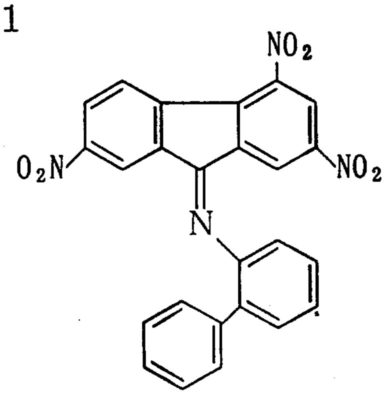

G−2

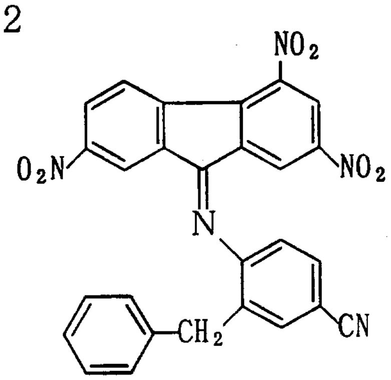

G−3

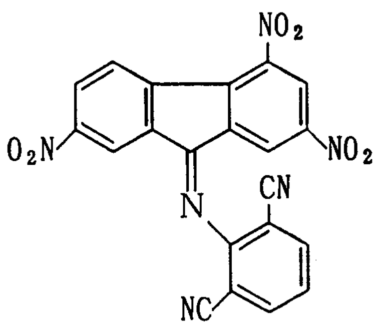

G−4

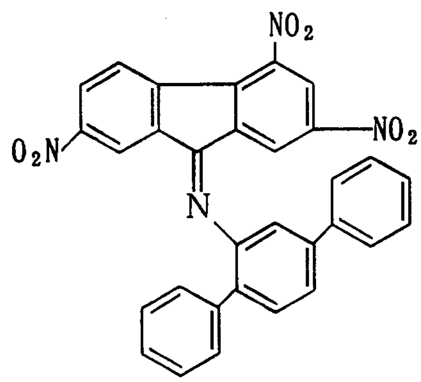

G−5

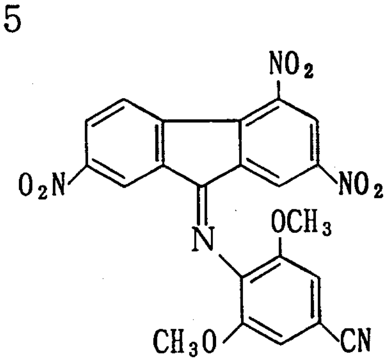

G−6

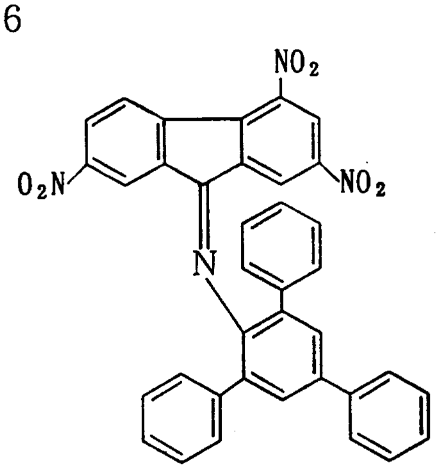

G－7

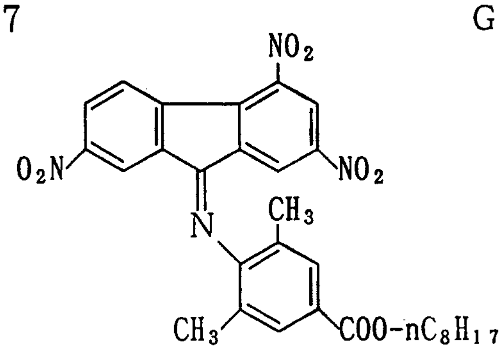

G－8

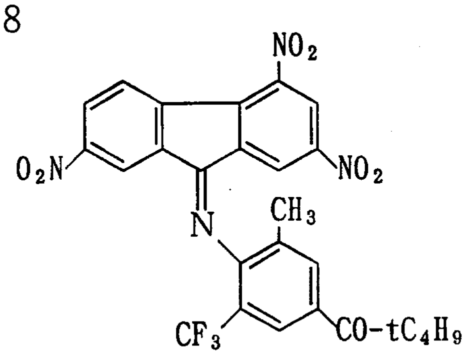

G－9

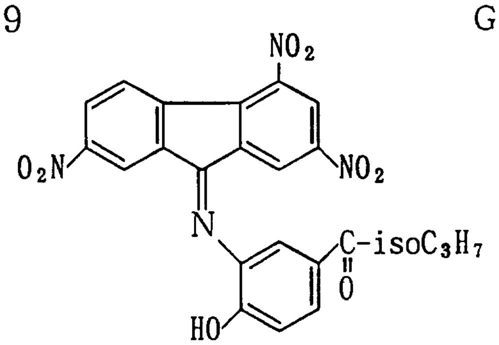

G－10

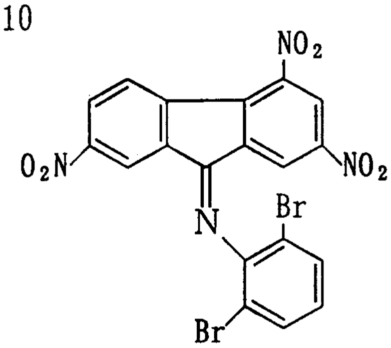

G－11

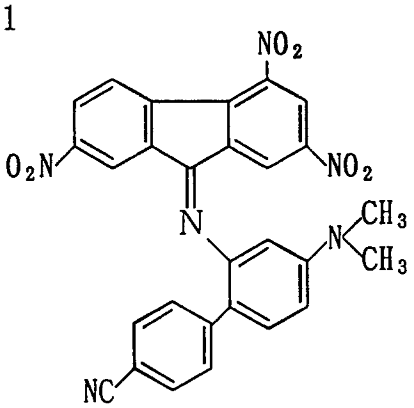

G－12

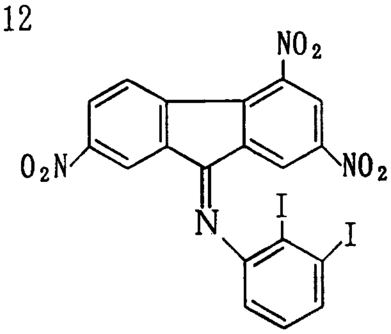

G−13

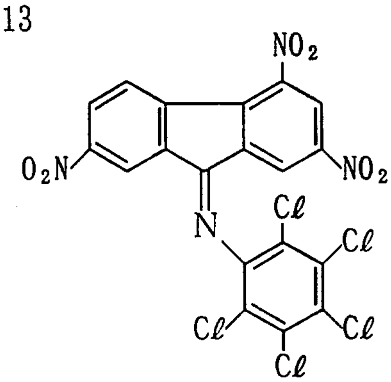

G−14

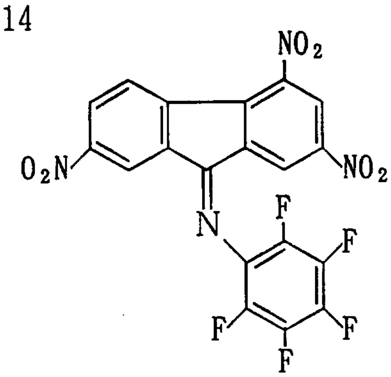

G−15

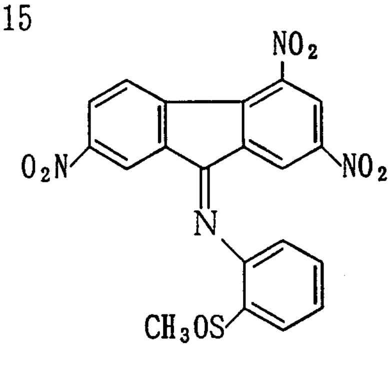

G−16

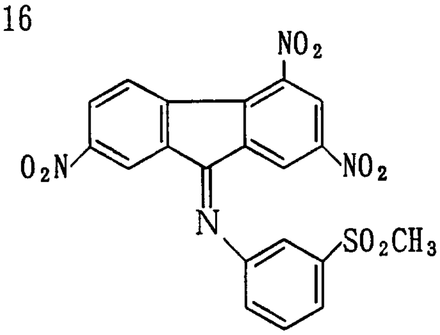

G−17

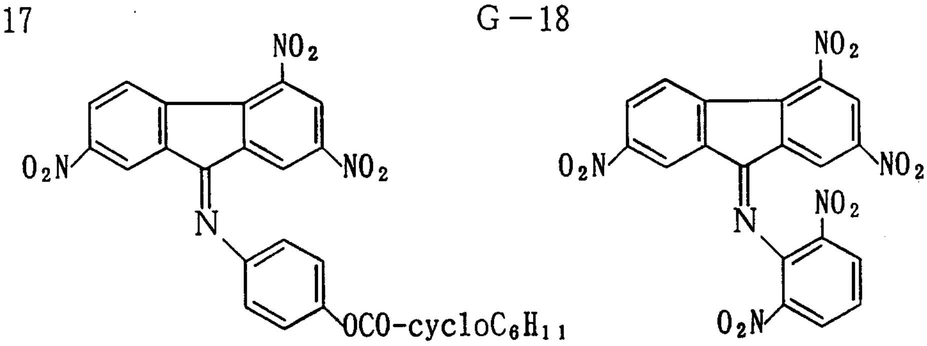

G−18

G−19

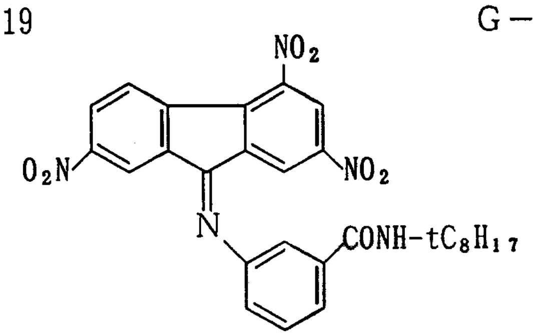

G−20

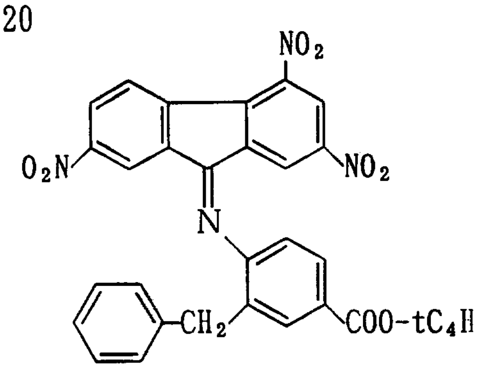

G−21

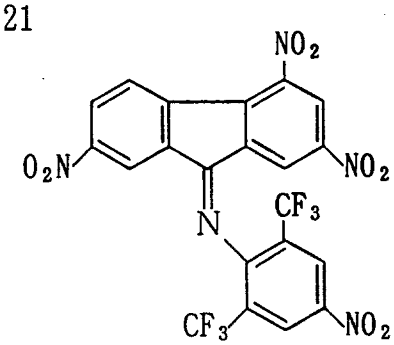

G−22

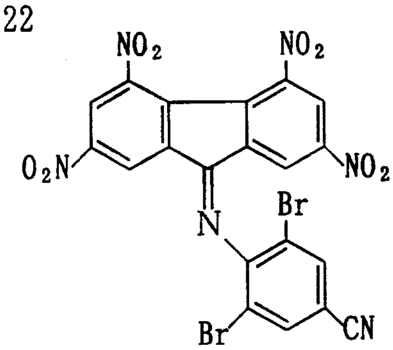

G−23

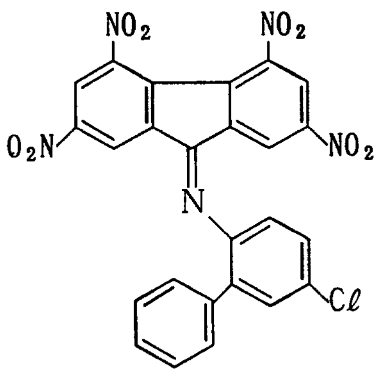

G−24

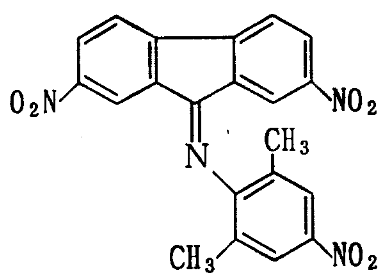

G－25

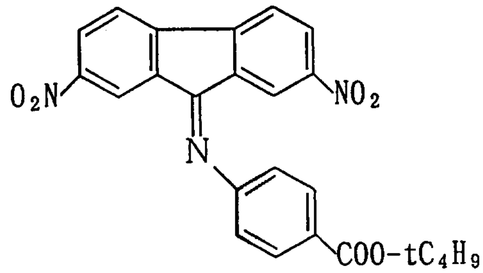

G－26

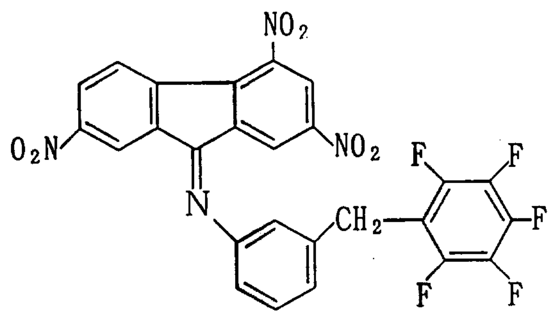

G－27

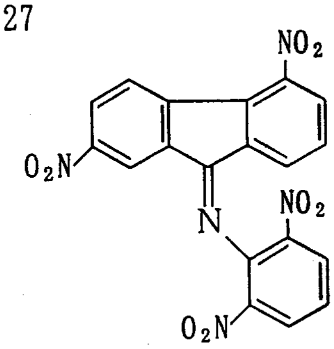

G－28

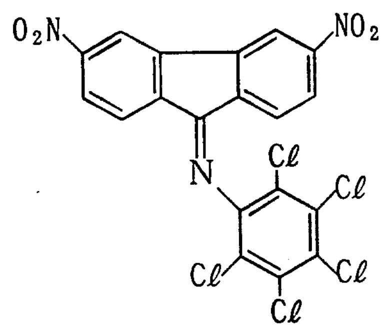

G－29

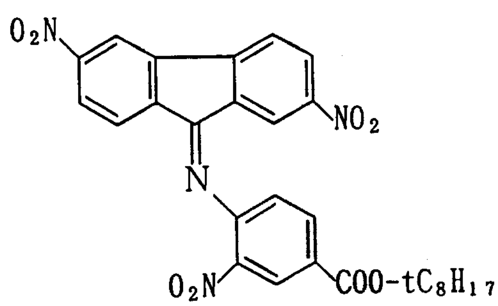

G－30

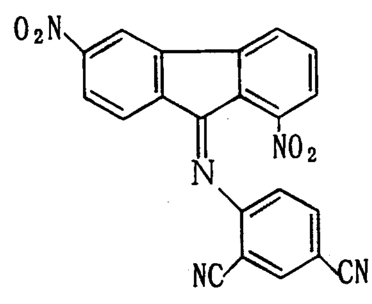

Synthesis Example

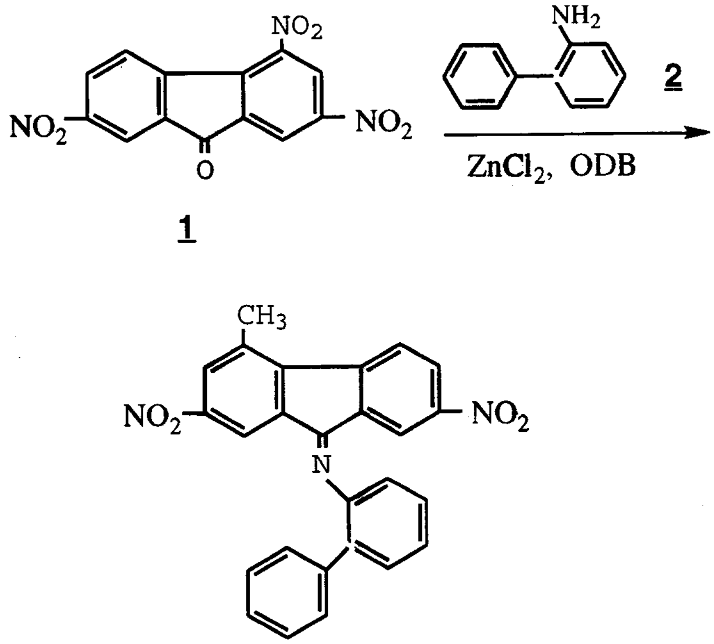


Exemplified Compound G-1

Exemplified compound G-1 was synthesized according to the above reaction route.

In a 100-ml four-necked flask was placed 10 g of 2,4,7-trinitrofluorenone(1) (Made by Tokyo Kasei Co., Ltd.), 20 g of zinc chloride, 2-aminobiphenyl (2) (Made by Aldrich) and 100 ml of ODB, and then an esterification tube and a thermometer were attached to the flask, followed by heating under reflux for 80 hours. After cooling the reaction mixture, the solid matter was sucked off and washed well with ethyl acetate. The solvent in the mother liquor was distilled away under reduced pressure, and the residue was subjected to silica gel column chromatography. The dissolved matter in a 1:1 mixture of toluene and n-hexane was crystallized from toluene. Thus, red crystals of exemplified compound G-1 was obtained in a yield of 12.1 g (81 %). Its melting point was 181-183 °C when measured by the method of measuring meting point specified in the Japanese Pharmacopoeia. Since the results obtained by elemental analysis also agreed well with the calculated values, the product was identified as exemplified compound G-1.

| | C | H | N |
|---|---|---|---|
| Calculated Values | 64.38% | 3.03% | 12.01% |
| Measured Values | 64.21% | 3.07% | 11.92% |

(I) Compounds Represented by Formula H

Example of Compounds:

H－1

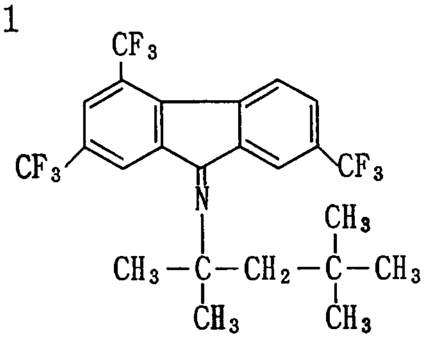

H－2

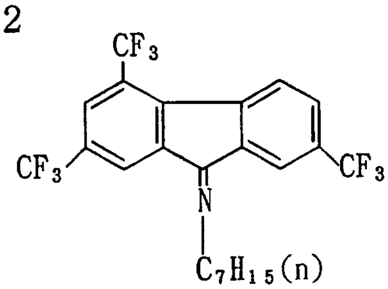

H－3

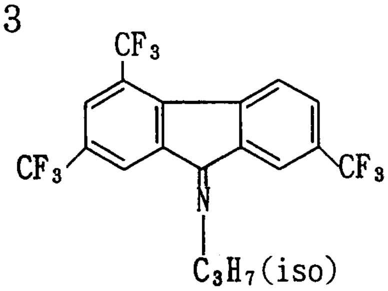

H－4

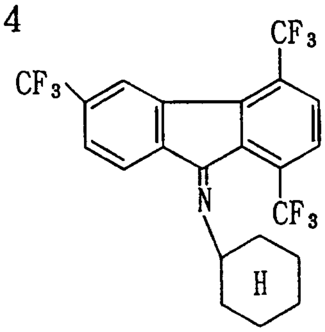

H－5

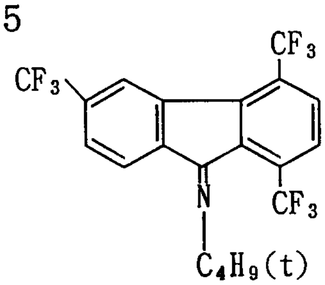

H－6

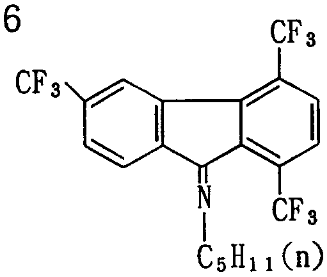

H－7

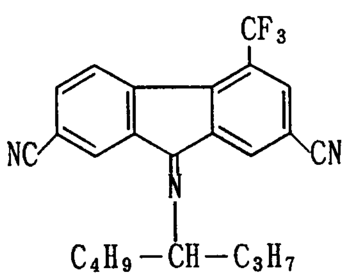

H－8

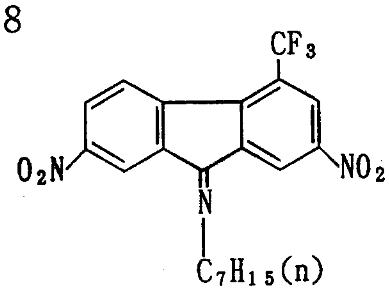

H -9

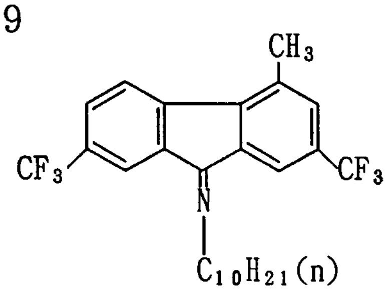

H -10

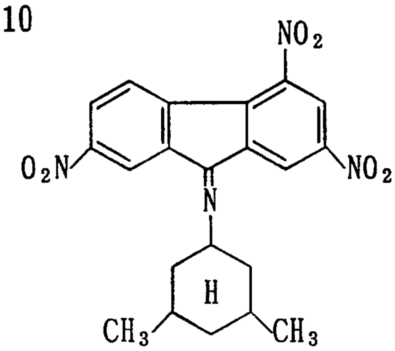

H -11

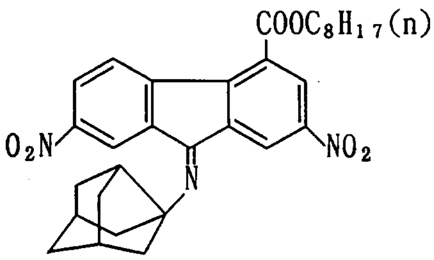

H -12

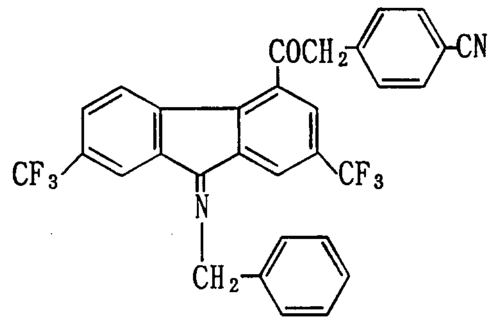

H -13

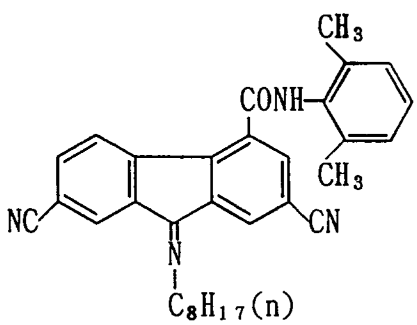

H -14

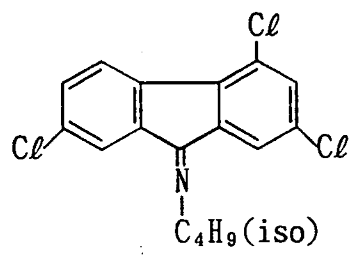

H -15

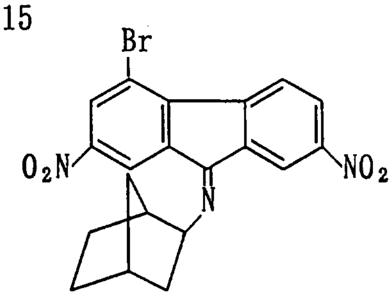

H -16

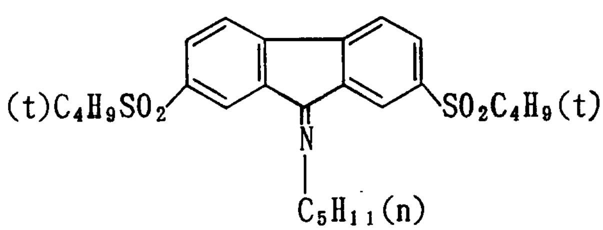

H −17

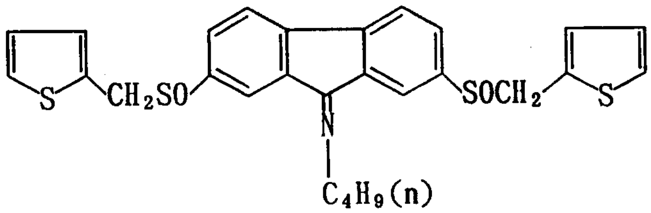

H −18

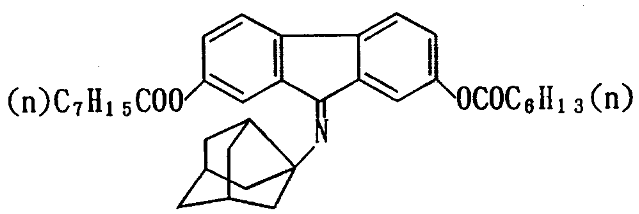

H −19

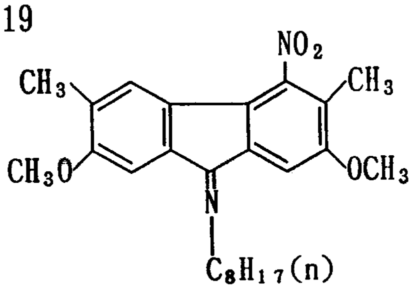

H −20

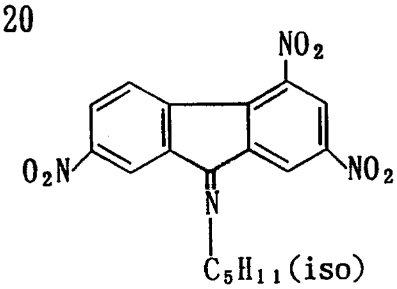

H −21

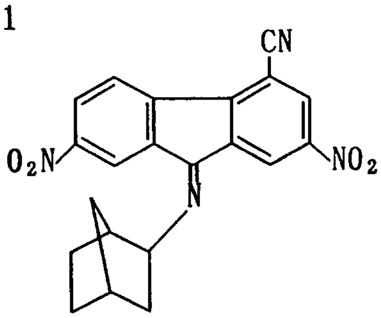

H −22

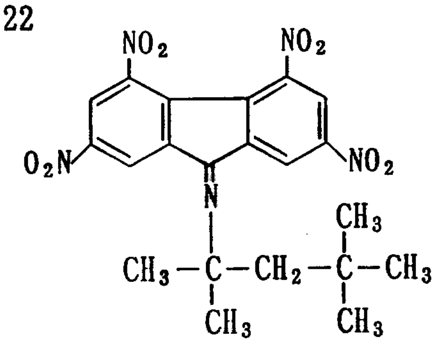

H －23

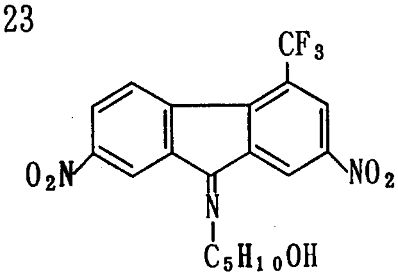

H －24

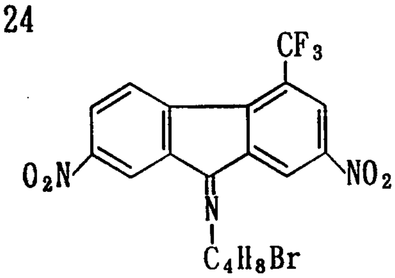

H －25

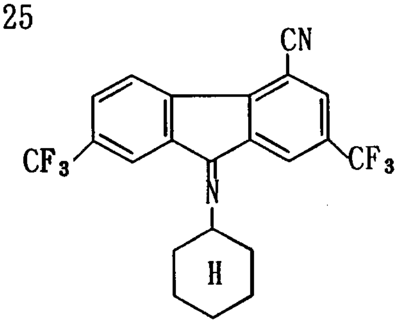

H －26

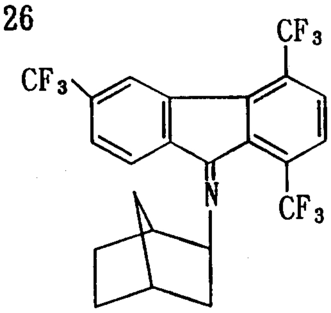

H －27

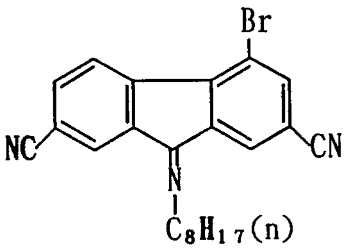

H －28

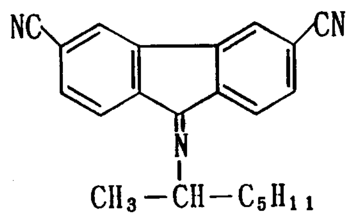

H －29

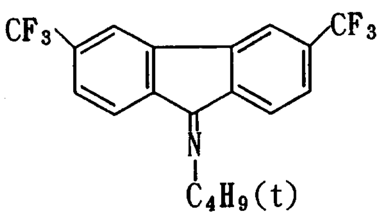

H －30

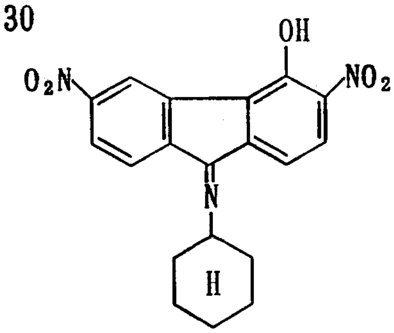

Synthesis Example

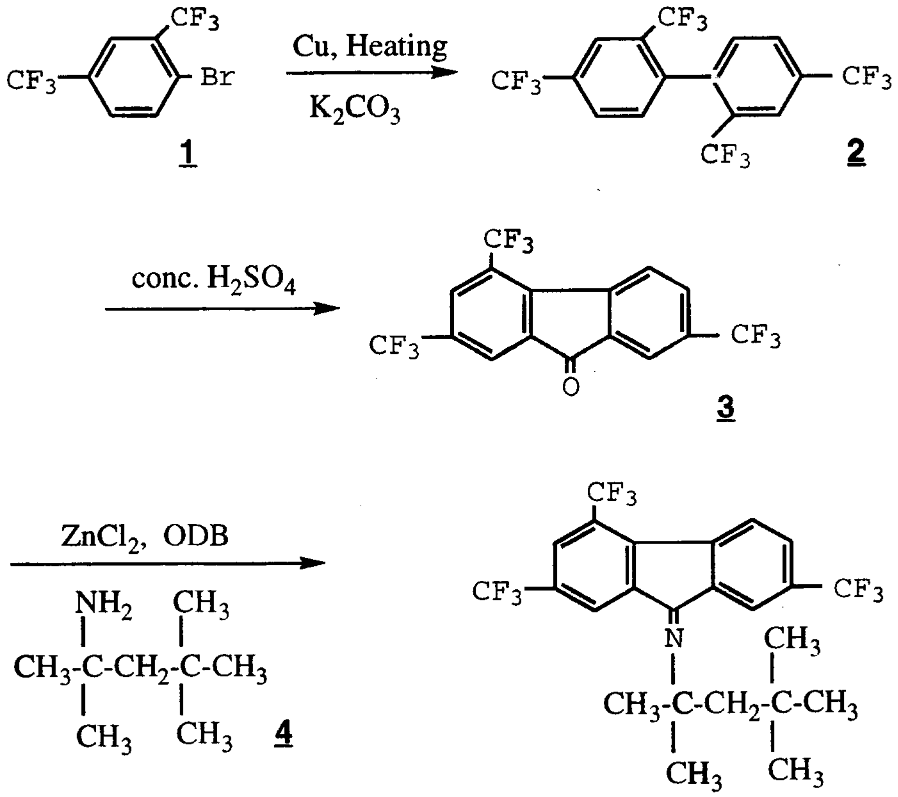


Exemplified Compound H-1

Exemplified Compound H-1 is synthesized according to the synthesis route. Compound (3) was synthesized according to a method known by J. Chem. Soc., 1071 - 6 (1954), from 2,4-bis(trifluoromethyl)-bromobenzene (1) (Made by Aldrich Co. ) as a starting material.

In a 100-ml four-necked flask was placed 10 g of Compound (3), 5 g of t-octylamine (4), 20 g of zinc chloride, and 100 ml of o-chlorobenzen (hereafter called as ODB), and then an esterification tube and a thermometer were attached to the flask, followed by heating under reflux for 55 hours. After cooling the reaction mixture, the solid matter was sucked off and washed well with ethyl acetate. The solvent in the mother liquor was distilled away under reduced pressure, and the residue was subjected to silica gel column chromatography. The dissolved matter in a 1:1 mixture of toluene and n-hexane was crystallized from n-hexane. Thus, red powder of exemplified compound H-1 was obtained in a yield of 7.9 g (61%). Its melting point was 171-173 °C when measured by the method of measuring meting point specified in the Japanese Pharmacopoeia. Since the results obtained by elemental analysis also agreed well with the calculated values, the product was identified as exemplified compound H-1.

| | C | H | N |
|---|---|---|---|
| Calculated Values | 58.18% | 4.48% | 2.83% |
| Measured Values | 57.97% | 4.55% | 2.78% |

(I) Compounds represented by Formula I

Example of Compound

I - 1

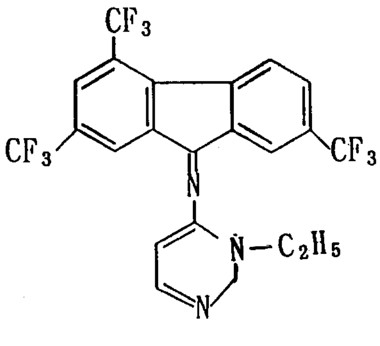

I - 2

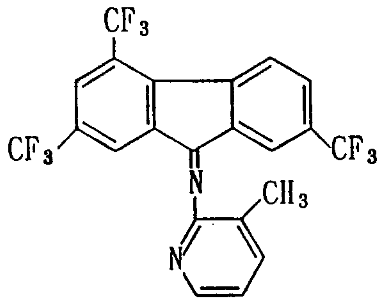

I - 3

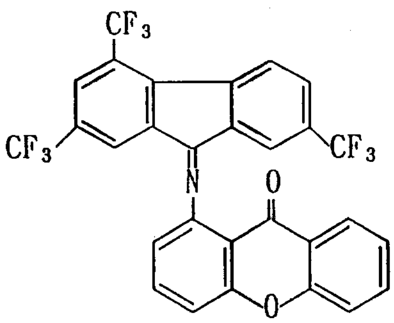

I - 4

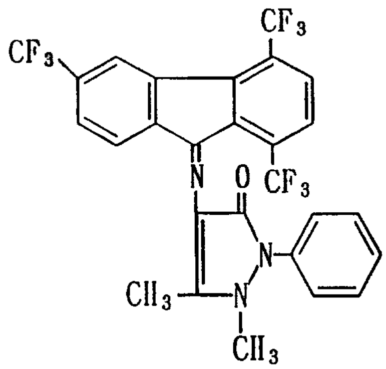

I - 5

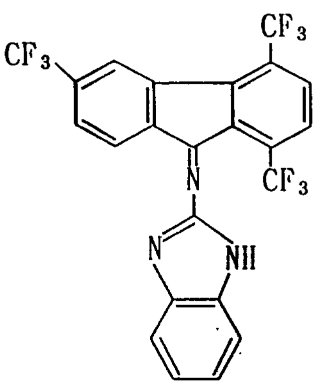

I - 6

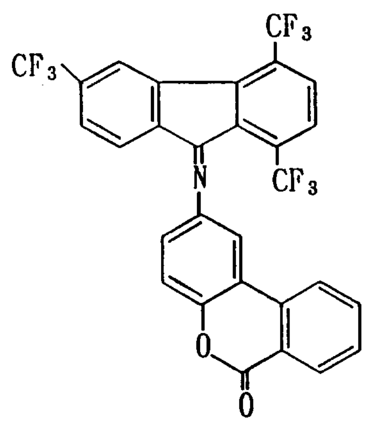

I －7

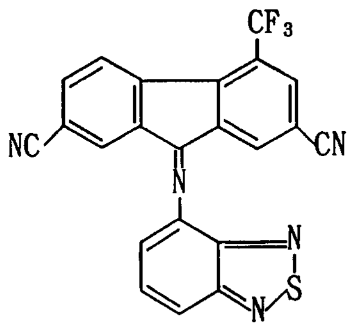

I －8

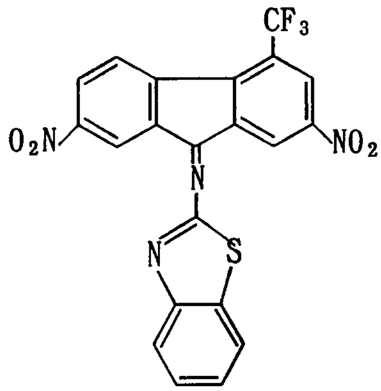

I －9

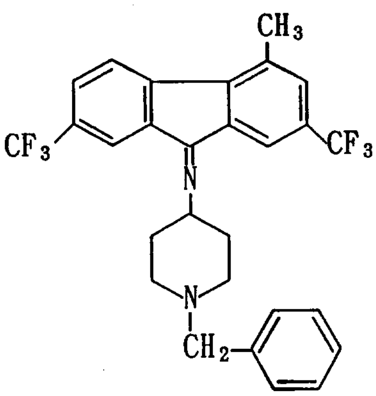

I －10

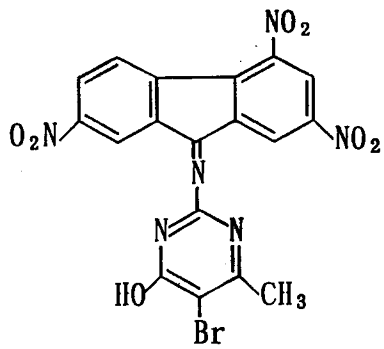

I －11

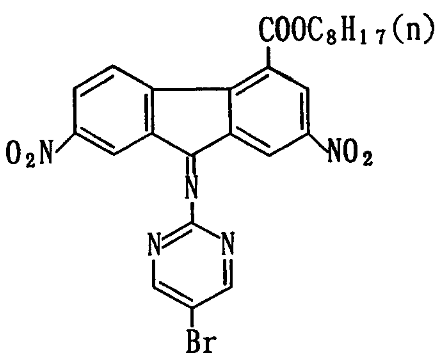

I －12

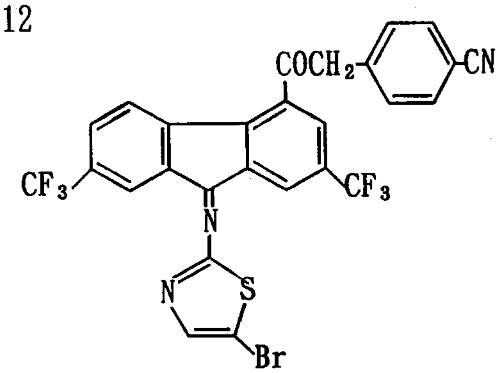

I −13

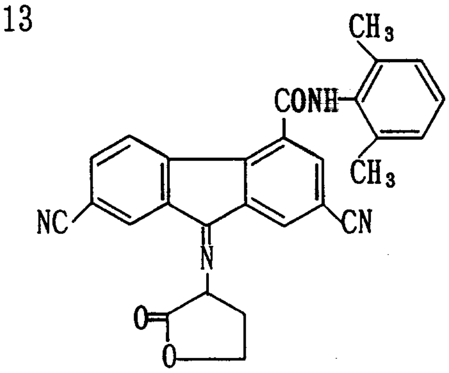

I −14

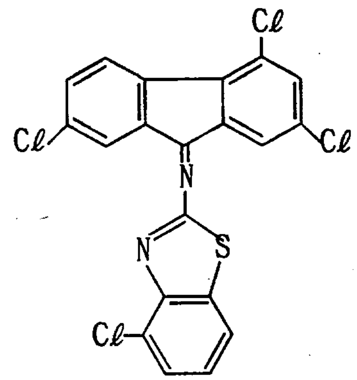

I −15

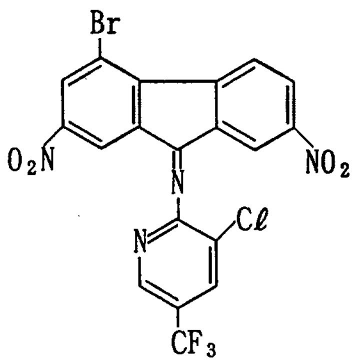

I −16

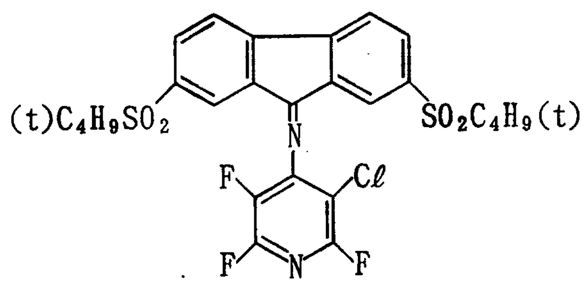

I −17

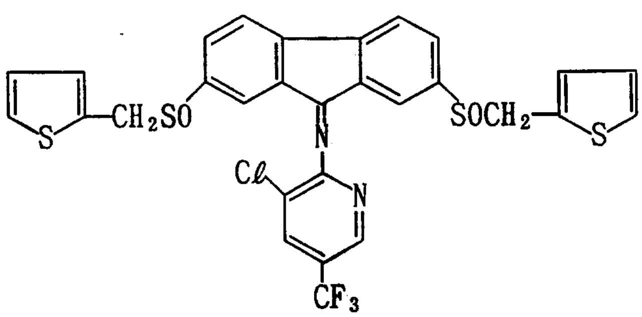

I −18

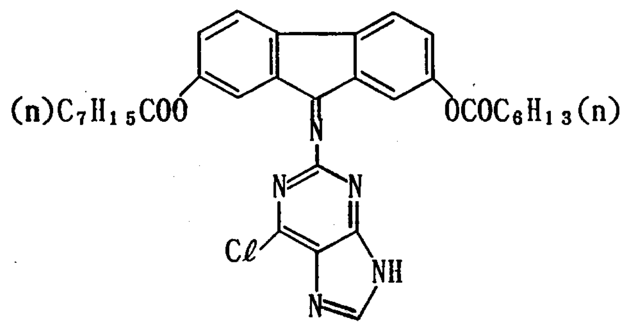

I −19

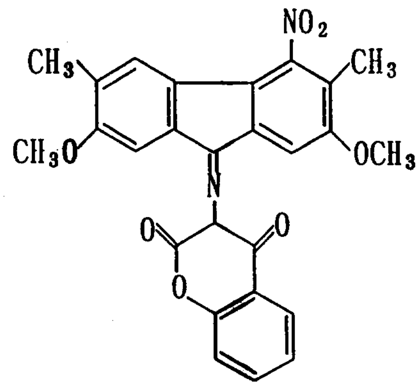

I −20

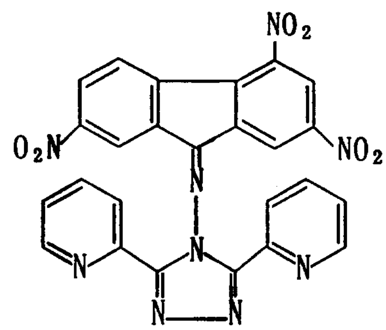

I －21

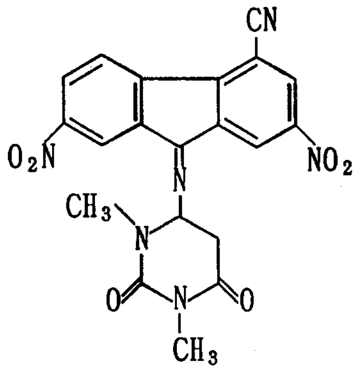

I －22

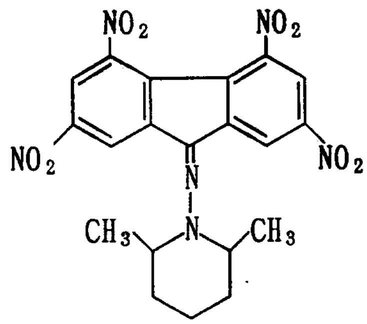

I －23

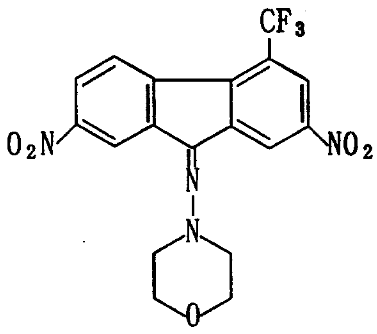

I －24

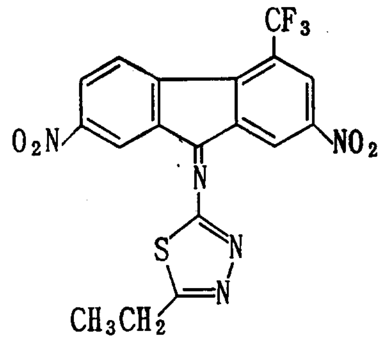

I －25

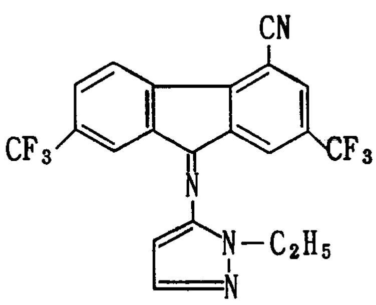

I －26

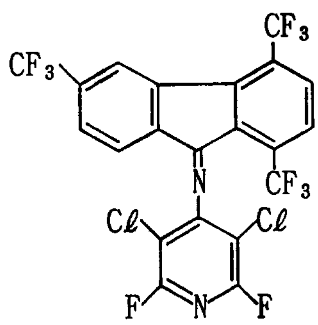

I -27

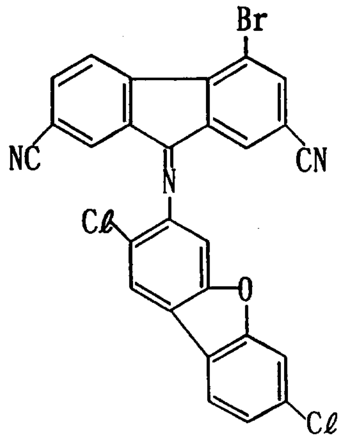

I -28

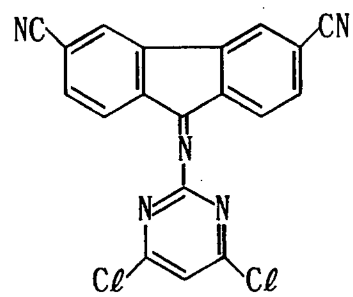

I -29

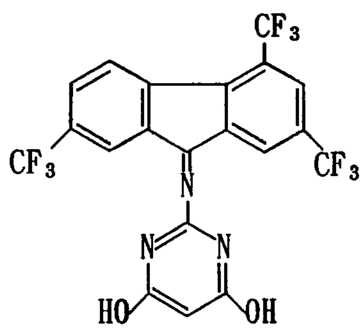

I -30

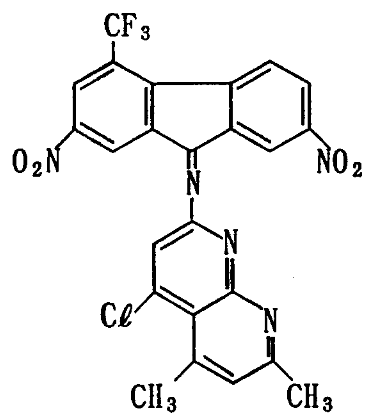

I -31

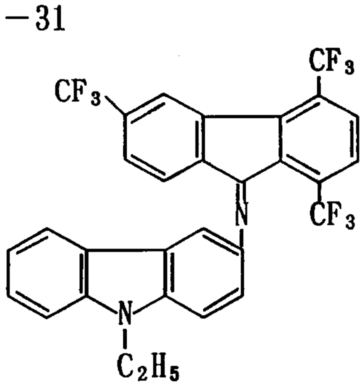

I -32

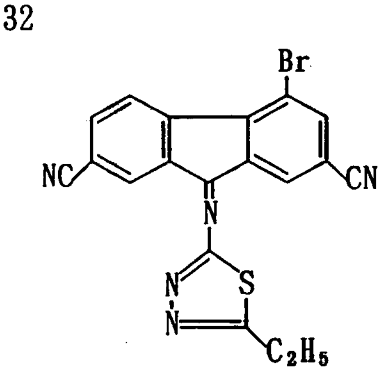

I −33

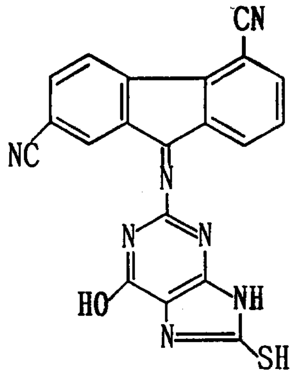

I −34

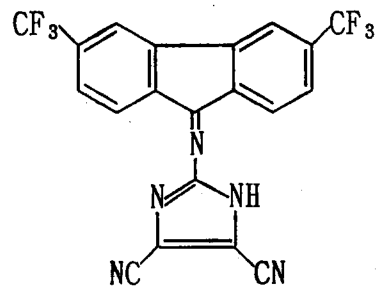

I −35

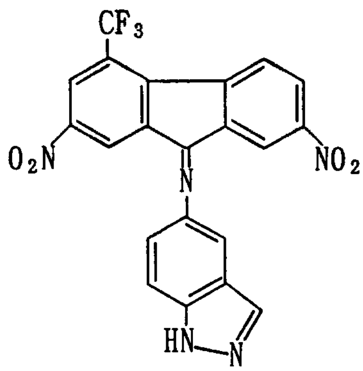

I −36

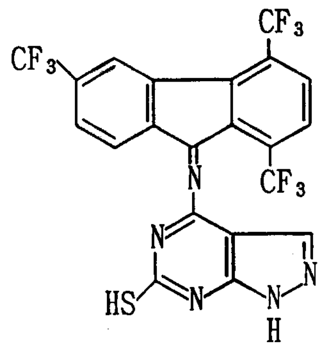

I −37

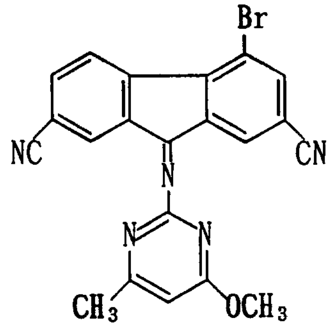

I −38

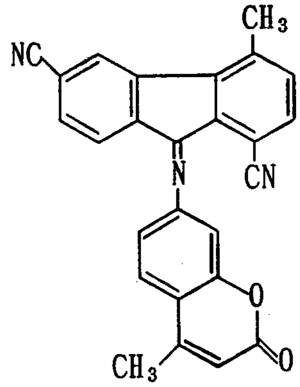

I −39

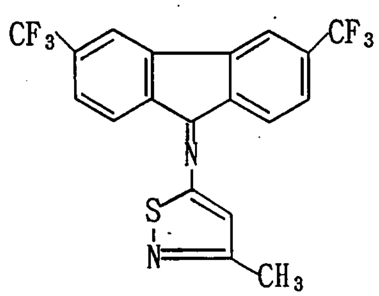

I −40

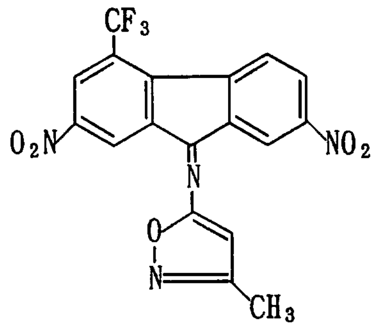

I −41

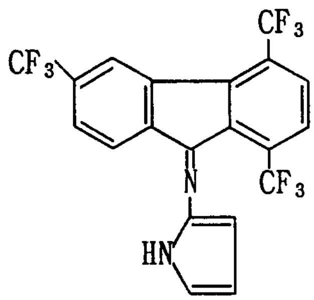

I −42

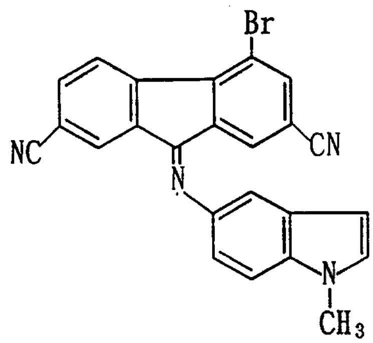

I −43

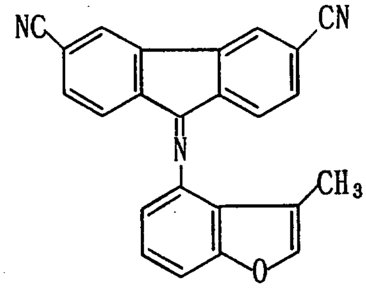

I −44

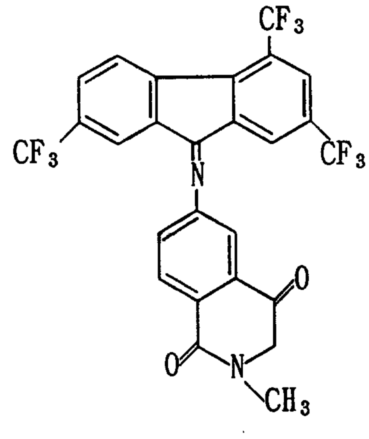

I −45

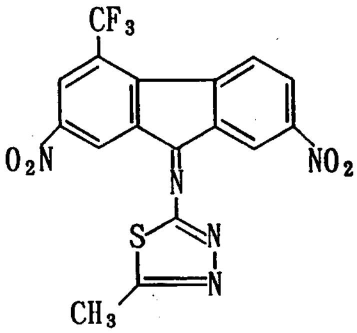

I −46

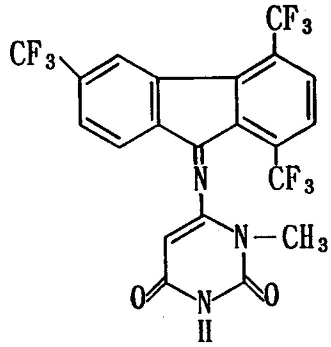

I −47

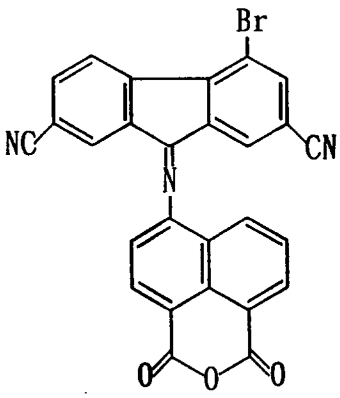

I −48

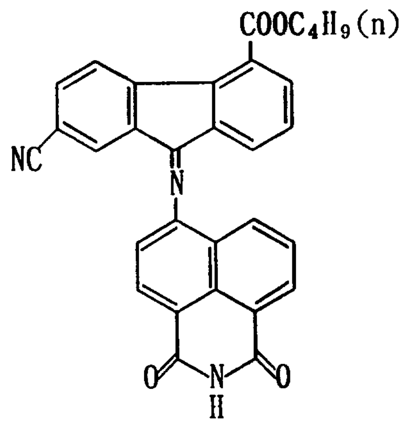

I −49

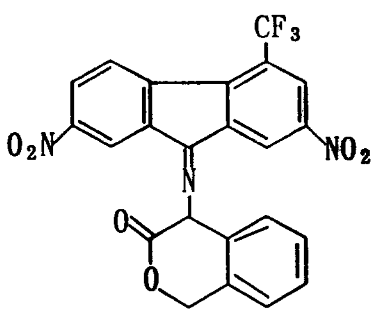

I −50

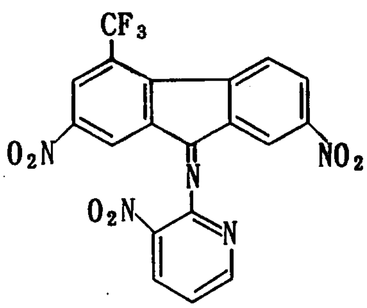

I -51

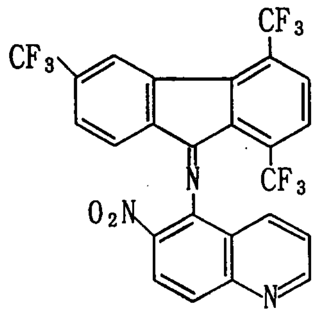

I -52

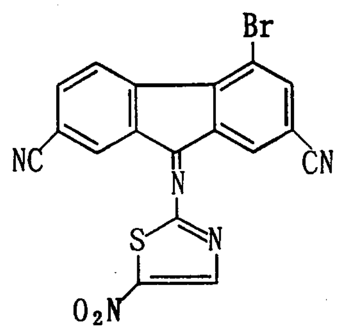

I -53

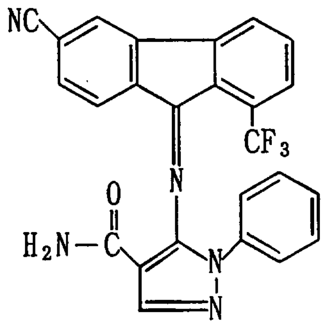

I -54

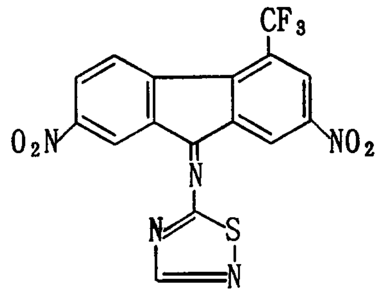

I -55

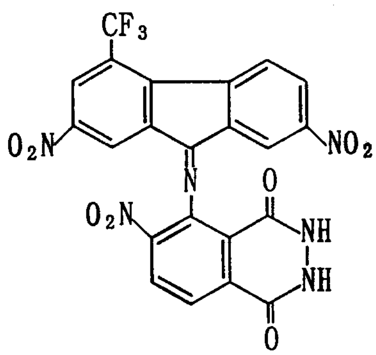

I -56

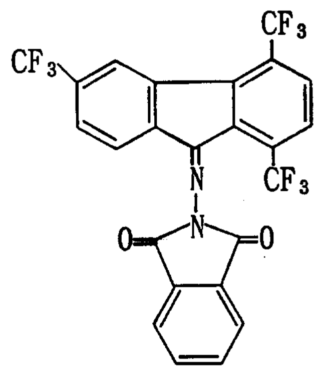

I -57

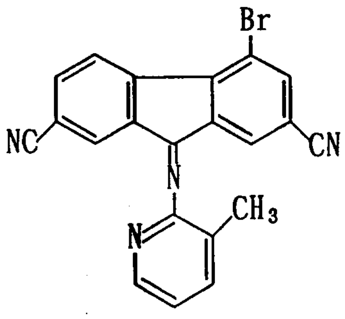

I -58

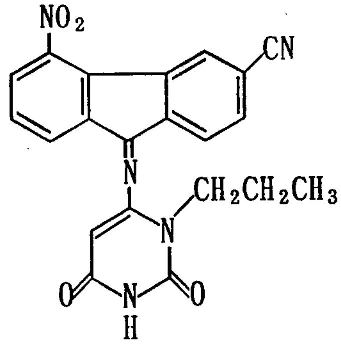

I -59

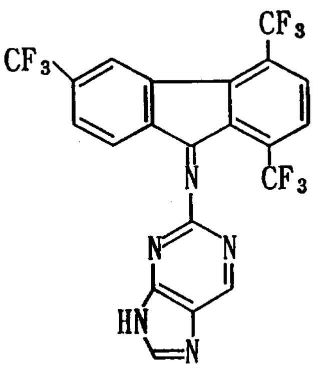

I -60

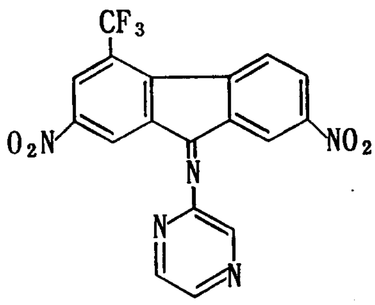

I -61

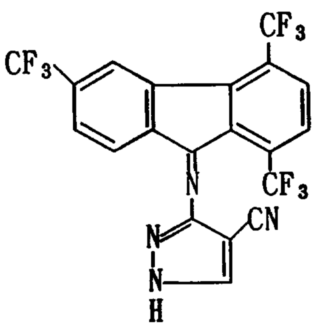

I -62

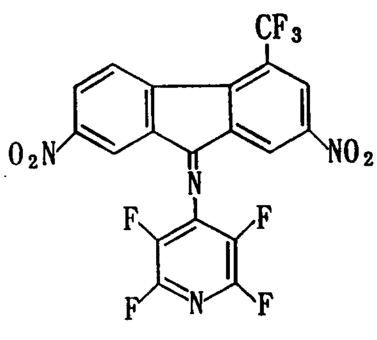

I −63

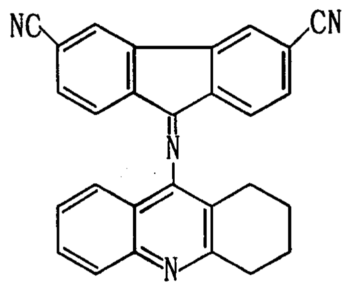

I −64

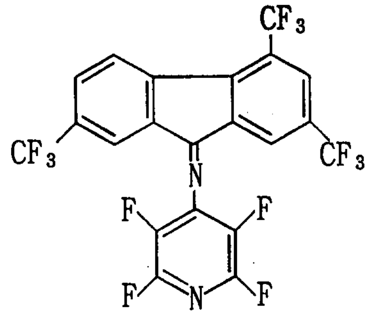

I −65

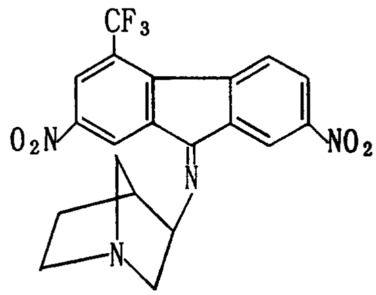

I −66

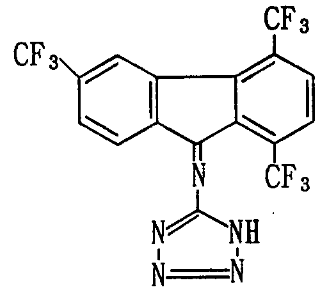

I −67

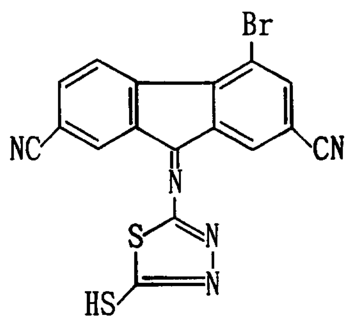

I −68

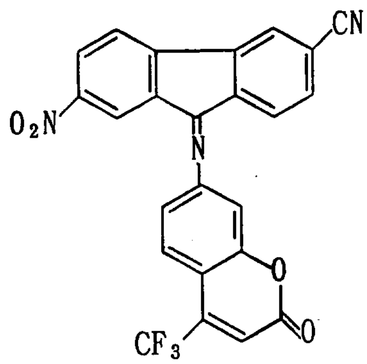

I −69

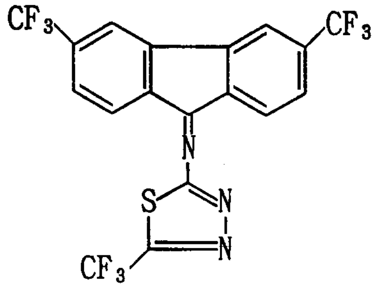

I −70

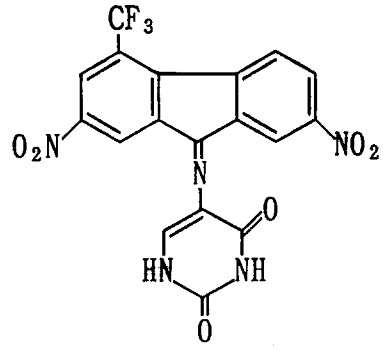

I −71

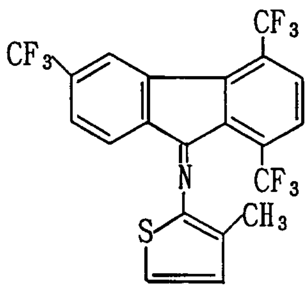

I −72

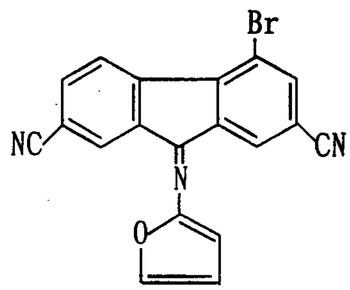

I −73

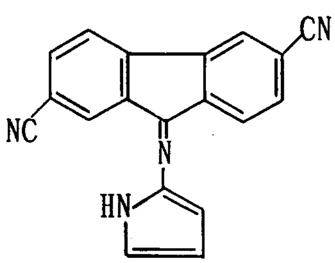

I −74

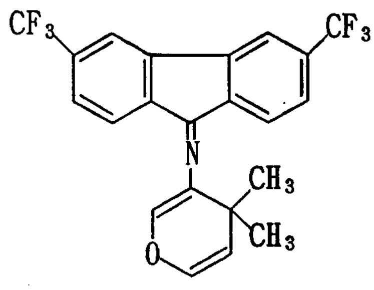

I −75

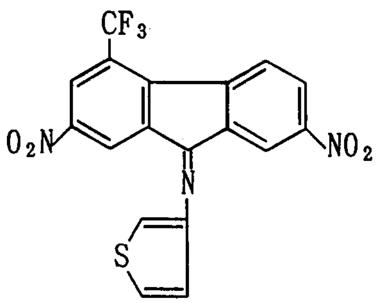

Synthesis Example

Synthesis Example I

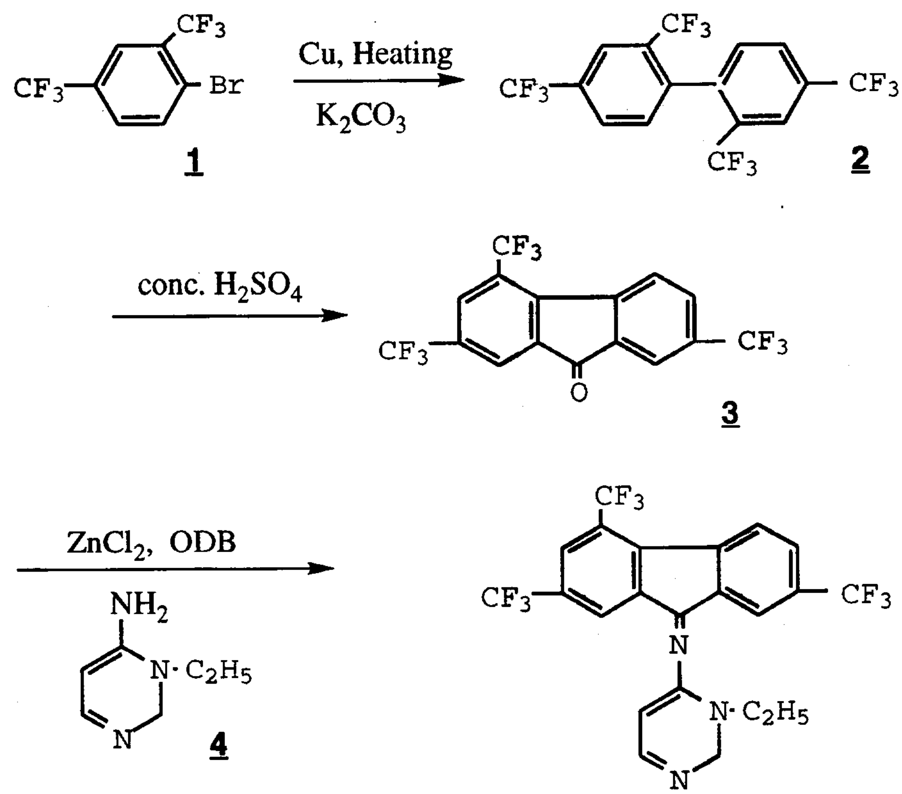


Exemplified compound I-1

Exemplified compound I-1 was synthesized by the above reaction procedure. Compound (3) was synthesized from 2,4,-bis(trifluoromethyl)bromobenzene (1) as a starting material according to known method (J. Chem. Soc., 1071 - 6 (1954)).

In a 100-ml four-necked flask was placed 10 g of the Compound (3), 20 g of zinc chloride, 5 g of 5-amino-1-ethlpyrazole (4) (Made by Aldrich Co.) and 100 ml of ODB, and then an esterification tube and a thermometer were attached to the flask, followed by heating under reflux for 40 hours. After cooling the reaction mixture, the solid matter was sucked off and washed well with DMSO. The solvent in the mother liquor was distilled away under reduced pressure, and the residue was subjected to silica gel column chromatography. The dissolved matter in a 9:1 mixture of toluene and n-hexane was crystallized from toluene. Thus, red crystals of exemplified compound D-2 was obtained in a yield of 9.3 g (75 %). Its melting point was 168-170 °C when measured by the method of measuring meting point specified in the Japanese Pharmacopoeia. Since the results obtained by elemental analysis also agreed well with the calculated values, the product was identified as exemplified compound I-1.

| | C | H | N |
|---|---|---|---|
| Calculated Values | 52.84% | 2.53% | 8.80% |
| Measured Values | 52.81% | 2.55% | 8.77% |

Conductive supports usable in the electrophotographic photoreceptor of the invention include, for example, metal plates, metal sheets, metal foils, polymer films subjected to conductivity-providing treatment, polymer films having a layer of deposited metal such as Al, and polymer films or paper coated with a metal oxide, a quaternary ammonium salt or the like.

In the electrophotographic photoreceptor of the invention, the photoreceptive layer unit provided on the conductive support may have single layer structure or multi-layer structure in which functions are divided between a charge generation layer and a charge transfer layer. Further, an adhesive layer may be provided between the conductive support and the photoreceptive layer unit.

As shown in Fig. 1(a), in one embodiment of the photoreceptor of the invention, there is provided, on conductive support 1, photoreceptive layer unit 4 consisting of a multi-layered body comprising charge generation layer (CGL) 2 containing a charge generation material (CGM) as a principal component and a charge transfer layer (CTL) 3 containing a charge transfer material (CTM) as a principal component. Or, as shown in Fig. 1(b), photoreceptive layer 4 may be formed via intermediate layer 5 provided on conductive support 1. When photoreceptive layer unit 4 is formed into such double-layered structure, photoreceptor's electrophotographic properties become the highest. Further, as shown in Figs. 1(c) and (d), there may be provided on conductive support 1, directly or via intermediate layer 5, photoreceptive layer unit 4 comprising layer 6 containing a CTM as a principal component and fine particles of CGM 7 dispersed in it.

Moreover, a protective layer (OCL) may be provided on photoreceptive layer unit 4, if necessary.

CGL 2 and CTL 3 which constitute photoreceptive layer unit 4 of double-layered structure can be each formed directly on conductive support 1, or via an intermediate layer such as an adhesive layer or a barrier layer provided thereon when necessary, by one of the following methods selected according to the properties of CTM and CGM.

(1) Vapor Phase Deposition

(2) Paint Coating

a) A method of coating a solution paint obtained by dissolving a CGM and a CTM in a solvent.

b) A method of coating a dispersion paint obtained by dispersing a CGM and a CTM into fine particles with a ball mill, a homo-mixer or the like and, if necessary, mixing with an a binding agent.

In carrying out vapor phase deposition, there can be employed the vacuum deposition method, sputtering method, ion plating method or CVD method. And paint coating can be performed by selecting a method suitable for the paint's properties from the methods of dipping coating, spray coating, air doctor coating, doctor blade coating, reverse roll coating and the like.

The adhesive layer may be a layer formed of a resin alone, a layer formed by coating a dispersion obtained by dispersing a low resistant compound such as tin oxide, indium oxide or titanium oxide in a resin, or a layer formed by depositing aluminum oxide, zinc oxide or silicon oxide. The resin used in this adhesive layer is not particularly limited, but preferred examples include vinylidene chloride-vinyl chloride copolymers, water-soluble polyvinylbutyral resins, alcohol-soluble polyamide resins, vinyl acetate type resins, polyvinyl alcohols, nitrocelluloses and polyimide resins.

The thickness of the adhesive layer is preferably from 0.01 to 10 μm and more preferably in the range of 0.01 to 1 μm.

When the photoreceptive layer unit consists of a single layer, the photoreceptive layer unit may be a layer which is formed of a known material such as poly-N-vinylcarbazole and contains one of the compounds represented by formulas A to G as a sensitizer, or a layer containing a known charge generation material and, further, one of the compounds represented by formulas A to G as a charge transfermaterial.

When the photoreceptive layer unit has multi-layered structure, the charge transfer layer may be a layer formed by depositing a charge generation material on a conductive support, or may be one obtained by coating a coating solution whose principal components are a charge generation material and a binder resin.

The charge generation material and the binder resin may be any of the conventional materials.

Suitable charge generation materials include, for example, inorganic semiconductors such as Te-Se, organic semiconductors such as poly-N-vinylcarbazoles and organic pigments such as bisazo compounds, trisazo compounds, non-metallic phthalocyanine compounds, metallic phthalocyanine compounds, pyrylium compounds, squarylium compounds, cyanine compounds, perylene compounds, polycyclic quinine compounds. Preferred examples of the charge generation material include the Y-type titanyl phthalocyanine pigment having an X-ray diffraction peak at 27.2° which is disclosed in JA. O.P.I. 64-17066, the A-type titanyl phthalocyanine pigment having an X-ray diffraction peak at 26.3° which is disclosed in JA O.P.I. 62-67094, the B-type titanyl phthalocyanine pigment having an X-ray diffraction peak at 28.7° which is disclosed in JA O.P.I. 61-239248, the non-metallic phthalocyanine pigment disclosed in JA Examined B49-4338, the copper phthalocyanine pigment in JA O.P.I. 57-163239, the vanadyl phthalocyanine pigment in JA O.P.I. 67-148747, the perylene pigment in Japanese Pat. O.P.I. Pub. No. 128734/1974, the condensed polycyclic pigment in JA O.P.I. 4718544, and the bisazo pigment in JA O.P.I. 1-150145. Preferred examples of the binder resin include polystyrenes, silicone resins, polycarbonate resins, acrylic resins, methacrylic resins, polyesters, vinyl copolymers, cellulose resins, butyral resins, silicon-modified butyral resins and

alkyd resins.

The thickness of the charge generation layer is preferably about 0.01 to 10 µm and more preferably in the range of 0.05 to 2 µm.

On the charge generation layer is formed a charge transfer layer. This charge transfer layer comprises a compound selected from those represented by formulas A to G and a binder resin and is formed by coating, on the charge generation layer, a coating solution containing one of the compounds represented by formulas A to G, a binder resin and a suitable solvent as principal components using an applicator, a bar coater or a dip coater. In such a coating solution, the mixing ratio of various compounds to binder resin is preferably 1:100 to 100:1 and especially 1:20 to 20:1.

The charge transfer material and binder resin used in the charge transfer layer may be any of the conventional materials employed for this purpose. Preferred examples of the binder resin include acrylonitrile-butadiene coplymers, styrene-butadiene copolymers, vinyltoluene-styrene copolymers, styrene-modified alkyd resins, silicon-modified alkyd resins, soybean-oil-modified alkyd resins, vinylidene chloride-vinyl chloride copolymers, polyvinyl butyrals, nitrated polystyrenes, polymethyl styrenes, polyisoprenes, polyesters, phenolic resins, ketone resins, polyamides, polycarbonates, polythiocarbonates, polyacrylates, polyhaloacrylates, polyvinyl acetate type resins, polystyrenes, polyaryl ethers, polyvinyl acrylates, polysulfones, and polymethacrylates. Of them, biphenyl Z-type polycarbonates are particularly preferred. Further, an electron donative material may be added to the charge transfer layer for the purpose of making a bipolar photoreceptor.

Moreover, an oxidizing agent and a radical trapping agent may also be added to the charge transfer layer.

The thickness of the charge transfer layer is preferably 2 to 100 µm and more preferably 5 to 50 µm.

In the electrophotographic photoreceptor of the invention, a barrier layer may be provided on the conductive support. Such a barrier layer is effective in preventing the inflow of unnecessary charges from the conductive support and, thereby, functions as a means for improving image quality. As materials to form the barrier layer, there can be used metal oxides such as aluminum oxide, acrylic resins, phenolic resins, polyester resins and polyurethanes. The barrier layer may be provided on or under the adhesive layer.

**EXAMPLES**

The invention is illustrated in detail with the following examples.

The examples described below are classified into groups A to G correspondingly to the compounds of formulas A to G according to the invention, and each group consists of:

(I) sensitivity evaluation (evaluation 1)···Examples 1 to 10 and Comparative Example (1)

(II) durability evaluation (evaluation 2)···Examples 11 to 20 and Comparative Example (2)

(III) image quality evaluation (evaluation 3)···Examples 21 to 30 and Comparative Example 3,

and test samples used in each group are the same in shape, kind and size, except that the compound according to the invention is different. Samples for Comparative Examples are different with evaluations but the same in every group when used in the same evaluation.

(I) Sensitivity Evaluation

Evaluation 1

Electrophotographic photoreceptor samples prepared in Example 1 and Comparative Example (1) were electrified to +800 V on an EPA-8100 electrostatic copying paper tester (Made by Kawaguchi Denki Co.) and exposed with white light of 10 lux to determine the exposure necessary to halve the surface potential, which was taken as the sensitivity. The results are shown in each column (I) of Tables 1 to 7.

Examples 1 to 10

On an aluminum-deposited PET film was provided a 0.5-µm thick intermediate layer consisting of CM8000 polyamide resin (Made by Toray Ind., Inc.), and a 0.3-µm thick charge generation layer was formed thereon by coating, with a wire bar, a dispersion prepared by dispersing 1 part of perylene pigment CGM-1 and 0.2 part of Eslec BMS polybutyral resin (Made by Sekisui Chemical Co., Ltd.) in 50 parts of methyl ethyl ketone using a sand mill. Subsequently, a 20-µm thick charge transfer layer was formed on the charge generation layer by the blade coating of a solution obtained by dissolving 1 part of the exemplified compound shown in each column (I) of Tables 1 to 7 and 1.5 parts of Iupilon Z-200

polycarbonate resin (Made by Mitsubishi Gas Chem. Co., Ltd.) in 10 parts of THF.

Comparative Example (1)

Comparative samples were prepared in the same manner as in Example 1, except that comparative compound CTM (1) was used in place of the exemplified compound.

(II) Durability Evaluation

Evaluation 2

Electrophotographic photoreceptor samples prepared in Example 11 and Comparative Example (2) were evaluated on durabilities by measuring the following items at the initial copying and after 10,000 times of copying using an improved Konica U-1017 electrophotographic copier.
$V_b$: potential at black portion, $V_w$: potential at white portion, $V_r$: residual potential

Examples 11 to 20

A 0.5-μm thick intermediate layer consisting of X-1874M polyamide resin (Made by Toray Co., Ltd.) was provided on an aluminum-deposited PET film, and a 0.3-μm thick charge generation layer was formed thereon by the wire bar coating of a dispersion prepared by dispersing 1 part of X-type non-metallic phthalocyanine pigment (Made by Dainippon Ink & Chemicals, Inc.) and 0.4 part of Eslec BX-1 polybutyral resin (Made by Sekisui Chemical Co., Ltd.) in 50 parts of methyl isopropyl ketone using a sand mill. Subsequently, a 20-μm thick charge transfer layer was formed on the charge generation layer by the blade coating of a solution obtained by dissolving 1 part of the exemplified compound shown in each column (II) of Tables 1 to 7 and 1.5 parts of Iupilon Z-200 polycarbonate resin (Made by Mitsubishi Gas Chem. Co., Ltd.) in 10 parts of THF.

Comparative Example (2)

Comparative samples were prepared in the same manner as in Example 11, except that comparative compound CTM (2) was used in place of the exemplified compound.

(III) Image Quality Evaluation

Evaluation 3

Electrophotographic photoreceptor samples prepared in Example 21 and Comparative Example (3) were subjected to image forming procedure on an improved Konica 9028 digital copier (electrification polarity: positive, reverse developing). The samples were then allowed to stand for 1 month at 10 °C and, after that, subjected to image forming procedure again under the same conditions. The image quality of these samples was rated by observing pepper spots in the white portion of copied images. The results are shown in each column (III) of Tables 1 to 7.

The observation of pepper spots was made with Omnicon 300 image analyzer (Made by Shimadzu Corp.) by measuring the size and number of pepper spots. And the image quality was rated by judging the number of pepper spots larger than 0.05 mm in diameter per $cm^2$ of white portion; the criterion for rating is shown below. Ratings A and B indicate to be serviceable in practical use, rating C indicates to be not serviceable in some cases, and rating D means to be unserviceable.

| Number of Pepper Spots Lager than 0.05 mm in Diameter (number/$cm^2$) | Rating |
|---|---|
| 0 | A |
| 1 to 3 | B |
| 4 to 10 | C |
| 11 or more | D |

Examples 21 to 30

A 0.5-mm thick intermediate layer consisting of CM8000 polyamide resin (Made by Toray Ind., Inc.) was provided on a cylindrical aluminum base, and a 0.3-μm thick charge generation layer was formed thereon by the dip coating of a dispersion prepared by dispersing 1 part of titanyl phthalocyanine having X-ray diffraction peaks at Bragg angles ($2\theta$) of 9.5°, 24.1° and 27.2° and 0.5 part of silicone butyral resin in 50 parts of methyl isopropyl ketone with a sand mill. Then, a 20-μm thick charge transfer layer was formed on the charge generation layer by the dip coating of a solution obtained by dissolving 1 part of the exemplified compound shown in each column (III) of Tables 1 to 7 and 1.5 parts of Iupilon Z-200 polycarbonate resin (Made by Mitsubishi Gas Chem. Co., Ltd.) in 10 parts of THF.

Comparative Example (3)

Comparative samples were prepared in the same manner as in Example 21, except that comparative compound CTM (3) was used in place of the exemplified compound.

CGM-1

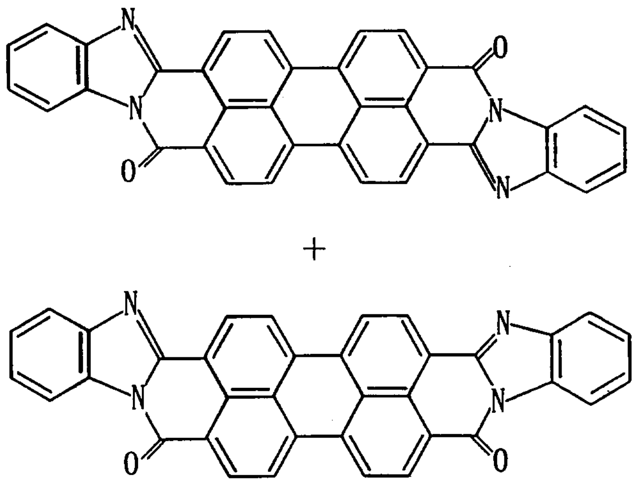

+

Mixture

CTM(1)

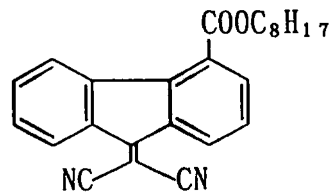

CTM(2)

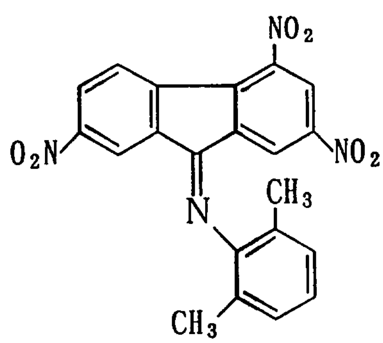

CTM(3)

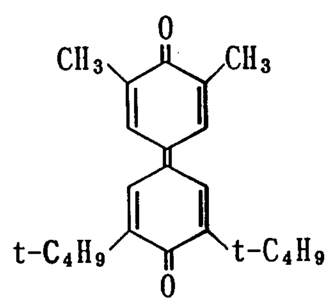

Table 1

Example Group A

A-1

| Example No. | Compound | Sensitivity (Lux·sec) |
|---|---|---|
| A-1 | A-1 | 4.2 |
| A-2 | A-3 | 5.0 |
| A-3 | A-4 | 5.1 |
| A-4 | A-9 | 6.5 |
| A-5 | A-13 | 4.5 |
| A-6 | A-16 | 4.0 |
| A-7 | A-18 | 6.3 |
| A-8 | A-24 | 5.4 |
| A-9 | A-27 | 5.1 |
| A-10 | A-29 | 4.9 |
| A-(1) | CTM(1) | 7.3 |

A-2

| Example No. | Compound | Initial | | | After 10,000 copy | | |
|---|---|---|---|---|---|---|---|
| | | Vb | Vw | Vr | Vb | Vw | Vr |
| A-1 | A-2 | 750 | 110 | 30 | 740 | 130 | 35 |
| A-2 | A-5 | 720 | 120 | 25 | 725 | 140 | 40 |
| A-3 | A-7 | 715 | 135 | 40 | 750 | 155 | 65 |
| A-4 | A-10 | 730 | 115 | 30 | 745 | 135 | 40 |
| A-5 | A-11 | 730 | 144 | 25 | 740 | 175 | 20 |
| A-6 | A-16 | 705 | 110 | 45 | 715 | 134 | 25 |
| A-7 | A-20 | 740 | 125 | 30 | 745 | 140 | 80 |
| A-8 | A-21 | 765 | 135 | 35 | 750 | 150 | 40 |
| A-9 | A-25 | 770 | 130 | 20 | 750 | 165 | 25 |
| A-10 | A-30 | 755 | 115 | 50 | 735 | 140 | 5 |
| A-(1) | CTM(2) | 730 | 125 | 40 | 725 | 250 | 100 |

A-3

| Example | Compound | Initial | After 10,000 copy |
|---|---|---|---|
| A-21 | A-1 | A | A |
| A-22 | A-6 | A | A |
| A-23 | A-8 | A | A |
| A-24 | A-12 | A | A |
| A-25 | A-14 | A | B |
| A-26 | A-15 | A | A |
| A-27 | A-17 | A | B |
| A-28 | A-22 | A | B |
| A-29 | A-23 | A | B |
| A-30 | A-28 | A | A |
| A-(3) | CTM(3) | C | D |

Table 2

Example Group B

B-1

| Example No. | Compound | Sensitivity (Lux·sec) |
|---|---|---|
| B-1 | B-1 | 4.1 |
| B-2 | B-2 | 4.0 |
| B-3 | B-4 | 4.2 |
| B-4 | B-6 | 4.0 |
| B-5 | B-8 | 4.3 |
| B-6 | B-10 | 6.2 |
| B-7 | B-12 | 4.7 |
| B-8 | B-18 | 6.3 |
| B-9 | B-20 | 5.1 |
| B-10 | B-24 | 5.2 |
| B-(1) | CTM(1) | 7.3 |

B-2

| Example No. | Compound | Initial | | | After 10,000 copy | | |
|---|---|---|---|---|---|---|---|
| | | Vb | Vw | Vr | Vb | Vw | Vr |
| B-1 | B-1 | 750 | 105 | 20 | 730 | 115 | 30 |
| B-2 | B-2 | 730 | 110 | 25 | 720 | 125 | 30 |
| B-3 | B-3 | 725 | 105 | 20 | 710 | 120 | 35 |
| B-4 | B-5 | 735 | 115 | 30 | 745 | 120 | 35 |
| B-5 | B-7 | 740 | 115 | 35 | 755 | 135 | 40 |
| B-6 | B-9 | 705 | 120 | 30 | 715 | 140 | 75 |
| B-7 | B-13 | 710 | 135 | 40 | 715 | 155 | 40 |
| B-8 | B-17 | 735 | 145 | 45 | 745 | 165 | 75 |
| B-9 | B-19 | 720 | 155 | 45 | 750 | 170 | 80 |
| B-10 | B-25 | 745 | 150 | 50 | 750 | 180 | 85 |
| B-(1) | CTM(2) | 730 | 125 | 40 | 725 | 250 | 100 |

B-3

| Example | Compound | Initial | After 10,000 copy |
|---|---|---|---|
| B-21 | B-2 | A | A |
| B-22 | B-4 | A | A |
| B-23 | B-6 | A | A |
| B-24 | B-8 | A | A |
| B-25 | B-10 | A | B |
| B-26 | B-11 | A | A |
| B-27 | B-15 | A | A |
| B-28 | B-18 | A | A |
| B-29 | B-21 | A | A |
| B-30 | B-23 | A | A |
| B-(3) | CTM(3) | C | D |

Table 3

Example Group C

C-1

| Example No. | Compound | Sensitivity (Lux·sec) |
|---|---|---|
| C-1 | C-1 | 4.3 |
| C-2 | C-3 | 4.4 |
| C-3 | C-5 | 4.1 |
| C-4 | C-9 | 4.6 |
| C-5 | C-10 | 4.7 |
| C-6 | C-13 | 4.8 |
| C-7 | C-15 | 5.0 |
| C-8 | C-19 | 6.5 |
| C-9 | C-25 | 5.3 |
| C-10 | C-31 | 5.1 |
| C-(1) | CTM(1) | 7.3 |

C-2

| Example No. | Compound | Initial | | | After 10,000 copy | | |
|---|---|---|---|---|---|---|---|
| | | Vb | Vw | Vr | Vb | Vw | Vr |
| C-1 | C-2 | 760 | 105 | 20 | 750 | 130 | 35 |
| C-2 | C-4 | 730 | 120 | 25 | 735 | 125 | 40 |
| C-3 | C-6 | 720 | 125 | 20 | 745 | 130 | 45 |
| C-4 | C-7 | 725 | 110 | 25 | 715 | 135 | 50 |
| C-5 | C-11 | 735 | 115 | 30 | 700 | 130 | 55 |
| C-6 | C-18 | 740 | 115 | 30 | 750 | 130 | 60 |
| C-7 | C-20 | 740 | 135 | 35 | 755 | 145 | 80 |
| C-8 | C-21 | 710 | 130 | 40 | 720 | 140 | 65 |
| C-9 | C-25 | 760 | 135 | 45 | 750 | 150 | 50 |
| C-10 | C-30 | 755 | 145 | 50 | 750 | 155 | 65 |
| C-(1) | CTM(2) | 730 | 125 | 40 | 725 | 250 | 100 |

C-3

| Example | Compound | Initial | After 10,000 copy |
|---|---|---|---|
| C-21 | C-1 | A | A |
| C-22 | C-3 | A | A |
| C-23 | C-4 | A | A |
| C-24 | C-6 | A | A |
| C-25 | C-9 | A | A |
| C-26 | C-12 | A | B |
| C-27 | C-18 | A | A |
| C-28 | C-27 | A | B |
| C-29 | C-29 | A | A |
| C-30 | C-34 | A | A |
| C-(3) | CTM(3) | C | D |

Table 4

Example Group D

D-1

| Example No. | Compound | Sensitivity (Lux·sec) |
|---|---|---|
| D-1 | D-2 | 4.0 |
| D-2 | D-3 | 4.0 |
| D-3 | D-5 | 4.1 |
| D-4 | D-8 | 6.1 |
| D-5 | D-12 | 6.2 |
| D-6 | D-18 | 6.4 |
| D-7 | D-20 | 4.9 |
| D-8 | D-21 | 4.2 |
| D-9 | D-27 | 4.1 |
| D-10 | D-30 | 6.2 |
| D-(1) | CTM(1) | 7.3 |

D-2

| Example No. | Compound | Initial | | | After 10,000 copy | | |
|---|---|---|---|---|---|---|---|
| | | Vb | Vw | Vr | Vb | Vw | Vr |
| D-1 | D-1 | 740 | 105 | 30 | 750 | 110 | 30 |
| D-2 | D-2 | 715 | 115 | 25 | 730 | 125 | 40 |
| D-3 | D-4 | 720 | 125 | 30 | 720 | 135 | 35 |
| D-4 | D-6 | 725 | 130 | 30 | 725 | 140 | 45 |
| D-5 | D-7 | 730 | 135 | 45 | 730 | 140 | 80 |
| D-6 | D-10 | 710 | 145 | 50 | 745 | 155 | 75 |
| D-7 | D-13 | 715 | 125 | 40 | 710 | 150 | 85 |
| D-8 | D-22 | 725 | 115 | 55 | 730 | 150 | 45 |
| D-9 | D-23 | 730 | 110 | 25 | 745 | 120 | 30 |
| D-10 | D-29 | 740 | 115 | 30 | 755 | 130 | 25 |
| D-(1) | CTM(2) | 730 | 125 | 40 | 725 | 250 | 100 |

D-3

| Example | Compound | Initial | After 10,000 copy |
|---|---|---|---|
| D-21 | D-1 | A | A |
| D-22 | D-2 | A | A |
| D-23 | D-5 | A | A |
| D-24 | D-9 | A | B |
| D-25 | D-14 | A | B |
| D-26 | D-16 | A | A |
| D-27 | D-19 | A | B |
| D-28 | D-22 | A | A |
| D-29 | D-24 | A | B |
| D-30 | D-28 | A | A |
| D-(3) | CTM(3) | C | D |

Table 5

Example Group E

E-1

| Example No. | Compound | Sensitivity (Lux·sec) |
|---|---|---|
| E-1 | E-1 | 4.1 |
| E-2 | E-3 | 4.3 |
| E-3 | E-6 | 4.4 |
| E-4 | E-8 | 5.2 |
| E-5 | E-12 | 4.7 |
| E-6 | E-15 | 5.3 |
| E-7 | E-17 | 4.6 |
| E-8 | E-21 | 4.3 |
| E-9 | E-23 | 6.1 |
| E-10 | E-27 | 5.8 |
| E-(1) | CTM(1) | 7.3 |

E-2

| Example No. | Compound | Initial | | | After 10,000 copy | | |
|---|---|---|---|---|---|---|---|
| | | Vb | Vw | Vr | Vb | Vw | Vr |
| E-1 | E-2 | 740 | 115 | 35 | 750 | 120 | 40 |
| E-2 | E-4 | 730 | 110 | 40 | 720 | 130 | 45 |
| E-3 | E-7 | 725 | 105 | 25 | 715 | 130 | 30 |
| E-4 | E-11 | 715 | 120 | 35 | 730 | 140 | 40 |
| E-5 | E-16 | 730 | 115 | 40 | 740 | 145 | 45 |
| E-6 | E-18 | 755 | 105 | 50 | 750 | 145 | 55 |
| E-7 | E-19 | 750 | 130 | 25 | 760 | 145 | 30 |
| E-8 | E-22 | 725 | 135 | 20 | 715 | 140 | 80 |
| E-9 | E-24 | 715 | 125 | 30 | 720 | 140 | 50 |
| E-10 | E-30 | 725 | 130 | 35 | 740 | 135 | 40 |
| E-(1) | CTM(2) | 730 | 125 | 40 | 725 | 250 | 100 |

E-3

| Example | Compound | Initial | After 10,000 copy |
|---|---|---|---|
| E-21 | E-1 | A | A |
| E-22 | E-2 | A | A |
| E-23 | E-5 | A | A |
| E-24 | E-9 | A | A |
| E-25 | E-10 | A | A |
| E-26 | E-14 | A | A |
| E-27 | E-20 | A | A |
| E-28 | E-22 | A | B |
| E-29 | E-25 | A | B |
| E-30 | E-28 | A | A |
| E-(3) | CTM(3) | C | D |

Table 6

Example Group F

F-1

| Example No. | Compound | Sensitivity (Lux·sec) |
|---|---|---|
| F-1 | F-1 | 4.2 |
| F-2 | F-3 | 4.1 |
| F-3 | F-4 | 4.3 |
| F-4 | F-7 | 4.4 |
| F-5 | F-13 | 4.5 |
| F-6 | F-17 | 4.6 |
| F-7 | F-21 | 5.2 |
| F-8 | F-26 | 5.6 |
| F-9 | F-29 | 5.1 |
| F-10 | F-33 | 6.5 |
| F-(1) | CTM(1) | 7.3 |

F-2

| Example No. | Compound | Initial | | | After 10,000 copy | | |
|---|---|---|---|---|---|---|---|
| | | Vb | Vw | Vr | Vb | Vw | Vr |
| F-1 | F-2 | 760 | 115 | 25 | 750 | 130 | 35 |
| F-2 | F-5 | 740 | 110 | 20 | 730 | 140 | 40 |
| F-3 | F-8 | 730 | 120 | 35 | 725 | 145 | 45 |
| F-4 | F-10 | 725 | 130 | 25 | 730 | 145 | 50 |
| F-5 | F-15 | 750 | 140 | 30 | 740 | 150 | 75 |
| F-6 | F-18 | 755 | 115 | 45 | 750 | 145 | 50 |
| F-7 | F-22 | 750 | 120 | 50 | 755 | 135 | 55 |
| F-8 | F-27 | 745 | 130 | 25 | 765 | 135 | 40 |
| F-9 | F-30 | 725 | 115 | 30 | 730 | 140 | 35 |
| F-10 | F-35 | 715 | 120 | 25 | 725 | 145 | 70 |
| F-(1) | CTM(2) | 730 | 125 | 40 | 725 | 250 | 100 |

F-3

| Example | Compound | Initial | After 10,000 copy |
|---|---|---|---|
| F-21 | F-1 | A | A |
| F-22 | F-4 | A | B |
| F-23 | F-6 | A | A |
| F-24 | F-9 | A | A |
| F-25 | F-11 | A | A |
| F-26 | F-12 | A | B |
| F-27 | F-19 | A | A |
| F-28 | F-23 | A | A |
| F-29 | F-24 | A | B |
| F-30 | F-32 | A | A |
| F-(3) | CTM(3) | C | D |

Table 7

Example Group G

G-1

| Example No. | Compound | Sensitivity (Lux·sec) |
|---|---|---|
| G-1 | G-1 | 4.0 |
| G-2 | G-4 | 4.3 |
| G-3 | G-5 | 6.1 |
| G-4 | G-11 | 4.5 |
| G-5 | G-14 | 4.6 |
| G-6 | G-16 | 4.2 |
| G-7 | G-19 | 4.7 |
| G-8 | G-21 | 6.2 |
| G-9 | G-24 | 5.9 |
| G-10 | G-29 | 5.1 |
| G-(1) | CTM(1) | 7.3 |

G-2

| Example No. | Compound | Initial | | | After 10,000 copy | | |
|---|---|---|---|---|---|---|---|
| | | Vb | Vw | Vr | Vb | Vw | Vr |
| G-1 | G-2 | 730 | 120 | 30 | 740 | 125 | 35 |
| G-2 | G-5 | 720 | 115 | 20 | 730 | 120 | 30 |
| G-3 | G-6 | 715 | 110 | 35 | 720 | 130 | 40 |
| G-4 | G-9 | 725 | 130 | 25 | 725 | 135 | 30 |
| G-5 | G-12 | 705 | 125 | 20 | 715 | 130 | 40 |
| G-6 | G-17 | 715 | 110 | 40 | 720 | 130 | 45 |
| G-7 | G-18 | 725 | 115 | 35 | 730 | 130 | 40 |
| G-8 | G-22 | 730 | 125 | 20 | 735 | 135 | 50 |
| G-9 | G-25 | 710 | 110 | 25 | 740 | 125 | 45 |
| G-10 | G-30 | 720 | 135 | 30 | 740 | 140 | 40 |
| G-(1) | CTM(2) | 730 | 125 | 40 | 725 | 250 | 100 |

G-3

| Example | Compound | Initial | After 10,000 copy |
|---|---|---|---|
| G-21 | G-3 | A | A |
| G-22 | G-5 | A | C |
| G-23 | G-8 | A | A |
| G-24 | G-10 | A | A |
| G-25 | G-13 | A | B |
| G-26 | G-15 | A | A |
| G-27 | G-20 | A | A |
| G-28 | G-23 | A | B |
| G-29 | G-26 | A | B |
| G-30 | G-28 | A | B |
| G-(3) | CTM(3) | C | D |

Table 8

Example Group H

H-1

| Example No. | Compound | Sensitivity (Lux·sec) |
|---|---|---|
| 1 | H-1 | 4.1 |
| 2 | H-4 | 4.3 |
| 3 | H-7 | 4.4 |
| 4 | H-10 | 5.5 |
| 5 | H-13 | 4.2 |
| 6 | H-16 | 5.4 |
| 7 | H-19 | 5.6 |
| 8 | H-22 | 5.5 |
| 9 | H-25 | 4.4 |
| 10 | H-28 | 5.3 |
| (1) | CTM(1) | 7.3 |

H-2

| Example No. | Compound | Initial | | | After 10,000 copy | | |
|---|---|---|---|---|---|---|---|
| | | Vb | Vw | Vr | Vb | Vw | Vr |
| 11 | H-2 | 730 | 120 | 25 | 720 | 130 | 30 |
| 12 | H-5 | 745 | 130 | 30 | 730 | 145 | 35 |
| 13 | H-8 | 725 | 135 | 35 | 710 | 140 | 40 |
| 14 | H-11 | 715 | 125 | 30 | 725 | 150 | 35 |
| 15 | H-14 | 720 | 140 | 50 | 730 | 145 | 60 |
| 16 | H-17 | 735 | 125 | 55 | 740 | 135 | 65 |
| 17 | H-20 | 755 | 130 | 45 | 755 | 135 | 55 |
| 18 | H-23 | 765 | 135 | 25 | 750 | 140 | 30 |
| 19 | H-26 | 750 | 145 | 35 | 740 | 150 | 40 |
| 20 | H-29 | 740 | 130 | 50 | 735 | 145 | 70 |
| (2) | CTM(2) | 730 | 125 | 40 | 725 | 250 | 100 |

H-3

| Example | Compound | Initial | After 10,000 copy |
|---|---|---|---|
| 21 | H-2 | A | A |
| 22 | H-4 | A | A |
| 23 | H-6 | A | A |
| 24 | H-8 | A | A |
| 25 | H-10 | A | A |
| 26 | H-11 | A | A |
| 27 | H-15 | A | A |
| 28 | H-18 | A | A |
| 29 | H-21 | A | A |
| 30 | H-23 | A | B |
| (3) | CTM(3) | C | D |

Table 9

Example Group I

I-1

| Example No. | Compound | Sensitivity (Lux·sec) |
|---|---|---|
| 1 | I-1 | 4.3 |
| 2 | I-2 | 4.2 |
| 3 | I-4 | 4.4 |
| 4 | I-8 | 4.2 |
| 5 | I-12 | 4.1 |
| 6 | I-17 | 5.7 |
| 7 | I-26 | 4.2 |
| 8 | I-28 | 5.5 |
| 9 | I-35 | 4.3 |
| 10 | I-44 | 4.2 |
| (1) | CTM(1) | 7.3 |

I-2

| Example No. | Compound | Initial | | | After 10,000 copy | | |
|---|---|---|---|---|---|---|---|
| | | Vb | Vw | Vr | Vb | Vw | Vr |
| 11 | I-2 | 740 | 120 | 25 | 750 | 125 | 30 |
| 12 | I-5 | 730 | 135 | 30 | 740 | 140 | 35 |
| 13 | I-8 | 725 | 140 | 55 | 740 | 145 | 65 |
| 14 | I-11 | 735 | 135 | 60 | 7540 | 150 | 75 |
| 15 | I-14 | 750 | 145 | 65 | 755 | 150 | 75 |
| 16 | I-17 | 755 | 130 | 25 | 750 | 135 | 30 |
| 17 | I-20 | 740 | 125 | 30 | 745 | 130 | 30 |
| 18 | I-23 | 725 | 130 | 40 | 730 | 135 | 45 |
| 19 | I-26 | 730 | 145 | 35 | 735 | 150 | 40 |
| 20 | I-29 | 735 | 150 | 40 | 750 | 155 | 45 |
| (2) | CTM(2) | 730 | 125 | 40 | 725 | 250 | 100 |

I-3

| Example | Compound | Initial | After 10,000 copy |
|---|---|---|---|
| 21 | I-5 | A | A |
| 22 | I-6 | A | A |
| 23 | I-7 | A | A |
| 24 | I-10 | A | B |
| 25 | I-11 | A | A |
| 26 | I-15 | A | B |
| 27 | I-26 | A | A |
| 28 | I-31 | A | A |
| 29 | I-44 | A | A |
| 30 | I-62 | A | A |
| (3) | CTM(3) | C | D |

As apparent from the Tables a photoreceptor using a charge transfer material of the invention is higher in sensitivity, more stable in photosensitive characteristics when used repeatedly, and less occurrence of image defect when stored at low temperature than the photoreceptor using a conventional charge transfer material.

Compounds of the invention have electron transfer ability and can provide a practical positive charge photoreceptor, having high sensitivity, low residual potential, good image keeping property.

## Claims

1. An electrophotographic photoreceptor comprising a conductive support having thereon a photoreceptive layer unitcontaining a charge generation material and a charge transfermaterial, wherein the photoreceptive layer unit contains an electron transport material compound represented by a formula A, B, C, D, E, F, G ,H or I:

A

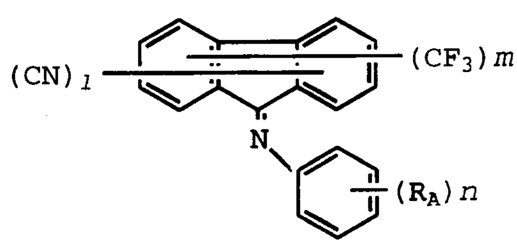

in formula, $l$, $m$ and n represent integers of $l \geq 0$, $m \geq 0$ and $n \geq 0$, respectively, provided $m \geq 2$ when $l = 0$, $m \geq 1$ when $l \geq 1$ and $m \geq 0$ when $l \geq 2$ are satisfied; $R_A$ represents a nitro, OH or $CF_3$ group, a halogen, a substituted or unsubstituted alkyl, alkoxy, aryl, aralkyl or alkylamino group, or a $CONH_2$, $COOR_A'$, $CONHR_A'$, $CON(R_A')_2$ or $COR_A'$ group; $R_A'$ represents a substituted or unsubstituted alkyl, aryl or aralkyl group; and $R_A$ may be the same or different when $n \geq 2$;

B

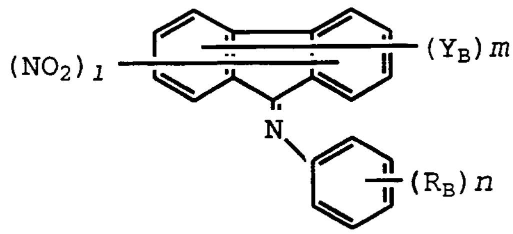

in formula, *l*, *m* and n represent integers of *l* ≧0, *m* ≧0 and *n* ≧0, respectively; $R_B$ represents a nitro, OH, a halogen, $CF_3$, a substituted or unsubstituted alkyl, alkoxy, aryl, aralkyl or alkylamino group, or a $CONH_2$, $COOR_B'$, $CONHR_B'$, $CON(R_B')_2$ or $COR_B'$ group; $R_B'$ represents a substituted or unsubstituted alkyl, aryl or aralkyl group; and $R_A$ may be the same or different when *n* ≧2; $Y_B$ is CN or $CF_3$:

C

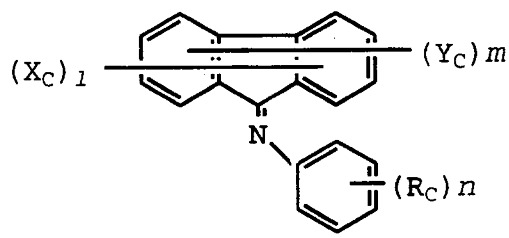

in formula, *l*, *m* and n represent integers of *l* ≧1, *m* ≧0 and *n* ≧1, respectively, provided *m* ≧1 when *l* =1, and *m* ≧0 when *l* ≧2 are satisfied; $R_C$ represents a nitro, OH or $CF_3$ group, a halogen, a substituted or unsubstituted alkyl, alkoxy, aryl, aralkyl or alkylamino group, or a $CONH_2$, $COOR_C'$, $CONHR_C'$, $CON(R_C')_2$ or $COR_C'$ group; $R_C'$ represents a substituted or unsubstituted alkyl, aryl or aralkyl group; and $R_C$ may be the same or different when n≧2; $X_C$ is $OCOR_C'$, $COOR_C'$, $CONHR_C'$, $COR_C'$, CHO or $CON(R_C')_2$; $Y_C$ is CN, $NO_2$, halogen, $CF_3$, COOH, $SO_2NH_2$, $SOR_C'$ or $SO_2R_C'$;

D

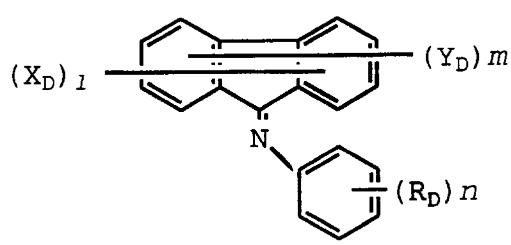

in formula, *l*, *m* and n represent integers of *l* ≧1, m ≧0 and *n* ≧1, respectively, provided *m* ≧1 when *l* =1, and *m* ≧0 when *l* ≧2 are satisfied; $R_D$ represents a nitro, OH, a halogen, $CF_3$, a substituted or unsubstituted alkyl, alkoxy, aryl, aralkyl or alkylamino group, or a $CONH_2$, $COOR_D'$, $CONHR_D'$, $CON(R_D')_2$ or $COR_D'$ group; $R_D'$ represents a substituted or unsubstituted alkyl, aryl or aralkyl group; and $R_D$ may be the same or different when *n* ≧2; $X_D$ is COOH, $SO_2NH_2$, $SOR_D'$ or $SO_2R_D'$; $Y_D$ is CN, $NO_2$, halogen or $CF_3$;

E

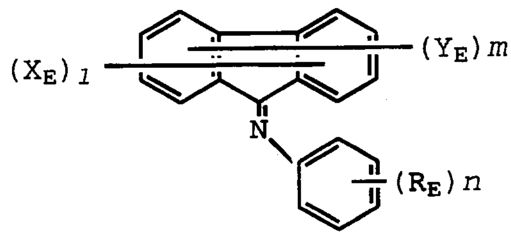

in formula, *l*, *m* and n represent integers of $l \geq 1$, $m \geq 0$ and $n \geq 1$, respectively, provided $m \geq 1$ when $l = 1$, $m \geq 0$ when $l \geq 2$ are satisfied; $R_E$ represents a nitro, OH or $CF_3$ group, a halogen, a substituted or unsubstituted alkyl, alkoxy, aryl, aralkyl or alkylamino group, or a $CONH_2$, $COOR_E'$, $CONR_E'$, $CON(R_A')_2$ or $COR_E'$ group; $R_E'$ represents a substituted or unsubstituted alkyl, aryl or aralkyl group; and $R_E$ may be the same or different when $n \geq 2$; $X_E$ is halogen; $Y_E$ is CN, $NO_2$, or $CF_3$;

F

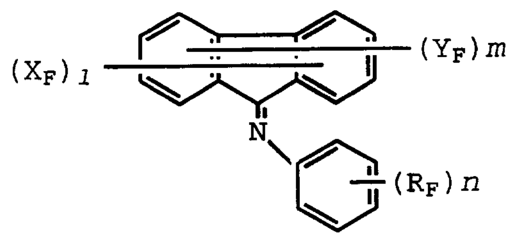

in formula, *l*, *m* and n represent integers of $l \geq 2$, $m \geq 1$ and $n \geq 1$, respectively; $R_F$ represents a nitro, OH, a halogen, $CF_3$, a substituted or unsubstituted alkyl, alkoxy, aryl, aralkyl or alkylamino group, or a $CONH_2$, $COOR_F'$, $CONHR_F'$, $CON(R_F')_2$ or $COR_F'$ group; $R_F'$ represents a substituted or unsubstituted alkyl, aryl or aralkyl group; and $R_F$ may be the same or different when $n \geq 2$; $R_{0F}$ is a substituted or unsubstituted alkyl, aryl or aralkyl group; $X_F$ is CN, $NO_2$, halogen atom, $CF_3$, CHO, COOH, $SO_2NH_2$, $SOR_F'$ or $SO_2R_F'$, $OCOR_F'$, $CONHR_F'$, $COOR_F'$, $CONHR_F'$ or $COR_F'$;

G

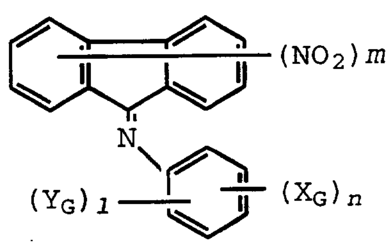

in formula, *l*, *m* and n represent integers of $l \geq 0$, $m \geq 2$ and $n \geq 1$, respectively; $X_G$ represents a nitro, OH, a halogen, CN, a substituted or unsubstituted aryl or aralkyl group, or a $N(R_G')_2$, $OR_G'$, $OCOR_G'$, $COOR_G'$, $CONHR_G'$, $CON(R_G')_2$ or $COR_G'$ group; $R_G'$ represents a substituted or unsubstituted aryl or aralkyl group; and $X_G$ may be the same or different when $n \geq 2$; $Y_G$ is a substituted or unsubstituted alkyl group;

H

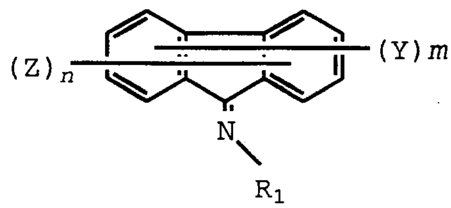

in formula, Y is an $NO_2$, CN, $CF_3$, $COR_2$, $COOR_2$, $CONHR_2$, a halogen, $SOR_2$, or $SO_2R_2$; Z is an alkyl, or aryl group, OH, or $N(R_2)_2$; $R_1$ is an alkyl group; $R_2$ is an alkyl, or aryl group; *m* is an integer of $m \geqq 2$, n is an integers of $n \geqq 0$, respectively, provided the sum of *m* and *n* being 2 to 8; and Y, Z and $R_2$ may be the same or different when each of them exists two or more;

I

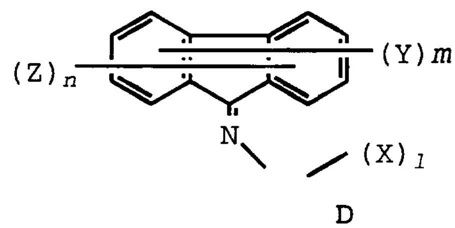

in formula, D is a group of atoms to form a heterocycle; X and Y each represent $NO_2$, CN, $CF_3$, $COR_2$, $COOR_2$, $CONHR_2$, a halogen atom, $SOR_2$, $SO_2R_2$ or $OCOR_2$; Z is an alkyl or aryl group, $OR_2$, OH, or $N(R_2)_2$; $R_1$ is an alkyl group; $R_2$ is an alkyl or aryl group; *l*, *m* and n represent 0 or positive integers respectively, provided $l + m \geqq 2$, and $2 \leqq m + n \leqq 8$; and Y, Z and $R_2$ may be the same or different when each of them exists two or more.

2. An electrophotographic photoreceptor as defined in claim 1, wherein the compound is represented by formula A, B, C, D, E, F and G.

3. An electrophotographic photoreceptor as defined in claim 2, wherein the compound is represented by formula A.

4. An electrophotographic photoreceptor as defined in claim 3, wherein the compound represented by formula A is a compound represented by the following formula a-1:

a-1

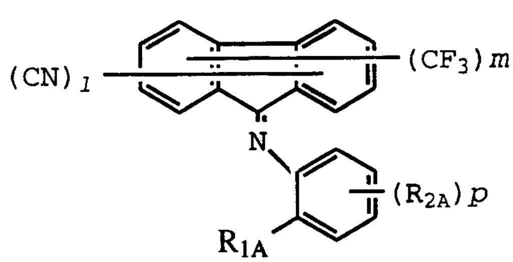

in the formula *l* and *m* are the same as defined in the formula A, *p* is an integer of $p \geqq 0$; $R_{1A}$ and $R_{2A}$ are an alkyl group that may have a substituent, cyano group or halogen atom.

5. An electrophotographic photoreceptor as defined in claim 2, wherein the compound is represented by formula B.

6. An electrophotographic photoreceptor as defined in claim 5, wherein the compound represented by formula B is a compound represented by the following formula b-1:

b-1

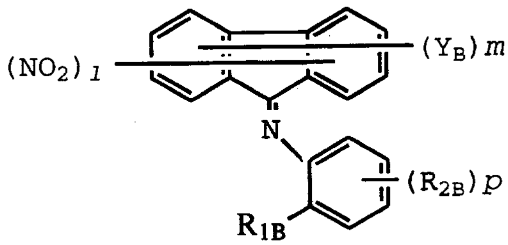

in the formula *l* and *m* are the same as defined in the formula B, *p* is an integer of $p \geq 0$; $R_{1B}$ and $R_{2B}$ are an alkyl group that may have a substituent, cyano group or a halogen atom.

7. An electrophotographic photoreceptor as defined in claim 2, wherein the compound is represented by formula C.

8. An electrophotographic photoreceptor as defined in claim 7, wherein the compound represented by formula A is a compound represented by the following formula c-1:

c-1

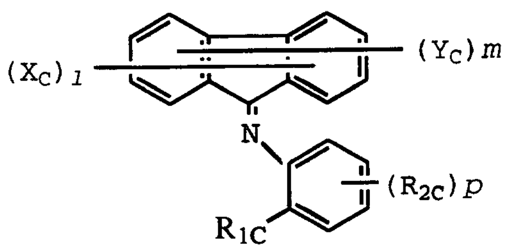

in the formula $X_C$, $Y_C$, *l* and *m* are the same as defined in the formula C, *p* is an integer of $p \geq 0$; $R_{1C}$ and $R_{2C}$ are an alkyl group that may have a substituent, cyano group or halogen atom.

9. An electrophotographic photoreceptor as defined in claim 2, wherein the compound is represented by formula D.

10. An electrophotographic photoreceptor as defined in claim 9, wherein the compound represented by formula D is a compound represented by the following formula d-1:

d-1

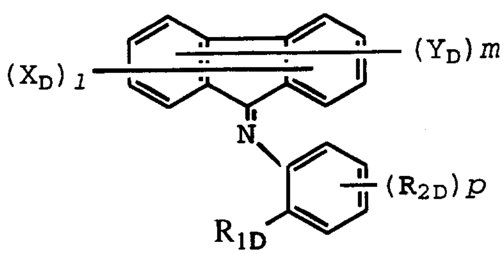

in the formula $X_D$, $Y_D$ *l* and *m* are the same as defined in the formula D, *p* is an integer of $p \geq 0$; $R_{1D}$ and $R_{2D}$ are, respectively, an alkyl group that may have a substituent, cyano group or halogen atom.

**11.** An electrophotographic photoreceptor as defined in claim 2, wherein the compound is represented by formula E.

**12.** An electrophotographic photoreceptor as defined in claim 11, wherein the compound represented by formula E is a compound represented by the following formula e-1:

e-1

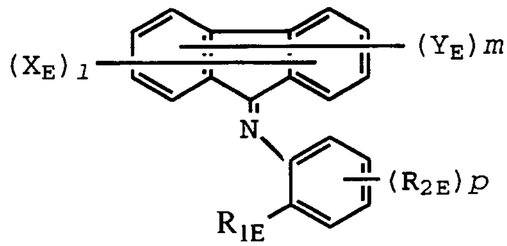

in the formula $X_E$, $X_E$, *l* and *m* are the same as defined in the formula E, *p* is an integer of p ≧0; $R_{1E}$ and $R_{2E}$ are an alkyl group that may have a substituent, cyano group or halogen atom.

**13.** An electrophotographic photoreceptor as defined in claim 2, wherein the compound is represented by formula F.

**14.** An electrophotographic photoreceptor as defined in claim 3, wherein the compound represented by formula F is a compound represented by the following formula f-1:

f-1

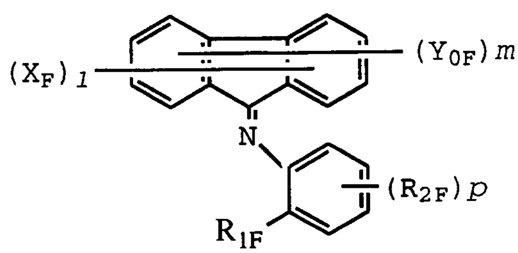

in the formula $X_F$, $R_{0F}$,*l* and *m* are the same as defined in the formula F, *p* is an integer of *p* ≧0; $R_{1F}$ and $R_{2F}$ are an alkyl group that may have a substituent, cyano group or halogen atom.

**15.** An electrophotographic photoreceptor as defined in claim 2, wherein the compound is represented by formula G.

**16.** An electrophotographic photoreceptor as defined in claim 13, wherein the compound represented by formula G is a compound represented by the following formula g-1:

g-1

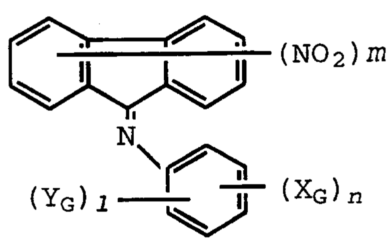

in the formula *l*, *m* and n represent integers of *l* ≧0, *m* ≧3 and *n* ≧1, respectively; $X_G$ represents a

nitro, a halogen, CN, a substituted or unsubstituted aryl or group, or a $COOR_G'$, $OCOR_G'$, $CONHR_G'$, $CON(R_G')_2$ or $COR_G'$ group; $R_G'$ represents a substituted or unsubstituted aryl or aralkyl group; the other groups are the same as defined in the formula G.

**17.** An electrophotographic photoreceptor as defined in claim 2, wherein the compound is represented by formula H or I.

**18.** An electrophotographic photoreceptor as defined in claim 17, wherein the compound is represented by formula H.

**19.** An electrophotographic photoreceptor as defined in claim 18, wherein the compound is represented by formula I.

FIG. 1 (a)

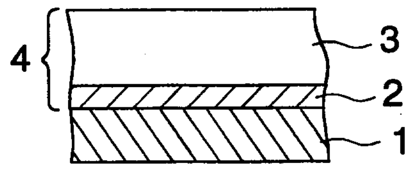


FIG. 1 (b)

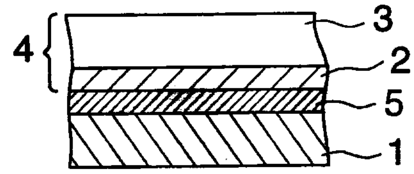


FIG. 1 (c)

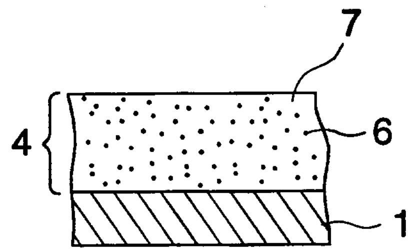


FIG. 1 (d)